# EUROPEAN PATENT SPECIFICATION

(11) **EP 4 565 881 B1**
(45) Date of publication and mention of the grant of the patent: **12.08.2026**
(21) Application number: 23750778.5
(22) Date of filing: 01.08.2023
(51) Int. Cl.: G01N 33/53, G01N 33/68

(54) **MEANS FOR DETECTING SCHISTOSOMA INFECTION**
MITTEL ZUM NACHWEIS EINER SCHISTOSOMA-INFEKTION
MOYENS DE DÉTECTION D'UNE INFECTION À SCHISTOSOMA

(30) Priority: 02.08.2022 NL 2032668
(43) Date of publication of application: 11.06.2025
(73) Proprietor: Academisch Ziekenhuis Leiden (h.o.d.n. LUMC), 2333 ZA Leiden (NL)
(72) Inventor: KILDEMOES, Anna Marie Overgaard, 2333 ZA Leiden (NL); HOKKE, Cornelis Hendrik, 2333 ZA Leiden (NL)
(74) Representative: HGF
(86) International application number: PCT/NL2023/050411
(87) International publication number: WO 2024/030024

(56) References cited:
- WO-A1-00/17654
- WO-A1-2012/145398
- WO-A2-2006/107198
- VERMEER H J ET AL: "Immunodiagnostically applicable monoclonal antibodies to the circulating anodic antigen of Schistosoma mansoni bind to small, defined oligosaccharide epitopes", PARASITOLOGY RESEARCH, vol. 90, no. 4, 2003, pages 330 - 336, XP002513961
- HALKES K M ET AL: "Preparation of spacer-containing di-, tri-, and tetrasaccharide fragments of the circulating anodic antigen of Schistosoma mansoni for diagnostic purposes", CARBOHYDRATE RESEARCH, vol. 309, no. 2, 1998, pages 175 - 188, XP004145731
- BERGWERFFS ALDERT A ET AL: "The Immunologically Reactive Part of Immunopurified Circulating Anodic Antigen from Schistosoma mansoni Is a Threonine-linked Polysaccharide Consisting of -6)-(P-~-GlcpA-( 1-3))-P-~-GalpNAc-( 1-Repeating Units*", THE JOURNAL OF BIOLOGICAL CHEMISTRY, vol. 269, no. 50, 1994, pages 31510 - 31517, XP093032102
- DAM GOVERT J. ET AL: "The Immunologically Reactive O-Linked Polysaccharide Chains Derived from Circulating Cathodic Antigen Isolated from the Human Blood Fluke Schistosoma Mansoni have Lewis x as Repeating Unit", EUROPEAN JOURNAL OF BIOCHEMISTRY, vol. 225, no. 1, 1994, pages 467 - 482, XP093032103

## Description

The present invention provides methods for detecting an *anti-Schistosoma* antibody in a sample obtained from a subject. Corresponding compounds, compositions, kits and methods for monitoring a subject to determine if they have contracted a *Schistosoma* infection are also provided.

### Background

Schistosomiasis is a disease caused by parasitic blood flukes of the genus *Schistosoma* (superfamily *Schistosomatidae*) with different manifestations in acute to chronic stages. Individuals become infected when larval forms of the parasite (cercariae), released by freshwater snails, penetrate the skin during contact with infested water. In the body, the larvae develop into adult schistosomes. Adult worms live in the blood vessels where the females release eggs. Some of the eggs are passed out of the body in the faeces, urine or genital fluids to continue the parasite's lifecycle. Others become trapped in body tissues, causing immune reactions and progressive damage to organs. Transmission occurs when people suffering from schistosomiasis contaminate freshwater sources with their excreta, urine or genital fluids containing parasite eggs, which hatch in water, subsequently leading to infection of new snails and snail borne larvae infective to humans and mammals.

Schistosomiasis is prevalent in tropical and subtropical areas, especially in poor communities without access to safe drinking water and adequate sanitation. Schistosomiasis transmission has been reported from 78 countries and it is estimated that at least 90% of those requiring treatment for schistosomiasis live in Africa. However, with the rise in eco-tourism and travel "off the beaten track", increasing numbers of tourists are also contracting schistosomiasis through popular activities as swimming and rafting. In the last decade autochtonous transmission in Corsica, Europe has also been re-established and climate changes combined with altered human migration patterns increase the risk of expanding transmission zones. Contracting schistosomiasis can lead to severe morbidity and disease such as bladder cancer, infertility, oesophagal varices, stunting, aneamia, liver fibrosis and more rarely neuroschistosomiasis manifestations such as seizures, paralysis or spinal cord inflammation. Estimates from the World Health Organisation show that at least 236.6 million people required preventive treatment for schistosomiasis in 2019, out of which more than 105.4 million people were reported to have been treated.

The focus of the schistosomiasis intervention agenda is shifting from morbidity control to elimination, and there is a WHO-mandated objective to eliminate schistosomiasis as a public health concern and interrupt transmission in selected areas. It is therefore imperative that methods to detect infection are appropriately sensitive, very specific and rapid in their execution in order to diagnose new cases of disease, assess the effectiveness of control measures and be applicable to large-scale disease surveillance. Most existing serological tests for schistosomiasis are based on crude soluble antigen from eggs, parasite larvae (cercariae) or adult worms. However, using crude antigen from schistosome parasites means that there is inherent variability between crude antigen batches, making it difficult to standardize assays. Furthermore, material has to be derived from schistosome life cycles which is costly and time consuming. WO00/17654A1 discloses the detection of antibodies against Schistosoma by capturing with Le^{x} saccharide antigen.

Schistosome crude antigens are highly glycosylated, and many glycan elements present in the material can be shared with other organisms such as common co-infections. Such shared epitopes between schistosomes and for example soil-transmitted helminths lowers the specificity and can give false positives in these crude antigen-based tests. There is a significant need for improved reagents and methods for detecting schistosome infection.

### Brief summary of the disclosure

The inventors have now identified a new approach to testing for *Schistosoma* infection, wherein the infected individual's antibody response to infection is detected. The methods developed herein are particularly advantageous for identifying new *Schistosoma* infections with high specificity and sensitivity (often before symptoms of infection occur). The methods described herein have particular utility in detecting primary *Schistosoma* infection and/or acute schistosomiasis in those at risk of infection (e.g. during or after travel to a region where *Schistosoma* infection is prevalent and/or endemic; and/or during or after suspected exposure to schistosome-infected water).

The inventors have used a defined, highly immunogenic and highly unique glycan structure from circulating anodic antigen (CAA) as a means for detecting *Schistosoma* infection-specific antibodies. This new approach preserves the high sensitivity of antibody detection but eliminates the cross-reactive potential of using crude *Schistosoma* antigen extract (which has low specificity). The approach provided herein also eliminates batch to batch variation, the compounds provided herein comprise synthetically produced polysaccharides and thus can be standardised.

Accordingly, the invention provides a method of detecting an anti-*Schistosoma* antibody in a biological sample obtained from a subject, the method comprising:
a) contacting the sample with a compound R-X-Y, wherein R is according to Formula I: 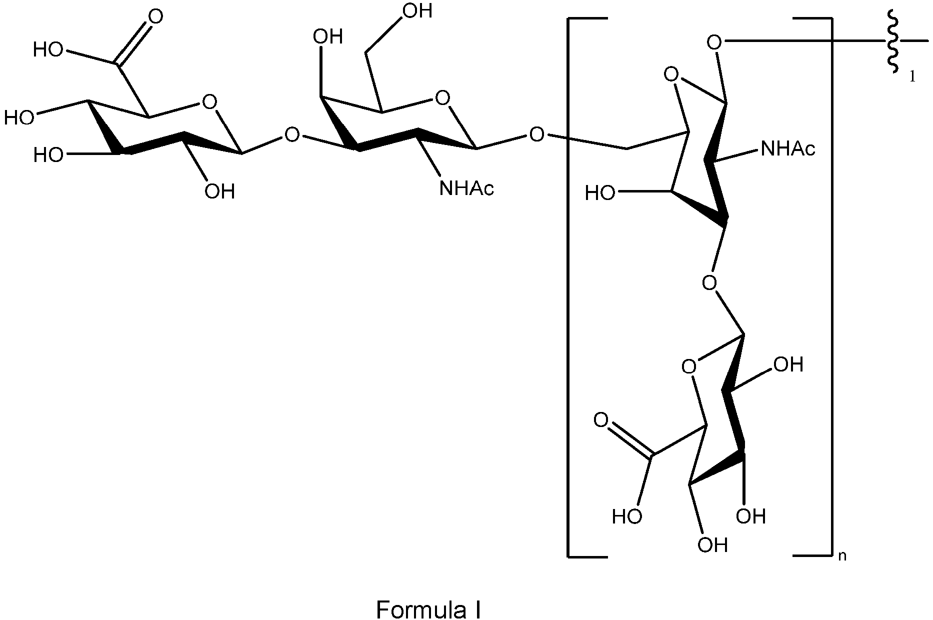
   wherein n is a whole integer selected from 2 to 19; and
   wherein 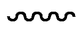 1 denotes the point of attachment of R to X, wherein X is selected from the group consisting of: a bond and a linker; and wherein Y is selected from the group consisting of: H, a reporter molecule, a carrier molecule, and a solid support; and
b) determining if binding between the sample and the compound occurs;
wherein binding between the sample and the compound is indicative of the presence of the antibody in the sample.

Suitably, n may be selected from n = 2, 3, 4, or 5.

Suitably, X may be C₂-C₁₀-alkylene-NH- (e.g. C₆H₁₂NH-).

Suitably:
(a) Y may be H; or
(b) Y may be a reporter molecule, a carrier molecule, or a solid support.

Suitably, the presence of the antibody may be indicative of a current or prior *Schistosoma* infection in the subject.

Suitably, the sample may be obtained from a subject that is at risk of contracting a *Schistosoma* infection and/or has one or more symptoms indicative of a *Schistosoma* infection.

Suitably, the subject may be at risk of contracting a *Schistosoma* infection if they have been to a location where *Schistosoma* infection is prevalent and/or endemic.

Suitably, the sample may be obtained from the subject at least 4 weeks after having one or more symptoms indicative of a *Schistosoma* infection.

Suitably, symptoms indicative of a *Schistosoma* infection may include one or more of: a skin rash, fever, headache, myalgia, general malaise, diarrhoea, non-productive cough, muscle pain, joint pain, abdominal tenderness or pain, hepatosplenomegaly, eosinophilia, urticaria, angiooedema, (micro)haematuria, transient pulmonary infiltrates and/or parasite eggs in one or more of stool, urine and genital fluids.

In another aspect, the invention provides a method of monitoring a subject to determine if they have contracted a *Schistosoma* infection, the method comprising:
a) contacting a first biological sample obtained from the subject at a first time point with a compound R-X-Y, wherein R is according to Formula I: 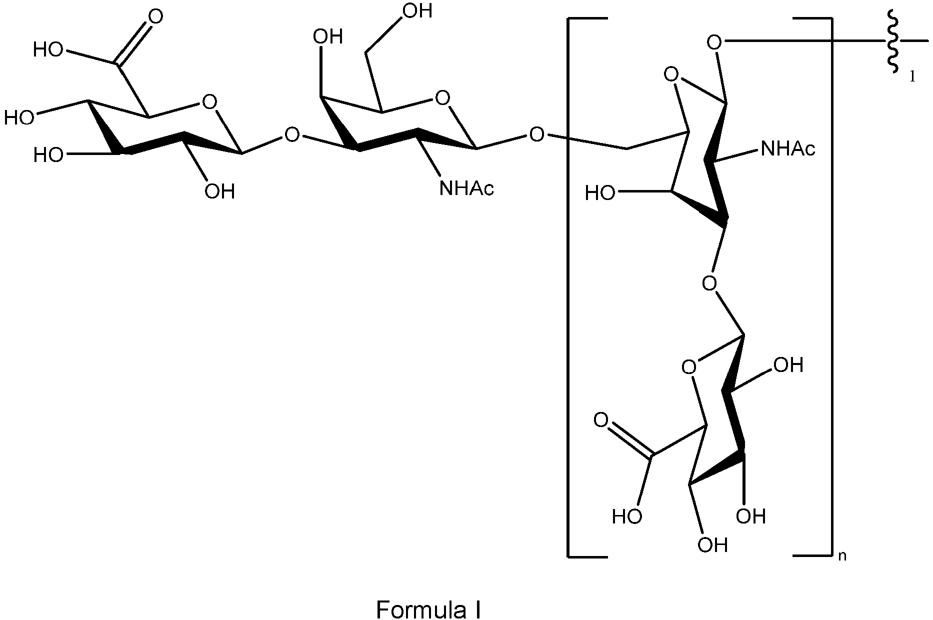 wherein 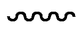 1 denotes the point of attachment of R to X, wherein X is selected from the group consisting of: a bond and a linker; and wherein Y is selected from the group consisting of: H, a reporter molecule, a carrier molecule, and a solid support, and n is a whole integer selected from 2 to 19; and determining if binding between the compound and the first sample occurs;
b) contacting a second biological sample with the compound, wherein the second sample is obtained from the subject at a later time point than the first sample, and determining if binding between the compound and the second sample occurs;
c) comparing the binding determined in step b) with the binding determined in step a), wherein an increase in b) as compared to a) is indicative of the subject having contracted the infection.

Suitably, n may be selected from n = 2, 3, 4, or 5.

Suitably, X may be --C₂-C₁₀-alkylene-NH- (e.g. C₆H₁₂NH-).

Suitably:
(a) Y may be H or
(b) Y may be a reporter molecule, a carrier molecule, or a solid support.

Suitably, the time period between the first and second sample may be at least 4 weeks.

Suitably, an increase in b) as compared to a) may be indicative of the subject having contracted the infection between the first time point and later time point.

Suitably, the second sample may be obtained from a subject that is at risk of contracting the *Schistosoma* infection and/or has one or more symptoms indicative of the *Schistosoma* infection.

Suitably, the subject may be at risk if they have been to a location where *Schistosoma* infection is prevalent and/or endemic.

Suitably, the second sample may be obtained from the subject at least 4 weeks after having one or more symptoms indicative of the *Schistosoma* infection.

Suitably, the symptoms may include one or more of: a skin rash, fever, headache, myalgia, general malaise, diarrhoea, non-productive cough, muscle pain, joint pain, abdominal tenderness or pain, hepatosplenomegaly, eosinophilia, urticaria, angiooedema, (micro)haematuria, transient pulmonary infiltrates and/or parasite eggs in one or more of stool, urine and genital fluids.

Suitably, the subject may have acute schistosomiasis.

Suitably, the subject may have a primary *Schistosoma* infection.

Suitably, the *Schistosoma* infection may be caused by a *Schistosoma* species selected from the group consisting of: *Schistosoma mansoni, Schistosoma japonicum, Schistosoma mekongi, Schistosoma guineensis, Schistosoma intercalatum,* and *Schistosoma haematobium.*

Suitably, the antibody may be selected from the group consisting of: IgM, IgA, and IgG.

Suitably, the subject may be human.

Suitably, the sample may be selected from the group consisting of: a blood sample, nasal sample, urine sample, genital fluid sample and a stool sample.

Suitably, the blood sample may be a serum sample, plasma sample, or a whole blood sample.

Suitably, the nasal sample may be obtained by nasosorption sampling.

Suitably, when the sample is a nasal sample or a blood sample, the antibody may be IgA.

Suitably, when the sample is a blood sample, the antibody may be IgM and/or IgG.

Suitably, binding between the sample and the compound may be detected by a technique selected from the group consisting of: microarray assay, ELISA, immunoblotting assay, bead-based multiplex assay, and lateral-flow assay.

In another aspect, the invention provides a compound R-X-Y, wherein R is according to Formula I: 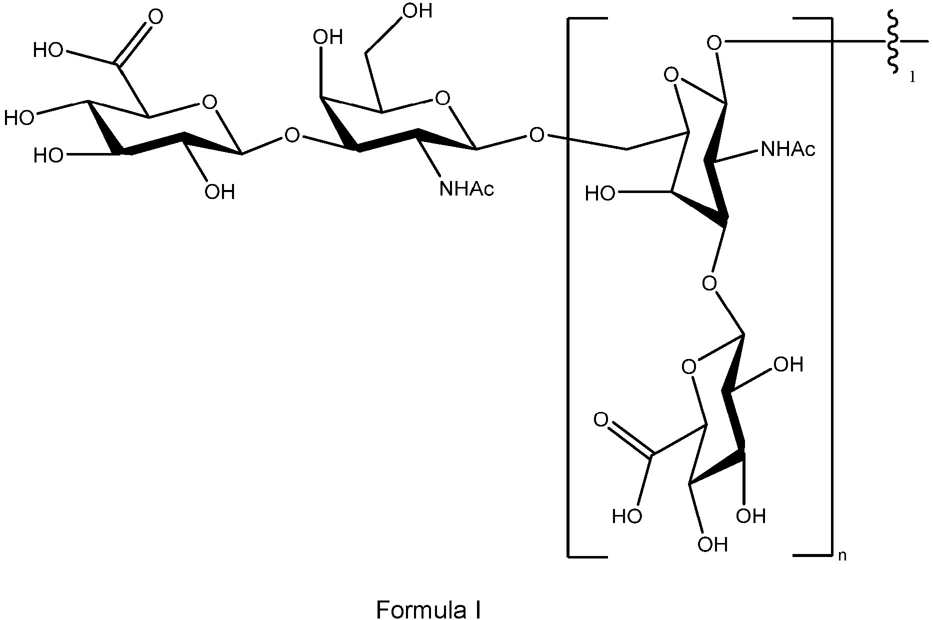 wherein 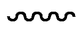 1 denotes the point of attachment of R to X, wherein X is selected from the group consisting of: a bond and a linker; and Y is selected from the group consisting of: H, a reporter molecule, a carrier molecule, and a solid support; and n is whole integer selected from 2 to 19.

Suitably, n may be selected from n = 2, 3, 4, or 5.

Suitably, X may be --C₂-C₁₀-alkylene-NH- (e.g. C₆H₁₂NH-).

Suitably:
(a) Y may be H or
(b) Y may be a reporter molecule, a carrier molecule, or a solid support.

Suitably, Y may comprise a solid support.

Suitably, the solid support may be selected from the group consisting of a membrane, plastic, polymer, and glass.

In another aspect, the invention provides a composition comprising a plurality of distinct compounds according to the invention.

Suitably, the composition may comprise at least two compounds selected from the group consisting of:
(i) a compound of Formula I, wherein n = 2;
(ii) a compound of Formula I, wherein n = 3;
(iii) a compound of Formula I, wherein n = 4; and
(iv) a compound of Formula I, wherein n = 5.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

Various aspects of the invention are described in further detail below.

### Brief description of the Figures

Embodiments of the invention are further described hereinafter with reference to the accompanying drawings, in which:

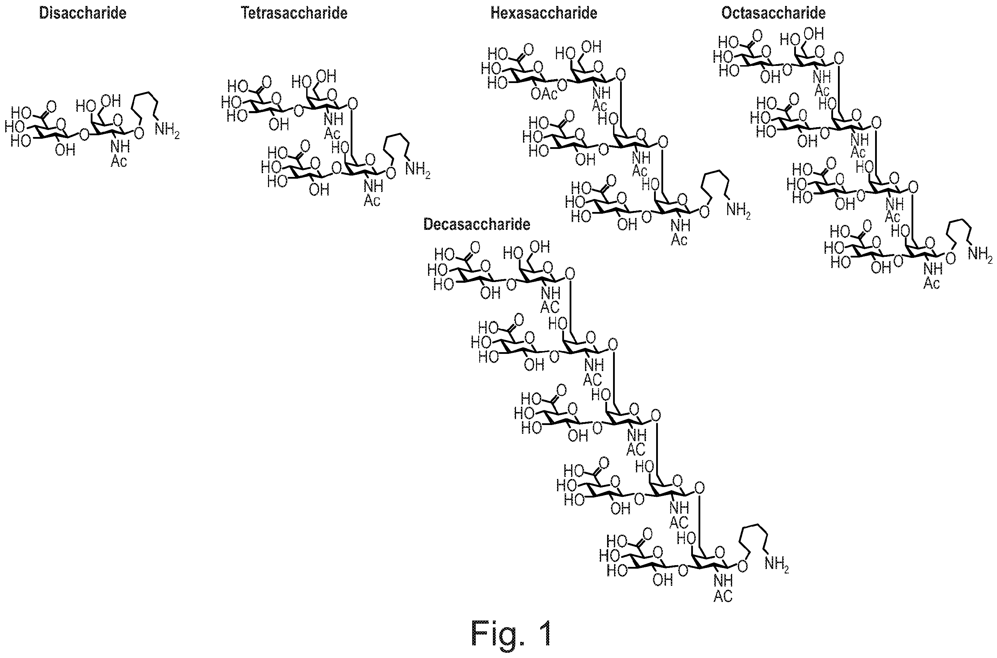

**Figure 1** shows example compounds described herein.
**Figure 2** shows IgM and IgG antibodies binding to native CAA in controlled human infection samples.
**Figure 3** shows IgM binding to example compounds described herein, 10 cercariae (parasite larvae) infection dose.
**Figure 4** shows IgM binding to example compounds described herein, 20 cercariae (parasite larvae) infection dose.
**Figure 5** shows IgM binding to example compounds described herein, 30 cercariae (parasite larvae) infection dose.
**Figure 6** shows IgG binding to example compounds described herein, 10 cercariae (parasite larvae) infection dose.
**Figure 7** shows IgG binding to example compounds described herein, 30 cercariae (parasite larvae) infection dose.
**Figure 8** shows IgM (left) and IgG (right) binding to native CAA and synthetic CAA-repeat hexasaccharide (hexa) and octasaccaride (octa), in cases where there has been a natural exposure to cercariae (parasite larvae).
**Figure 9** shows IgA binding to native CAA, hexasaccharide and octasaccharide in serum samples, 20 cercariae infection dose.
**Figure 10** shows change in IgA response to native CAA, hexasaccharide and octasaccharide in nasosorption samples, 20 cercariae infection dose at baseline compared to 8 weeks post infection.
**Figure 11** shows IgM and IgG measured by ELISA: direct antigen coating (CAA) and gold nanoparticle-antigen conjugation coated (octasaccharide).
**Figure 12** shows IgG measured by Immunoblots: direct antigen coating (CAA) and gold nanoparticle-antigen conjugation coated (octasaccharide).
**Figure 13** shows IgM and IgG measured by Immunoblots: direct antigen coating (CAA) and gold nanoparticle-antigen conjugation coated (octasaccharide).
**Figure 14** shows IgG binding to native CAA and synthetic CAA compounds (di-, tetra-, hexa-, octa- and decasaccharide) in samples from three separate controlled human infections (20 cercariae exposure).

The patent, scientific and technical literature referred to herein establish knowledge that was available to those skilled in the art at the time of filing. In the case of any inconsistencies, the present disclosure will prevail.

Various aspects of the invention are described in further detail below.

### Detailed Description

The invention is based in part on the discovery that short polysaccharides are useful for the detection of antibodies against *Schistosoma* and/or for the diagnosis of *Schistosoma* infection in biological samples (such as a blood sample, a nasal sample, a urine sample, or a genital fluid sample) obtained from a human subject. The inventors have demonstrated, as illustrated in the experimental section below, that these polysaccharides can be used to efficiently detect antibodies, in particular IgG, IgA, and/or IgM, produced by the subject as part of the host immune response upon *Schistosoma* infection. Each of these polysaccharides, which are fragments of *Schistosoma* CAA, are recognized with great sensitivity and specificity by infected host's antibodies, and enable the diagnosis of *Schistosoma* infection in the majority of infected hosts, including infected individuals with a low infection dose. Advantageously, this facilitates the detection of new cases when the infection is still at an early stage and thus can be used to implement an efficient treatment strategy. Compounds are therefore provided herein, where the compounds are characterized by the presence of a polysaccharide, either alone; or attached to a linker, a reporter molecule, a carrier molecule, a solid support, or a combination thereof. Advantageously, these compounds can be produced synthetically, such that batch to batch variation is eliminated, assay reproducibility is improved, and quality control is possible.

### Compounds

CAA is a juvenile/adult worm gut-derived antigen that is produced by the *Schistosomatidae* superfamily. It is one of hundreds of antigens produced by the worm, and is a glycosaminoglycan-like molecule that is regurgitated into the host's bloodstream. CAA's polysaccharide structure renders it very stable, and it has not been identified in organisms other than schistosomes. It has a strong negative charge and is at least 10 kDa, but can vary in molecular weight. For further details on the structure of CAA see Bergwerff, A. A., *et al.* (1994).

The compounds provided herein are composed of a polysaccharide, where the polysaccharide is optionally attached to a linker, a reporter molecule, a carrier molecule, a solid support, or a combination thereof. The polysaccharide structures (R) provided herein are representative of fragments of CAA. The term "polysaccharide" as used herein refers to a molecule having at least two monosaccharides.

The inventors have suprisingly identified that antibodies to these specific structures are present in the majority of individuals having a *Schistosoma* infection. The compounds of the invention are described herein as R-X-Y, wherein R is according to Formula I: 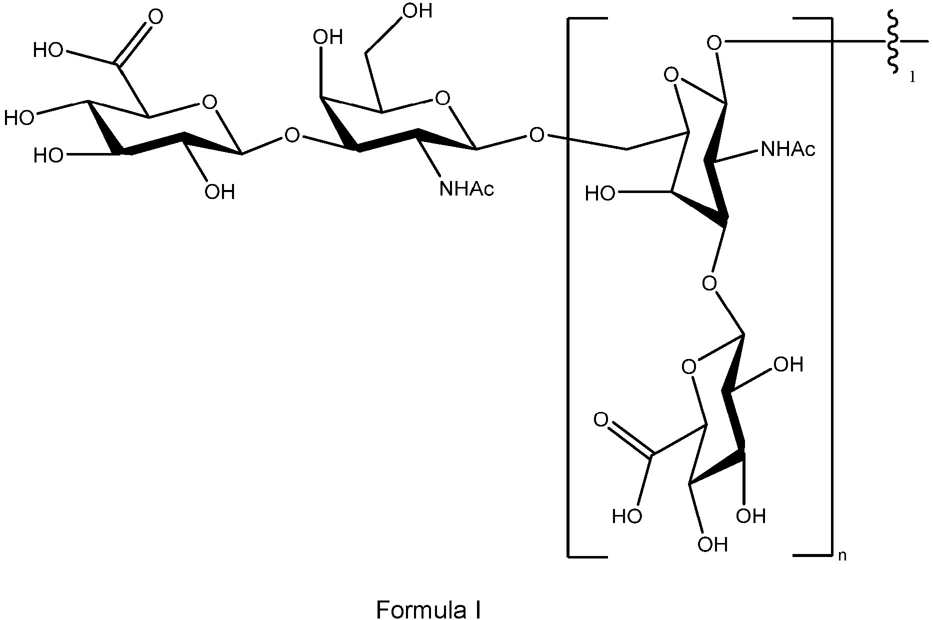 and wherein 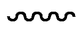 1 denotes the point of attachment of R to X; wherein X is selected from the group consisting of: a bond and a linker; and wherein Y is selected from the group consisting of: H, a reporter molecule, a carrier molecule, and a solid support.

R according to Formula I comprises a repeating unit, n, where n is a whole integer from 2 to 19. The repeating unit, n, may be selected from the group consisting of: 2, 3, 4, 5, 6, 7, 8, 9, 10, 11, 12, 13, 14, 15, 16, 17, 18 and 19.

In one example, n is a whole integer from 2 to 15. In another example, n is a whole integer from 2 to 10. In a further example, n is a whole integer from 2 to 7. In a further example, n is a whole integer from 2 to 5. It may be that in each of these examples, X is a linker. The linker may be C₂-C₁₀-alkylene-NH- (e.g. -C₆H₁₂NH-)-. It may be that Y is H. It may be that the unit -X-Y is 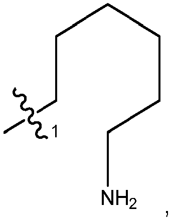 wherein 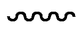 1 denotes the point of attachment of R to X.

In one particular example, n may equal a whole integer from 3 to 19. In one example, n is a whole integer from 3 to 15. In another example, n is a whole integer from 3 to 10. In a further example, n is a whole integer from 3 to 7. In a further example, n is a whole integer from 3 to 5. In each of these examples, X may be a linker. The linker may be -C₂-C₁₀-alkylene-NH- (e.g. -C₆H₁₂NH-) . It may be that Y is H. It may be that the unit -X-Y 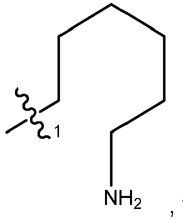 wherein 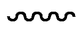 1 denotes the point of attachment of R to X.

In one particular example, n may equal a whole integer from 4 to 19. In one example, n is a whole integer from 4 to 15. In another example, n is a whole integer from 4 to 10. In a further example, n is a whole integer from 4 to 7. In a further example, n is a whole integer of 4 or 5. In each of these examples, X may be a linker. The linker may be -C₂-C₁₀-alkylene-NH- (e.g. -C₆H₁₂NH-). It may be that Y is H. It may be that the unit -X-Y is 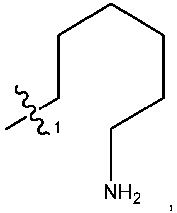 wherein 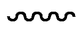 1 denotes the point of attachment of R to X.

In one particular example, n may equal a whole integer from 5 to 19. In one example, n is a whole integer from 5 to 15. In another example, n is a whole integer from 5 to 10. In a further example, n is a whole integer from 5 to 7. In a further example, n is 5. In each of these examples, X may be a linker. The linker may be -C₂-C₁₀-alkylene-NH- (e.g. - C₆H₁₂NH-). It may be that Y is H. It may be that the unit -X-Y is 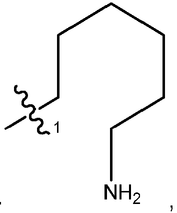 wherein 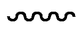 1 denotes the point of attachment of R to X.

It may be that X is a bond.

It may be that, X is a linker. The linker may be -C₂-C₁₀-alkylene-NH- (e.g. -C₆H₁₂NH-.i.e. 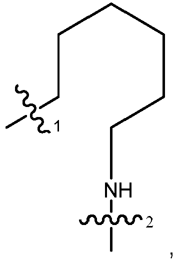 wherein 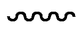 1 denotes the point of attachment of R to X, and 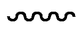 2 denotes the point of attachment of X to Y). Y may be selected froma reporter molecule, a carrier molecule, and a solid support.

It may be that Y is H.

It may be that Y is selected from reporter molecule, a carrier molecule, and a solid support. It may be that Y is a reporter molecule. It may be that Y is a carrier molecule. It may be that Y is a solid support.

It may be that X is a bond and Y is H. It may be that X is a bond and Y is selected from reporter molecule, a carrier molecule, and a solid support.

It may be that X is a linker and Y is H. It may be that X is a bond and Y is selected from reporter molecule, a carrier molecule, and a solid support.

In other words, X may be a linker that attaches R to a reporter molecule, a carrier molecule, or a solid support. In one example, X cis a linker and Y is a solid support. In another example, X is a linker and Y is a carrier molecule, such as BSA.

When R has a repeating unit of n = 2 it may also be referred to as a hexasaccharide, as it has three disaccharide repeats. The compound R-X-Y, wherein R has a repeating unit of n = 2, is therefore referred to as a "hexasaccharide", "hexamer", "hexa" or "6-mer" herein.

When R has a repeating unit of n = 3 it may also be referred to as an octasaccharide, as it has four disaccharide repeats. The compound R-X-Y, wherein R has a repeating unit of n = 3, is therefore also referred to as a "octasaccharide", "octamer", "octa" or "8-mer" herein.

When R has a repeating unit of n = 4 it may also be referred to as an decasaccharide, as it has five disaccharide repeats. The compound R-X-Y, wherein R has a repeating unit of n = 4, is therefore also referred to as a "decasaccharide", "decamer", "deca" or "10-mer" herein.

When R has a repeating unit of n = 5 it may also be referred to as an dodecasaccharide, as it has six disaccharide repeats. The compound R-X-Y, wherein R has a repeating unit of n = 5, is therefore also referred to as a "dodecasaccharide", "dodecamer", "dodeca" or "12-mer" herein.

Equivalent nomenclature for a compound wherein R has a repeating unit of n = 6, 7, 8, 9, 10, 11, 12, 13, 14,15, 16, 17, 18 or 19 would be readily identifiable to a person of skill in the art based on the disclosure provided herein.

For comparative purposes, for compounds of formula I, wherein n = 1, R may also be referred to as a tetrasaccharide, as it has two disaccharide repeats. The compound R-X-Y, wherein R has a repeating unit of n = 1 may therefore also referred to as a "tetrasaccharide", "tetramer", "tetra" or "4-mer" herein.

Furthermore, for comparative purposes, compounds of formula I, wherein n = 0, R may be referred to as a disaccharide, as it has one disaccharide repeat. The compound R-X-Y, wherein R has a repeating unit of n = 0 may therefore also referred to as a "disaccharide", "di" or "2-mer" herein.

The compounds described herein may be generated by any appropriate means known in the art. In one example, R is a synthetic polysaccharide. An example of how to generate synthetic polysaccharides is provided in the examples section below. In another example, biotechnological means can be used, for example production of polysaccharides in engineered expression systems containing the relevant glycosyltransferase enzymes.

The compounds described herein may be present within a composition. For example, the composition may comprise a plurality of distinct (structurally different) compounds described herein (e.g. to increase sensitivity and/or specificity when used in a method described herein). In other words, a composition comprising a mix of two or more structurally different compounds is provided, wherein each of the structurally different compounds is R-X-Y, wherein R is according to Formula I: 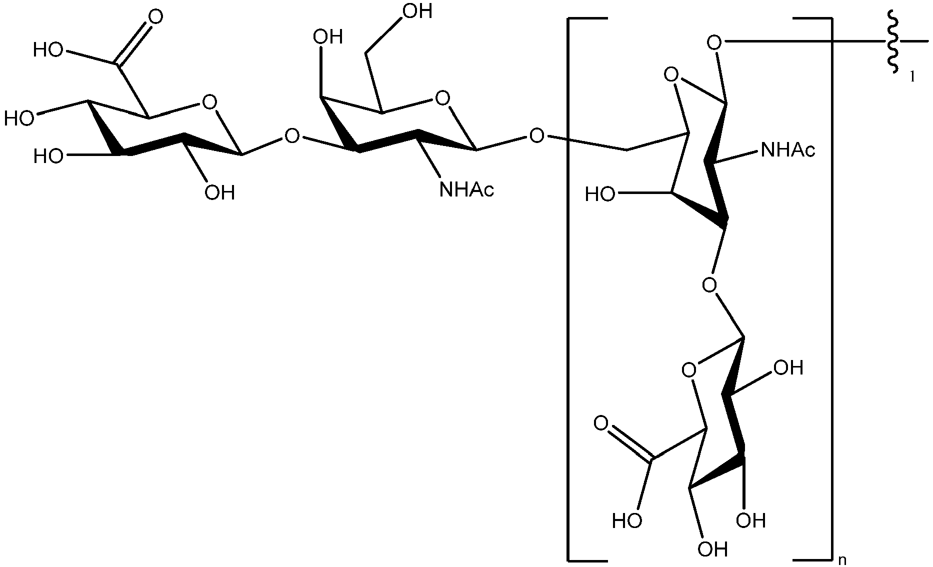 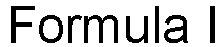 wherein 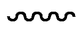 1 denotes the point of attachment of R to X, wherein X is selected from: a bond and a linker; and wherein Y is selected from the group consisting of: H, a reporter molecule, a carrier molecule, and a solid support, and wherein n is a whole integer selected from 2 to 19.

In one example, the composition may comprise at least two (e.g. at least three, at least four etc) compounds of the invention, wherein the at least two (e.g. at least three, at least four etc) compounds each have a distinct number of repeating units selected from the group consisting of: n = 2, n = 3, n = 4, and n = 5.

The compounds or compositions described herein encompass compounds wherein X is a bond and Y is a solid support. Accordingly, a solid support is provided herein, wherein the solid support immobilizes R.

The solid support may be any suitable solid support. By "solid support" it is herein referred to material which is insoluble, or can be made insoluble by a subsequent reaction. Numerous and varied solid supports are known to those in the art and include, without limitation, nitrocellulose, the walls of wells of a reaction tray, multi-well plates, test tubes, polystyrene beads, magnetic beads, membranes, microparticles (such as latex particles), and sheep (or other animal) red blood cells. Any suitable porous material with sufficient porosity to allow access by detector reagents and a suitable surface affinity to immobilize capture reagents is contemplated by this term. For example, the porous structure of nitrocellulose has excellent absorption and adsorption qualities for a wide variety of reagents, for instance, capture reagents. Nylon possesses similar characteristics and is also suitable. Microporous structures are useful, as are materials with gel structure in the hydrated state.

Further examples of useful solid supports include: natural polymeric carbohydrates and their synthetically modified, cross-linked or substituted derivatives, such as agar, agarose, cross-linked alginic acid, substituted and cross-linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber; synthetic polymers which may be prepared with suitably porous structures, such as vinyl polymers, including polyethylene, polypropylene, polystyrene, polyvinylchloride, polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides; porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and mixtures or copolymers of the above classes, such as graft copolymers obtained by initializing polymerization of synthetic polymers on a preexisting natural polymer.

Further examples of useful solid supports include: a microfluidic chip, a silicon chip, a microscope slide, a microplate well, solid and semi-solid matrixes, resins, beads, biochips, multi-well plates, membranes, conducting and non-conducting metals, glass, magnetic supports, silica gels, polymeric membranes, particles, derivatised plastic films, derivatised glass, derivatised silica, glass beads, cotton, plastic beads, alumina gels, polysaccharides, polyvinylchloride, polypropylene, polyethylene, nylon, latex bead, magnetic bead, paramagnetic bead, or superparamagnetic bead; or Sepharose, poly(acrylate), polystyrene, poly(acrylamide), polyol, agarose, agar, cellulose, dextran, starch, FICOLL, heparin, glycogen, amylopectin, mannan, inulin, nitrocellulose, diazocellulose or starch.

Y may be a solid support. The surface of a solid support may have been activated by chemical processes that allow covalent linkage of an agent comprising R (where X is a bond) or R-X (where X is a linker) to the solid support. Except as otherwise physically constrained, a solid support may be used in any suitable shapes, such as films, sheets, strips, or plates, or it may be coated onto or bonded or laminated to appropriate inert carriers, such as paper, glass, plastic films, or fabrics. It may be in the form of a microarray chip, a microarray wafer, a microarray strip, a microwell, and a bead.

In one example, the solid support may be selected from the group consisting of: a membrane, plastic, polymer, and glass. For example, the solid support may be a membrane such as a nitrocellulose or PVDF membrane. In another example, the solid support may be a polymer, such as silicone.

The compounds or compositions described herein encompass compounds wherein X is a bond and Y is a carrier molecule. In some examples, the carrier molecule may be a carrier protein. Any appropriate carrier protein may be used. In one example, the carrier protein is, avidin, streptavidin, transferrin, neutravidin or serum albumin (e.g. bovine serum albumin, BSA). In other examples, the protein may be a schistosome protein antigen that is not a CAA antigen (such that the compound provides a bivalent antigen for anti-schistosome antibodies). Alternatively, the protein may be a protein derived from a distinct pathogen (i.e. a pathogen that is not *Schistosoma*), such that the compound can be used to simultaneously detect antibodies specific for a plurality of pathogens.

In some examples, the carrier molecule is biotin.

By "protein" "polypeptide" and "peptide" it is herein referred to a molecule comprising amino acids joined via peptide bonds. In general, "peptide" is used to refer to a sequence of 20 or less amino acids and "polypeptide" is used to refer to a sequence of greater than 50 amino acids. However, in the present application, for the sake of clarity and ease of reading, the "proteins", "polypeptides" and "peptides" of the invention are all referred to as "proteins". Proteins, polypeptides and peptides may be purified, produced by a recombinant process (i.e., expression of exogenous nucleic acid encoding the peptide, polypeptide or protein in an organism, host cell, or cell-free system) or produced by chemical synthesis.

The compounds or compositions described herein encompass compounds wherein X is a bond and Y is a reporter molecule. Any appropriate reporter molecule may be used. A reporter molecule is understood to be any moiety that facilitates detection using a method for detection, whereby such a reporter molecule may be a fluorophore such as fluorescein, a chromophore, a radioactive tracer, a specific isotope, a diagnostic marker, or a hapten, wherein the hapten is preferably biotin. In one example, the reporter molecule comprises a radioactively labeled amino acid, e.g. a tritium-labelled amino acid. The reporter molecule may be for in vivo, ex vivo, or in vitro diagnostic purposes. Commonly used imaging labels, radio labels or fluorescent labels such as FAM, FITC, VIC, Cy3, Cy5, Cy5.5 and the like, or green fluorescent protein (GFP), may be used as reporter molecules.

Suitable linkers are known in the art. The linker may be selected from: -C(O)-C₂-C₁₀-alkylene-, -C₂-C₁₀-alkylene-NR¹-(e.g. C₂-C₁₀-alkyl-NH-), -C₂-C₁₀-alkylene-O-, -C₂-C₁₀-alkylene-S-, -C₂-C₁₀-alkylene-C(O)O-, -C₂-C₁₀-alkylene-C(O)NH-, polyethylene glycol, and an amino acid or peptide,. The linker may comprise two or more of -C(O)-C₂-C₁₀-alkylene-, -C₂-C₁₀-alkylene-NR¹-(e.g. C₂-C₁₀-alkyl-NH-), -C₂-C₁₀-alkylene-O-, -C₂-C₁₀-alkylene-S-, -C₂-C₁₀-alkylene-C(O)O-, -C₂-C₁₀-alkylene-C(O)O-, -C₂-C₁₀-alkylene-C(O)NH-, polyethylene glycol, and an amino acid or peptide.

It may be that the linker is selected from: -C₂-C₁₀-alkylene-NH-, -C₂-C₁₀-alkylene-O-, and -C₂-C₁₀-alkylene-S-. It may be that the linker is -C₂-C₁₀-alkylene-NH-; optionally -C₄-C₈-alkylene-NH-; further optionally -C₆-alkylene-NH-.

The term "Cₓ-C_{y}-alkylene" refers to a bivalent linear saturated hydrocarbon chain having x to y carbons. The alkylene groups are typically unsubstituted but in certain circumstances they may substituted by one or more substituents, e.g. C₁-C₄-alkyl groups or fluoro.

In some examples, the linker is a linker involving covalent chemical coupling involving e.g. alkyl chains with reactive groups e.g. amino-, succinimide-, thiol-, carboxyl-.

### Kits

The compounds provided herein may be part of a kit, preferably for use in the methods described herein. Kits are therefore provided comprising a compound as described herein and optionally a leaflet with instructions on how to use the compound in a method described herein. The kit may further comprise anti-human IgG detection antibodies and/or nanobodies attached to (e.g. conjugated to) a detectable label. The compound of R-X-Y within the kit may be such that R is immobilized to a solid support as defined elsewhere herein.

### Methods

The compounds described herein have particular utility in a method of detecting an anti-*Schistosoma* antibody in a biological sample obtained from a subject (in particular, detecting an antibody that specifically binds to CAA). Methods described herein that detect the presence of the antibody in a biological sample obtained from a subject may be used to detect (e.g. diagnose) a current or prior *Schistosoma* infection in the subject. Accordingly, a method of detecting an anti-Schistosoma antibody (in particular an antibody that specifically binds to CAA) in a biological sample obtained from a subject as described herein may also be referred to as a method of detecting (e.g. diagnosing) a current or prior *Schistosoma* infection in the subject. This is because the presence of the antibody in the biological sample is indicative of a current or prior *Schistosoma* infection in the subject.

The compounds described herein are also particularly useful when monitoring a subject to determine if they have contracted a *Schistosoma* infection. In this context, "contracting" a disease means to catch or acquire an illness through the exposure to a contagious pathogen. In such methods, two biological samples are tested (referred to as a "first biological sample" and a "second biological sample"), where the second sample is obtained from the subject at a later time point than the first. This method is particularly useful when monitoring a subject, for example, before and after (or during) travel to a location where *Schistosoma* infection is prevalent and/or endemic, as testing the same subject at two time intervals eliminates inter-person variability. The method can advantageously detect an increase in anti-Schistosoma antibody in the subject over time (from the first sample to the second sample), which would be indicative of the subject having contracted a *Schistosoma* infection.

This method is also particularly useful when monitoring a subject, for example, before and after (or during) suspected exposure to schistosome-infected water, as testing the same subject at two time intervals eliminates inter-person variability. The method can advantageously detect an increase in anti-*Schistosoma* antibody in the subject over time, (from the first sample to the second sample), which would be indicative of the subject having contracted a *Schistosoma* infection during that time period.

A method of monitoring a subject to determine if they have contracted a *Schistosoma* infection as described herein may also be referred to as a method of detecting (e.g. diagnosing) a *Schistosoma* infection in a subject.

Typically, the methods described herein are *in vitro* methods.

The invention therefore provides a method of detecting an anti-*Schistosoma* antibody in a biological sample obtained from a subject, the method comprising:
a) contacting the sample with a compound R-X-Y, wherein R is according to Formula I: 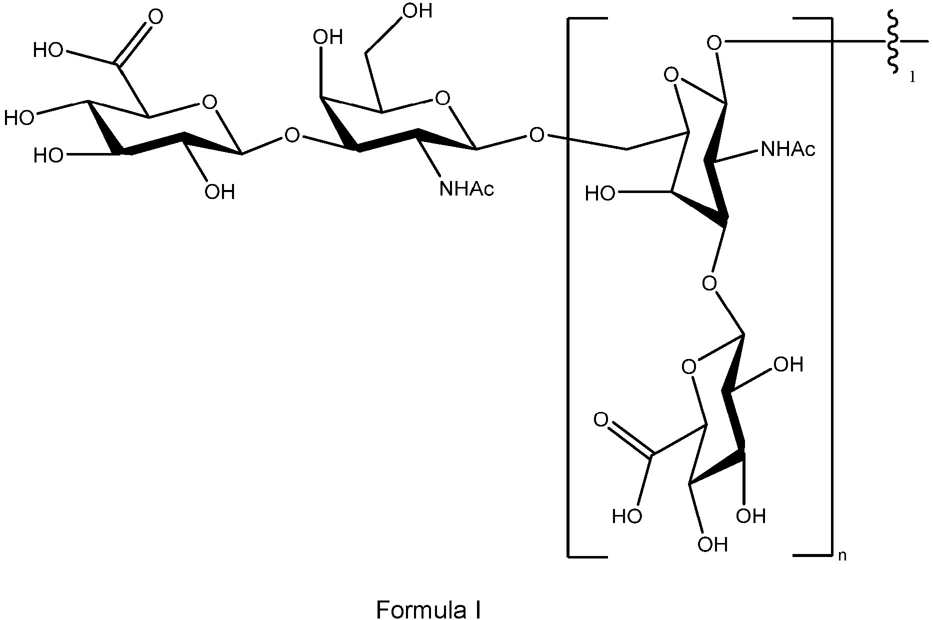 wherein 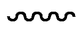 1 denotes the point of attachment of R to X, wherein X is selected from: a bond and a linker; and wherein Y is selected from the group consisting of H, a reporter molecule, a carrier molecule, and a solid support; and wherein n is a whole integer selected from 2 to 19; and
b) determining if binding between the sample and the compound occurs;
wherein binding between the sample and the compound is indicative of the presence of the antibody in the sample.

In a particular example, the anti-*Schistosoma* antibody is an antibody that specifically binds to CAA.

In one example, the biological sample may be obtained from a subject that is at risk of having or at risk of contracting a *Schistosoma* infection.

In one example, the biological sample may be obtained from a subject that has one or more symptoms indicative of a *Schistosoma* infection.

For example, the sample may be obtained from the subject at least 1 day after the subject first developed one or more symptoms indicative of a *Schistosoma* infection. In some examples, the sample may be obtained from the subject at least 2 days, at least 3 days or at least 4 days after the subject first developed one or more symptoms indicative of a *Schistosoma* infection. In some examples, the sample may be obtained from the subject at least 5 days, at least 6 days or at least 7 days after the subject first developed one or more symptoms indicative of a *Schistosoma* infection.

For example, the sample may be obtained from the subject at least 1 week after the subject first developed one or more symptoms indicative of a *Schistosoma* infection. In some examples, the sample may be obtained from the subject at least 2 weeks, at least 3 weeks or at least 4 weeks after the subject first developed one or more symptoms indicative of a *Schistosoma* infection.

In another example, the biological sample may be obtained from a subject that has no symptoms indicative of a *Schistosoma* infection (e.g. they are asymptomatic). In such cases, the biological sample may be obtained because the subject is considered to be at risk of having or at risk of contracting a *Schistosoma* infection, e.g. for one or more of the reasons provided elsewhere herein.

In one example, the biological sample may be obtained from a subject that has acute schistosomiasis. In other words, the methods provided herein may be used to monitor for acute schistosomiasis in a subject, e.g. may be used to diagnose acute schistosomiasis in a subject. As used herein, "acute" schistosomiasis refers to the disease phase presenting with typical symptoms/signs associated with a primary or recent primary *Schistosoma* infection such as but not limited to cercarial dermatitis (shortly after parasites penetrate the skin and the following typically presenting within 2 weeks to 3 months after infection), Katayama fever, eosinophilia, and transient pulmonary infiltrates.

In one example, the biological sample may be obtained from a subject that has a primary *Schistosoma* infection. In other words, the methods provided herein may be used to monitor for primary *Schistosoma* infection in a subject, e.g. may be used to diagnose primary *Schistosoma* infection in a subject. As used herein, "primary" *Schistosoma* infection refers to the first *Schistosoma* infection of the subject i.e. prior to this infection, the subject had not been infected with *Schistosoma.*

The invention also provides a method of monitoring a subject to determine if they have contracted a *Schistosoma* infection, the method comprising:
a) contacting a first biological sample obtained from the subject at a first time point with a compound R-X-Y, wherein R is according to Formula I: 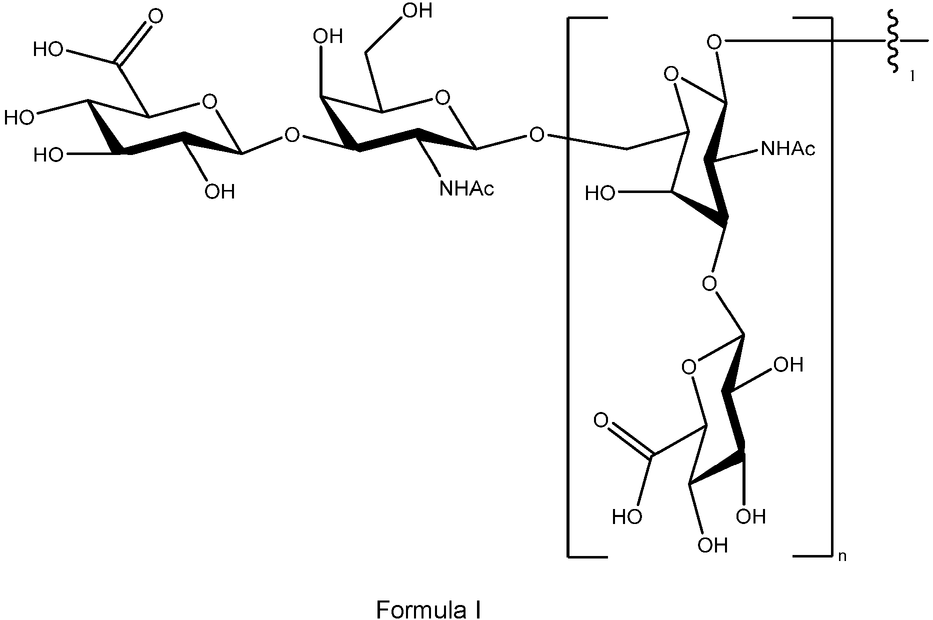 wherein 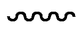 1 denotes the point of attachment of R to X, wherein X is selected from: a bond and a linker; and wherein Y is selected from the group consisting of: H, a reporter molecule, a carrier molecule, and a solid support and wherein n is a whole integer selected from 2 to 19; and determining if binding between the compound and the first sample occurs;
b) contacting a second biological sample with the compound, wherein the second sample is obtained from the subject at a later time point than the first sample, and determining if binding between the compound and the second sample occurs;
c) comparing the binding determined in step b) with the binding determined in step a), wherein an increase in b) as compared to a) is indicative of the subject having contracted the infection.

As would be clear to a person of skill in the art, the method of monitoring a subject to determine if they have contracted a *Schistosoma* infection provided herein may be used to determine that the subject contracted the infection between the first time point (at which the first biological sample was obtained) and the later time point (at which the second biological sample was obtained).

In a particular example, the method of monitoring a subject to determine if they have contracted a *Schistosoma* infection determines the presence of an anti-*Schistosoma* antibody in the first biological sample and the second biological sample. In a specific example, the anti- *Schistosoma* antibody is an antibody that specifically binds to CAA. In this particular example, the method of monitoring a subject to determine if they have contracted a *Schistosoma* infection provided herein may be used to determine that the subject had induced or increased their production of anti-*Schistosoma* antibodies (e.g. antibodies that specifically bind to CAA) between the first time point (at which the first biological sample was obtained) and the later time point (at which the second biological sample was obtained). In other words, the method may be used to determine the presence (induction) of an immune response against *Schistosoma* between the first time point (at which the first biological sample was obtained) and the later time point (at which the second biological sample was obtained).

The first biological sample and second biological sample may be obtained from the subject at any suitable time, and with any suitable time interval between them.

For example, the first biological sample may be obtained from the subject before the subject is at risk of contracting a *Schistosoma* infection. It may also/alternatively be obtained from the subject before the subject has symptoms that are indicative of the *Schistosoma* infection. For example, the first biological sample may be obtained just after the subject contracted the infection, but before an immune response (antibody response) against *Schistosoma* was detectable in the subject.

The second biological sample may be obtained from the subject at any suitable time point after the first biological sample. For example, the time period between the first and second sample may be at least 1 day. In some examples, the time period between the first and second sample may be at least 2 days, at least 3 days or at least 4 days. In some examples, the time period between the first and second sample may be at least 5 days, at least 6 days or at least 7 days.

For example, the time period between the first and second sample may be at least 1 week. In some examples, the time period between the first and second sample may be at least 2 weeks, at least 3 weeks or at least 4 weeks.

In one example, the second biological sample may be obtained from a subject that is at risk of having or at risk of contracting a *Schistosoma* infection.

In one example, the second biological sample may be obtained from a subject that has one or more symptoms indicative of a *Schistosoma* infection.

For example, the second biological sample may be obtained from the subject at least 1 day after the subject first developed one or more symptoms indicative of a *Schistosoma* infection. In some examples, the second sample may be obtained from the subject at least 2 days, at least 3 days or at least 4 days after the subject first developed one or more symptoms indicative of a *Schistosoma* infection. In some examples, the second sample may be obtained from the subject at least 5 days, at least 6 days or at least 7 days after the subject first developed one or more symptoms indicative of a *Schistosoma* infection.

For example, the second biological sample may be obtained from the subject at least 1 week after the subject first developed one or more symptoms indicative of a *Schistosoma* infection. In some examples, the second sample may be obtained from the subject at least 2 weeks, at least 3 weeks or at least 4 weeks after the subject first developed one or more symptoms indicative of a *Schistosoma* infection.

In another example, the second biological sample may be obtained from a subject that has no symptoms indicative of a *Schistosoma* infection (e.g. they are asymptomatic). In such cases, the second biological sample may be obtained because the subject is considered to be at risk of having or at risk of contracting a *Schistosoma* infection, e.g. for one or more of the reasons provided elsewhere herein.

In one example, the second biological sample may be obtained from a subject that has acute schistosomiasis. In other words, the methods provided herein may be used to monitor for acute schistosomiasis in a subject, e.g. may be used to diagnose acute schistosomiasis in a subject. Acute schistosomiasis is defined elsewhere herein.

In one example, the second biological sample may be obtained from a subject that has a primary *Schistosoma* infection. In other words, the methods provided herein may be used to monitor for primary *Schistosoma* infection in a subject, e.g. may be used to diagnose primary *Schistosoma* infection in a subject. Primary *Schistosoma* infection is defined elsewhere herein.

In the context of all of the methods provided herein, a subject may be considered to be at risk of having or contracting a *Schistosoma* infection if they have been to a location *Schistosoma* infection is prevalent and/or endemic. In another example, a subject may be considered to be at risk of having or contracting a *Schistosoma* infection if they have been exposed to or are being exposed to water that is suspected to Schistosome-infected water.

Symptoms of schistosomiasis are caused by inflammatory processes to the worms and eggs. Many low intensity infections are asymptomatic. There are three distinct phases of clinical disease progression: acute infection, established active infection and late chronic infection. Cercarial dermatitis, a local cutaneous hypersensitivity reaction following skin penetration by cercariae may occur and appears as a skin rash (e.g. small, itchy maculopapular lesions). Acute schistosomiasis (also known as Katayama fever) is a systemic hypersensitivity reaction that may occur weeks to months after the initial infection. Symptoms/signs/indications that are indicative of acute schistosomiasis include a skin rash, fever, headache, myalgia, general malaise, diarrhoea, non-productive cough, muscle pain, joint pain, abdominal tenderness or pain, eosinophilia, urticaria, angiooedema, transient pulmonary infiltrates and parasite eggs in stool and/or urine as well as genital fluids. As disease progresses to established active and late chronic infection symptoms/signs/indications include but are not limited to dyspnea, bloody diarrhoea, hepatosplenomegaly, haematuria, pseudopolyps, periportal fibrosis, hypertension, kidney nephrosis, oesophagal varices, ascites, and fertility problems. Disease severity is related to intensity of infection and duration. .

More specifically, intestinal schistosomiasis (caused by *S. intercalatum, S. japonicum, S. mansoni* or *S*. *mekongi*) typically presents with abdominal pain, bloody stool (haematochezia), diarrhoea and in advanced cases enlargement of the liver and spleen (hepatosplenomegaly). Urogenital schistosomiasis (UGS, caused by *S*. *haematobium*) typically presents with blood in urine (hematuria) and bladder fibrosis, ureter fibrosis and kidney damage in advanced cases.

Female Genital Schistosomiasis (FGS) is a disease manifestation of schistosomiasis caused by the *Schistosoma haematobium,* a waterborne parasite that affects both the urinary and genital tract of infected individuals. Symptoms of FGS include blood in urine, abdominal and pelvic pain, genital itching or burning, vaginal discharge, pain or difficulty urinating, pain and/or bleeding during or after sexual intercourse.

Male genital schistosomiasis (MGS) is an often-overlooked chronic consequence of urogenital schistosomiasis associated with *Schistosoma haematobium* eggs and associated pathologies in the genital system of afflicted men. Men suffering from MGS in endemic areas experience pelvic, coital or ejaculatory pain, abnormal ejaculates, haemospermia, abnormal swelling of genital organs and infertility.

In one example, the methods of the invention are used to detect FGS or MGS.

In the methods described herein, the compounds of the invention are contacted with a biological sample. "Biological sample", refers to a sample obtained from a subject (also referred to as a an infected individual herein). A biological sample may comprise tissues and/or biological fluids. Such samples can be obtained in vitro, ex vivo or in vivo. As a non-limiting example, the biological sample may be selected from tissues, organs, cells, or any isolated fraction of a human subject. The biological sample may also be selected from blood, plasma, lymph, saliva, urine, stool, tears, sweat, sperm, vaginal fluids, or cerebrospinal fluid, synovial, pleural, peritoneal, or pericardial, and any fraction or extracts thereof.

Preferably, the biological sample is a biological fluid that comprises IgG, IgA and/or IgM antibodies. As is known in the art, in humans, IgG antibodies can be found in blood (e.g. serum, plasma, whole blood), saliva, urine, lymph fluid, cerebrospinal fluid and peritoneal fluid. Similarly, it is known that IgM antibodies are mainly found in blood (e.g. serum, plasma, whole blood) and lymph fluid, whereas IgA is found in blood (e.g. serum, plasma, whole blood), the lining of the respiratory tract, genital tracts and digestive system, as well as in saliva, tears, and breast milk.

In one example, the biological sample is selected from the group consisting of a blood sample, nasal sample, urine sample, genital fluid sample and a stool sample. For example, the blood sample may be a serum sample, plasma sample, or a whole blood sample.

As used herein, the term "whole-blood" is a broad term and is used in its ordinary sense and refers, without limitation, to blood that has been withdrawn from a patient but that has not been otherwise processed, e.g., it has not been haemolysed, lyophilized, centrifuged, or separated in any other manner, after being removed from the patient. Whole-blood may contain amounts of other fluids, such as interstitial fluid or intracellular fluid, which may enter the sample during the withdrawal process or are naturally present in the blood.

The term "serum", as used herein, refers to the whole blood component that results after blood clotting and removal of the resulting clot.

The term "plasma" as used herein refers to the liquid fraction obtained by removing the blood cells contained in whole blood.

The term "genital fluid" as used herein encompasses vaginal fluid and sperm.

In a specific example, the blood sample is a serum sample, as is used in the examples section below. A blood sample (e.g. serum sample) may be used when detecting any one of IgM, IgA or IgG.

In another example, the biological sample may be a nasal sample, e.g. a sample that is obtained by nasosorption sampling. A nasal sample may be particularly relevant when detecting the presence of IgA.

The biological sample can be obtained by any technique known in the art.

The biological sample may be pre-processed to preserve the integrity of the antibodies of interest and/or to make them more accessible for further analysis. The biological sample may for instance undergo centrifugation, purification, or other treatment steps to facilitate access to antibodies, and/or to concentrate them. The biological sample may also be pre-processed so as to limit or lower the presence of antibodies susceptible to react in a non-specific way with the polysaccharides of the invention. Any suitable pre-processing steps may be performed, which will be well known to a person of skill in the art.

The biological sample is obtained from any suitable subject. The subject may be any antibody-producing organism that is capable of being a host (or reservoir) for *Schistosoma.* For example, various animals such as cattle, dogs, cats, rodents, pigs, horses, and goats, serve as reservoirs for *S. japonicum,* and dogs for *S*. *mekongi.* As a further example, *S*. *mansoni* is also frequently recovered from wild primates in endemic areas but is considered primarily a human parasite and not a zoonosis. The subject may therefore be a mammal, for example a mammal selected from the group consisting of: primates (e.g. humans, apes, monkeys) cattle, dogs, cats, rodents, pigs, horses, and goats.

In a particular example, the subject is human. The invention has particular utility when monitoring for *Schistosoma haematobium, S. japonicum,* or *S*. *mansoni* infection in humans.

The terms "Schistosomatidae infection", *"Schistosoma* infection", "schistosome infection" "Schistosomiasis", "Bilharziasis" are used interchangeably herein to refer to disease caused by caused by blood trematodes (blood flukes) of the superfamily *Schistosomatidae.* This includes blood trematodes of sub-family Schistosomatinae., e.g. genus *Schistosoma.* The three main species infecting humans are *Schistosoma haematobium, S. japonicum,* and *S*. *mansoni.* Three other species, more localized geographically, are *S*. *mekongi, S. intercalatum,* and *S. guineensis.* There have also been a few reports of hybrid schistosomes of cattle origin (*S. haematobium, x S. bovis, x S. curassoni, x S. mattheei*) infecting humans.

In one example, the *Schistosoma* infection described herein may be caused by a trematode worm selected from the group consisting of: *Schistosoma mansoni, Schistosoma japonicum, Schistosoma mekongi, Schistosoma guineensis, Schistosoma intercalatum, and Schistosoma haematobium.*

The term "infection" as used herein refers to the presence of the pathogen (in this context, *Schistosoma*) in the subject. The infection may develop into a disease (in this context, schistosomiasis).

Schistosomiasis is a parasitic disease characterized as either intestinal or urogenital, depending on where the adult flukes are located. Four species can cause intestinal schistosomiasis (*S*. *intercalatum, S. japonicum, S. mansoni and S*. *mekongi*) while *S*. *haematobium* causes urogenital schistosomiasis.

Schistosoma eggs are eliminated with feces or urine, depending on species. Under appropriate conditions the eggs hatch and release miracidia, which swim and penetrate specific snail intermediate hosts. The stages in the snail include two generations of sporocysts and the production of cercariae. Upon release from the snail, the infective cercariae swim, penetrate the skin of the human host, and shed their forked tails, becoming schistosomulae. The schistosomulae migrate via venous circulation to lungs, then to the heart, and then develop in the liver, exiting the liver via the portal vein system when mature. Male and female adult worms copulate and reside in the mesenteric venules, the location of which varies by species (with some exceptions). For instance, *S*. *japonicum* is more frequently found in the superior mesenteric veins draining the small intestine, and *S*. *mansoni* occurs more often in the inferior mesenteric veins draining the large intestine. However, both species can occupy either location and are capable of moving between sites. *S. intercalatum* and *S. guineensis* also inhabit the inferior mesenteric plexus but lower in the bowel than *S*. *mansoni. S. haematobium* most often inhabits in the vesicular and pelvic venous plexus of the bladder, but it can also be found in the rectal venules and genitals. The females (size ranges from 7-28 mm, depending on species) deposit eggs in the small venules of the portal and perivesical systems. The eggs are moved progressively toward the lumen of the intestine (*S*. *mansoni, S. japonicum, S. mekongi, S. intercalatum*/*guineensis*) and of the bladder and ureters (S. *haematobium*), and are eliminated with feces, genital fluids or urine, respectively.

The methods provided herein are based on the binding of *anti-Schistosoma* antibodies to the compounds disclosed herein. By "antibody" or "antibodies", it is herein generally referred to immunoglobulins. When referring to antibodies which presence and/or quantity is to be determined according to the invention, the term "antibodies" refer to naturally occurring antibodies, that is to say antibodies that would be present in the biological sample from the subject as a product of the subject's immune response, as opposed to antibodies which would have been added to the biological sample and/or would have been injected to the patient. In this context, the term antibodies refer to antibodies of the IgA, IgD, IgE, IgG and IgM isotype. Preferably in the context of the invention, the antibodies which presence and/or quantity is to be determined according to the invention are of the IgG, IgM or IgA isotype. For the avoidance of doubt, reference to an antibody "isotype" encompasses all known subclasses - for example, reference to "IgG isotype" in the context of humans encompasses IgG1, IgG2, IgG3, IgG4 subclasses. Similarly, reference to "IgA isotype" in the context of humans encompasses IgA1 and IgA2 subclasses.

In the context of the invention, the term *"anti-Schistosoma* antibodies" refer to naturally occurring antibodies capable of binding to *Schistosoma* antigens.

By "capable of binding", used in reference to antibodies, it is herein referred to antibodies capable of binding to defined proteins or polysaccharides under the usual experimental conditions of immunogenic assays. Preferably, the antibodies are capable of binding specifically to CAA as defined herein. In the context of the invention, an antibody which "binds specifically" (or an antibody that specifically binds) to a defined antigen (e.g. CAA) forms or undergoes a physical association with it, in an amount and for a time to sufficient allow detection of the antibody-antigen complex. By "specifically" or "preferentially," it is meant that the antibody has a higher affinity for defined antigen than for other antigens, such as for instance other antigens contained in the biological sample. In the context of the invention, the term "affinity" when referring to antibodies, designate the strength with which said antibody binds to a defined antigen, or a part thereof, and is measured by the affinity constant between the antibody and its antigen (defined as 1/KD, wherein KD is the dissociation constant as classically defined) measurement of the reaction rate constants can be used to define an equilibrium or affinity constant (1/KD ). The affinity of an antibody for its target is thus inversely correlated to the dissociation constant, i.e. the smaller the KD value the greater the affinity of the antibody for its target. For example, the antibody can have an affinity for the defined antigen of at least about 1.5-fold, 2-fold, 2.5-fold, 3-fold, or higher than for other antigens in the sample. Such affinity or degree of specificity can be determined by a variety of routine procedures, including competitive binding assays. It should be understood that in the context of the invention, the compounds of the invention have been selected for the ability to bind specifically to anti-*Schistosoma* antibodies (particularly antibodies that specifically bind to CAA), that is to say the compounds of the invention have a greater affinity for *anti-Schistosoma* antibodies (particularly for antibodies that specifically bind to CAA), for other antibodies present in human biological samples.

When referring to antibodies used as biological tools in an assay or test, for instance as secondary and/or detection antibodies, the term "antibodies" refer to any type of antibody that would be fit for that purpose, independent of the species of origin or isotype.

Binding between the biological sample and the compounds of the invention is determined after the sample is contacted with the compound. As used therein, the term "contacting" refers to any suitable means for delivering, or exposing, a sample to the compound described herein so as to permit physical and/or chemical interaction between the sample and the compound (specifically R within the compound). Suitably, the interaction may be between the antibodies in the sample and the compound (specifically R within the compound). More suitably, the interaction may be specific binding of the antibodies in the sample to the compound (specifically R within the compound). The term "specific binding" as used herein refers to antibody binding to a predetermined antigen (which in the context of the present disclosure is the compound (specifically R within the compound) described herein) with greater affinity than for other antigens. Typically, the antibody binds with a dissociation constant (K_{D}) of 10⁻⁷ M or less, and binds to the compound (specifically R within the compound) described herein with a K_{D} that is at least two-fold less than its K_{D} for binding to a non-specific antigen (e.g., other specified compound) other than the predetermined compound described (i.e. according to Formula I).

In some instances, the term "contacting" refers to providing the compound described herein (e.g., suspended in a solution) directly to the sample. Alternatively, in some instances the term "contacting" refers to providing the sample to the compound described herein (e.g., attached to a solid support). The term "contacting" can further comprise mixing the sample with the compound by any means known in the art (e.g., vortexing, pipetting, and/or agitating). In some instances, the term "contacting" can further comprise incubating the sample together with the compound for a sufficient amount of time, e.g., to allow binding of the antibodies in the sample to the compound. The contact time can be of any length, depending on the binding affinities and/or concentrations of the compound and/or antibodies in the sample, concentrations of a detection reagent, and/or incubation condition (e.g., temperature). For example, the contact time can be reduced if the sample and compound are incubated at a higher temperature. In some embodiments, the contact time between the sample and the compound can be at least about 30 seconds, at least about 1 minute, at least about 5 minutes, at least about 10 minutes, at least about 15 minutes, at least about 30 minutes, at least about 1 hour, at least about 2 hours, at least about 3 hours, at least about 4 hours, at least about 6 hours, at least about 8 hours, at least about 10 hours, at least about 12 hours, at least about 24 hours, at least about 48 hours or longer. One of skill in the art can adjust the contact time accordingly.

After the sample has been contacted with the compound the methods described herein comprise determining if binding between the sample and the compound has occurred. As used herein, the phrase "determining if binding between the sample and the compound has occurred" refers to assessing the presence ("detecting") and/or amount ("quantifying") of binding. Methods for determining binding such as the enzyme-linked immunosorbent assay (ELISA), are well known in the art, as are described in Receptor Binding Techniques Methods in Molecular Biology. 106. ed. M. Keen. Humana Press, 1999; Brooks et al. (1998) Cell 92:391-400; Brooks et al. (1996) Cell 85:683-693; and Brooks et al. (1993) J. Cell. Biol. 122:1351-1359. It will be appreciated that determining if binding between the sample and the compound has occurred may be qualitative (i.e. determining the presence or absence of binding) and/or quantitative (i.e. determining the amount of binding and/or determining the amount of antibody).

As explained elsewhere in the present description, the present invention is based upon the inventors' identification and synthesis of compounds that are useful for the detection of antibodies against *Schistosoma* (particularly against CAA, i.e. anti-CAA antibodies) and/or for the diagnosis of *Schistosoma* infection in biological samples obtained from a human subject. The inventors have demonstrated that these compounds can be used to efficiently detect antibodies produced by the subject as part of the host immune response upon *Schistosoma* infection. Accordingly, the determination of a presence of binding between the compound and sample is indicative of the presence of antibodies in the sample that may specifically bind to the compounds of the invention. The presence such antibodies is in turn indicative of a current or prior *Schistosoma* infection in the subject. By the same token, the absence of such antibodies is indicative of the fact that the subject has not had a *Schistosoma* infection, or indicative of the fact that the subject has not recently had a *Schistosoma* infection (such that anti-CAA antibodies are no longer detectable in the subject). Suitably, anti-CAA antibodies may no longer be detectable after, for example, 6 months, 12 months, 24 months, or more post *Schistosoma* infection.

In some embodiments it may be desirable to compare the amount of binding determined in a biological sample to a reference value. Such a reference value may be for example a control (for example a negative control or positive control) or a standard curve. Suitably, the standard curve may provide a correspondence between the amount of binding to antibody concentration.

In addition to determining the presence (or absence) of binding, thus determining the presence (or absence) of anti-CAA antibodies, the step of determining if binding between the sample and the compound has occurred may also involve determining the amount of binding. It shall be appreciated by a person skilled in the art that the amount of binding may be dependent upon the concentration of anti-CAA antibodies in the sample. Therefore, the step of determining if binding between the sample and the compound has occurred may include a quantitative assessment of the amount of binding and/or a quantitative assessment of the amount of anti-CAA antibodies in the sample.

Thus, the step of determining if binding between the sample and the compound has occurred may also include quantitative assessment of the amount of anti-CAA antibodies in the sample. Merely by way of example, such a quantitative assessment may be particularly useful for determining if a subject that has previously had a *Schistosoma* infection has or has had a further (more recent) *Schistosoma* infection.

For certain methods described herein, a comparison may be made between the binding observed in one sample and that observed in another sample (or control/reference). The binding may be compared using any suitable method of assessing, evaluating or processing of data relating to binding. As mentioned elsewhere in the present disclosure, the data relating to binding may be qualitative (i.e. providing information regarding the presence or absence of binding). Additionally or alternatively, the data may be quantitative (i.e. providing information regarding the amount the binding and/or providing information regarding the concentration of the antibody in the sample). It will be appreciated that whether quantitative and/or qualitative data is compared may depend upon the subject.

Merely by way of example, if the subject is suspected to never have had a *Schistosoma* infection before, it may be desirable to compare qualitative binding data determined in step b) of the monitoring method provided herein relative to that determined in step a). In such an example, the presence of binding in step b) compared to an absence of binding in step a) may be indicative of the subject having contracted the infection between the first time point and later time point. In other words, comparing qualitative data relating to binding may be useful for monitoring if the subject has contracted a primary *Schistosoma* infection. In one example, the qualitative binding data determined in step b) of the monitoring method provided herein relative to that determined in step a) may relate to the binding data obtained for a specific antibody isotype or subclass only e.g. the method may be used to determine binding between IgG and the compound only, or more specifically between IgG1 and the compound only. Alternatively, it may be used to determine binding between two different antibodies and the compound simultaneously (e.g. between IgG and the compound, as well as IgA and the compound, simultaneously) using two distinct reporter assays.

By way of another example, if the subject is suspected to have had a *Schistosoma* infection in the past, it may be desirable to compare quantitative binding data determined in step b) of the monitoring method provided herein relative to binding determined in step a). In such an example, an increase of binding determined in step b) compared to binding determined in step a) may be indicative of the subject having contracted the infection again between the first time point and later time point. In other words, comparing quantitative data relating to binding may be useful for monitoring if the subject has contracted a further *Schistosoma* infection. In one example, the quantitative binding data determined in step b) of the monitoring method provided herein relative to that determined in step a) may relate to the binding data obtained for a specific antibody isotype or subclass only e.g. the method may be used to determine binding between IgG and the compound only, or more specifically between IgG1 and the compound only. Alternatively, it may be used to determine binding between two different antibodies and the compound simultaneously (e.g. between IgG and the compound, as well as IgA and the compound, simultaneously) using two distinct reporter assays.

In both of these examples, the comparison between binding in step b) compared to that in step a) indicates an increase in binding over the appropriate time period. Suitably, the increase may be statistically significant. Suitably, the increase may be by at least 10%, at least 20%, at least 30%, at least 40%, at least 50%, at least 60%, at least 70%, at least 80%, at least 90%, at least 100%, or greater.

The invention involves assays to determine whether an antibody as part of the host's immune response to infection with *Schistosoma* is present in a sample, and therefore does not preclude situations wherein no such antibody is present or detected is the sample. In the context of the invention, the presence and/or quantity of the antibodies can be determined by determining binding between such antibodies and the compounds of the invention, using any appropriate technique known in the field, including but not limited to bead-based assays, lateral flow devices, microarray assays, immunoblotting assays and enzyme-linked immunosorbent assays (ELISA). Preferably, the presence and/or quantity of antibodies in the biological sample is assayed by immunoassay, more preferably antigen-based immunoassay. Such assays typically allow for the quantitative detection of antigen/antibody complex formation.

The term " immunoassay" refers to an assay that is meant to detect or measure an analyte based on the interaction between an immunological reagent, usually an antibody, and its ligand. For the sake of clarity, the terms "antigen-based immunoassay" herein refers to an immunoassay wherein one or several antigens are used as reagents to detect and/or quantify the analyte which is an antibody. Typically, in the case of an antigen-based immunoassay, at least one antigen of interest is immobilized on a solid support, and the sample to be tested is brought into direct contact under conditions such that any specific antibodies in the sample bind to the immobilized antigen. If such specific antibodies capable of binding to the antigen of interest are present in the sample, a complex is formed, which presence and/or quantity can be detected either by direct or indirect means, such as for instance by secondary antibodies, also called detection antibodies.

The term "secondary antibodies" refers to antibodies capable of binding to the anti-Schistosoma antibodies present in the biological sample. It will be immediately apparent that anti-human IgG antibodies can be used as secondary antibodies and enable the detection of the complex formed between the polysaccharides of the invention and any IgG anti -Schistosoma antibodies present in the sample to be assayed. Similarly, anti-human IgA antibodies or anti-human IgM antibodies can be used as secondary antibodies to enable the detection of the complex formed between the polysaccharides of the invention and any IgA or IgM anti -Schistosoma antibodies present in the sample respectively.

The secondary antibody may be conjugated to a detectable label. By "detectable label" it is herein referred to a molecule or composition bound to an analyte, analyte analog, detector reagent, or binding partner that is detectable by spectroscopic, photochemical, biochemical, immunochemical, electrical, optical or chemical means. Examples of labels, including enzymes, colloidal gold particles, colored latex particles, have been disclosed for instance in US 4,275,149, US 4,313,734, US 4,373,932 and US 4,954,452. Additional examples of useful labels include, without limitation, radioactive isotopes, co-factors, ligands, chemiluminescent or fluorescent agents, protein-adsorbed silver particles, protein-adsorbed iron particles, protein- adsorbed copper particles, protein-adsorbed selenium particles, protein-adsorbed sulphur particles, protein-adsorbed tellurium particles, protein-adsorbed carbon particles, and protein-coupled dye sacs. The attachment of a compound (e g., a detector reagent) to a label can be through covalent bonds, adsorption processes, hydrophobic and/or electrostatic bonds, as in chelates and the like, or combinations of these bonds and interactions and/or may involve a linking group. Preferably, the detectable label is chosen in the list consisting of a fluorophore, chemiluminescent and radioactive label. Examples of suitable labels include colloidal gold, a fluorophore (such as for instance a fluorescent dye such as FITC or Texas Red, a fluorescent protein such as GFP, or a nanocrystal such as Qdot probes) or an enzyme (such as for instance horseradish peroxidase (HRP)), alkaline phosphatase (AP) or P-galactosidase), all commonly used in immunoassays.

Standard solid phase ELISA and lateral flow immunoassay are quantitative immunoassays which can be used to perform either antigen-based immunoassays or antibody-based immunoassays and are particularly useful in determining the quantity or concentration of a protein or antibody from a variety of patient samples. As is common practice in the field, the person skilled in the art may set the conditions of the immunoassay so as to improve the sensitivity and specificity of the immunoassay. These techniques are well known.

By "sensitivity", in reference to a test, assay or diagnosis, it is herein referred to the test's ability to detect the proportion of true positive subjects with the disease in a total group of subjects with the disease. Hence, it relates to the potential of a test to identify subjects with the disease.

By "specificity", in reference to a test, assay or diagnosis, it is herein referred to the test's ability to correctly detect the proportion of subjects without the disease with negative test result in total of subjects without disease. In other words, specificity represents the probability of a negative test result in a subject without the disease. Therefore, specificity relates to the aspect of diagnostic accuracy that describes the test ability to identify subjects without the disease, i.e. to exclude the condition of interest.

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper
Collins Dictionary of Biology, Harper Perennial, NY (1991) provide those of skill in the art with a general dictionary of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a", "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

Aspects of the invention are demonstrated by the following non-limiting examples.

### EXAMPLES

### Materials and methods

### CAA synthesis

The following methods were used by the inventors to generate the polysaccharide compounds described herein. Compounds 30-33 are the 2,4,6 and 8-mers described elsewhere herein. Compound 35 is the 10-mer described elsewhere herein. Compounds 1 to 29, and 34 are intermediate products produced during the synthesis. Citation of [8] below refers to "M.R. Harvey. PhD Thesis. Synthesis and application of glycans unique to S. mansoni".

### 2-deoxy-3-O-β-D-glucoronic acid-2-amino-α/β-D-galactopyranose (1)

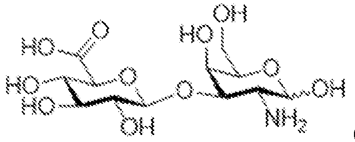

Chondroitin A trisodium salt (50 g) was suspended in H₂O (500 mL), amberlite DOWEX H⁺ resin was added and the flask was attached to the rotavap (40°C). When all chondroitin was dissolved (pH 1.5) the mixture was filtered and was washed with H₂O (400 mL) and transferred to a two-neck flask. The volume was adjusted to a total of 970 mL. Concentrated H₂SO₄ (28 mL) was added and the reaction mixture was refluxed for 6 hours. The solution was air cooled to RT. Ba(OH)₂ (~ 165 g) was added portion wise while stirring vigorously until the pH reached 3.5. The solids were left to subside overnight. The suspension was filtered over celite and concentrated to approximately 500 mL. The solution was slowly applied to a DOWEX H⁺ resin column (500 mL settled volume). The column was washed with H₂O (1L), AcOH/H₂O (3:1, 1L) and 1M HCl until no more ninhydrin positive material came off (±3L). The ninhydrin positive fractions were combined and concentrated yielding unprotected disaccharide 1.

### 2-deoxy-3-O-(methyl β-D-glucoronic acid)-2-amino-α/β-D-galactopyranose (2)

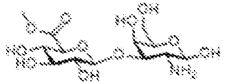

Disaccharide **1** was dissolved in dry HCl in methanol (500 mL, 0.02M HCl) and left to stir at 4°C for 72 hours. The volatiles were evaporated and the slurry was co-evaporated with EtOH twice to give **2** as a light brown solid (27 g). The product was used unpurified in the next step.

### 2-deoxy-3-O-(methyl β-D-glucoronic acid)-2-(2,2,2-trichloroacetamido-α/β-D-galactopyranose (3)

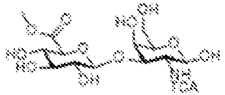

Crude disaccharide **2** (27 g) was suspended in pyridine (300 mL) and cooled to 0°C. Trichlororacetylchloride (84 mL) was slowly added via a dropfunnel and the reaction mixture was left to stir for 2h at RT. The reaction was stopped by slow addition of H₂O (25 mL). The reaction mixture was then diluted with DCM (1L) and quickly washed with H₂O (2x) and brine (1x), dried over MgSO₄ and filtered. The DCM was evaporated leaving the pyridine in the flask. MeOH (300 mL) and DCM (300 mL) were added to the pyridine residue and the mixture was left overnight. All volatiles were evaporated and the remaining compound was dryloaded on celite. The crude was purified by silicagel chromatography (DCM:MeOH, 19:1 → 4:1) giving **3** (16.1 g, 31.3 mmol). The spectral data was in accordance with those reported in literature.^{[8]}

### 1-O-Acetyl-4,6-O-benzylidene-2-deoxy-3-O-(methyl 2,3,4-tri-O-acetyl-β-D-glucoronic acid)-2-(2,2,2-trichloroacetamido-α/β-D-galactopyranose (5)

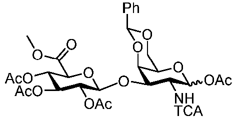

Disaccharide **3** (16.1 g, 31.3 mmol, 1.0 eq.) was dissolved in benzaldehyde (120 mL) and TFA (6mL) and stirred at RT under inert atmosphere for 24h. After this the reaction mixture was cooled to 0°C and pyridine (120 mL) and Ac₂O (75 mL) and NaOAc (10 g, 120 mmol, 4 eq.) were added subsequently. This mixture was left to stir for an additional 24 hours at RT. The resulting dark brown solution was poured into ice water (1L) and stirred for 2h after which it was extracted twice with EtOAc (2x 1L) and washed with sat. aq. NaHCO₃ (3x), aq. HCl (1M, 3x) and brine (1x). The brown organic layer was dried over MgSO₄, filtered and concentrated. The brown slurry was then purified by silicagel chromatography (PE:EtOAc, 1:1→ 1:4) giving the title compound as an off white solid (12.4 g, 16 mmol) in 52% yield. The spectral data was in accordance with those reported in literature.^{[8]}

### 4,6-O-benzylidene-2-deoxy-3-O-(methyl 2,3,4-tri-O-acetyl-β-D-glucoronic acid)-2-(2,2,2-trichloroacetamido-α/β-D-galactopyranose (6)

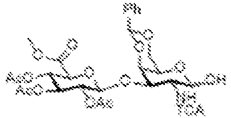

Acetylated **5** (6.4 g, 8.3 mmol, 1.0 eq.) was dissolved in DMF (55 mL, 0.15M) and hydrazine acetate (1.15 g, 12.5 mmol, 1.5 eq.) was added. The reaction mixture was left to stir at RT under nitrogen atmosphere for 2h, after which time it was diluted in EtOAc. The organic layer was washed with H₂O (3x) and brine (2x), before drying over MgSO₄, filtration and concentration *in vacuo.* The brown foam was purified by silicagel chromatography (PE:EtOAc, 1:1 → 1:4) giving **6** as a pale yellow foam in 71% yield (4.27 g, 5.86 mmol). The spectral data was in accordance with those reported in literature.^{[8]}

### Trichloroacetamido 4,6-O-benzylidene-1,2-deoxy-3-O-(methyl 2,3,4-tri-O-acetyl-β-D-glucoronic acid)-2-(2,2,2-trichloroacetamido-α-D-galactopyranose (7)

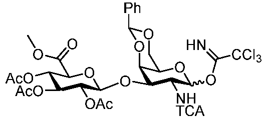

Hemi acetal **6** (4.25 g, 5.83 mmol, 1.0 eq.) was dissolved in dry DCM (40 mL, 0.15M). Trichloroacetonitrile 5.85 mL, 58.3 mmol, 10 eq.) and DBU (0.13 mL, 0.87 mmol, 0.15 eq.) were added subsequently and the reaction was left to stir at RT under inert atmosphere. After 30 min TLC analysis (PE:EtOAc 1:1) showed full conversion to a higher running spot and the reaction mixture was concentrated. The brown slurry was purified by silicagel chromatography (PE:EtOAc (+ 0.2% Et₃N), 4:1 → 1:1) giving the title compound (3.5 g, 4.0 mmol) in a yield of 69%. The spectral data was in accordance with those reported in literature.^{[8]}

### 6-azidohexyl 4,6-O-benzylidene-2-deoxy-3-O-(methyl 2,3,4-tri-O-acetyl-β-D-glucoronic acid)-2-(2,2,2-trichloroacetamido-β-D-galactopyranose (8)

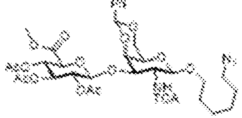

Donor **7** (1.74 g, 2.0 mmol, 1.0 eq.) and 6-azidohexan-1-ol (0.46 g, 3.2 mmol, 1.6 eq.) were co-evaporated thrice together with dry toluene and dissolved in dry DCM (15 mL, 0.15M). Flamedried molecular sieves (4Å) were added and the solution was cooled to -60°C and stirred at that temperature for 1h. BF₃·OEt₂ (0.2 mL, 1.0M in toluene, 0.1 eq.) was added and the reaction was allowed to warm to -40°C. After 90 min TLC analysis showed full consumption of the donor and the reaction was quenched by addition of Et₃N (0.1 mL). The cloudy mixture was filtered and concentrated. The yellow oil was purified by silicagel chromatography (PE:EtOAc 3:2) giving target compound **8** in 68% yield (1.16 g, 1.36 mmol). ¹H NMR (CDCl₃, 400 MHz): δ = 7.54 (d, 2H, *J*=6.7 Hz, Ph), 7.36 (q, 3H, *J*=7.9, 7.1 Hz, Ph), 7.09 (d, 1H, *J*=6.2 Hz, NH), 5.59 (s, 1H, CHPh), 5.23 (t, 1H, *J*=9.5 Hz, H-4'), 5.16 (t, 1H, *J*=8.9 Hz, H-3'), 5.09 - 5.00 (m, 2H, H-1, H-2'), 4.93 (d, 1H, *J*=7.6 Hz, H-1'), 4.68 (dd, 1H, *J*=11.1, 3.1 Hz, H-3), 4.44 (d, 1H, *J*=3.3 Hz, H-4), 4.33 (d, 1H, *J*=12.3 Hz, H-6), 4.09 (d, 1H, *J*=12.2 Hz, H-6), 4.02 (d, 1H, *J*=9.7 Hz, H-5'), 3.93 (dt, 1H, *J*=9.5, 6.3 Hz, OCH₂), 3.81 - 3.74 (m, 1H, H-2), 3.72 (s, 3H, OMe), 3.51 (s, 1H, H-5), 3.46 (dt, 1H, *J*=9.7, 6.9 Hz, OCH₂), 3.24 (t, 2H, J=6.9 Hz, CH₂N₃), 2.01 (s, 6H, CH₃, Ac), 1.99 (s, 3H, CH₃, Ac), 1.62 - 1.52 (m, 4H, CH₂, hexyl), 1.38 - 1.32 (m, 4H, CH₂, hexyl) ppm. ¹³C-APT NMR (CDCl₃, 101 MHz) δ 170.2, 169.5, 169.3 (C=O, Ac), 167.2 (C=O, CO₂Me), 162.2 (C=O, TCA), 137.8, 128.9, 128.2, 126.3 (Ph), 100.7 (CHPh), 100.2 (C-1'), 98.7 (C-1), 75.9 (C-4), 74.1 (C-3), 72.5 (C-3'), 72.1 (C-5'), 71.4 (C-2'), 69.9 (OCH₂), 69.2 (C-6), 69.1 (C-4'), 66.7 (C-5), 55.5 (C-2), 53.0 (OMe), 51.5 (CH₂N₃), 29.4, 28.9, 26.6, 25.7 (CH₂, hexyl), 20.9, 20.7, 20.6 (CH₃, Ac) ppm. [M+NH₄]⁺ for C₃₄H₄₃Cl₃N₄O₁₅ calculated: 870.21240, found 870.21288.

### 6-azidohexyl 4-O-benzyl-2-deoxy-3-O-(methyl 2,3,4-tri-O-acetyl-β-D-glucoronic acid)-2-(2,2,2-trichloroacetamido-β-D-galactopyranose (9)

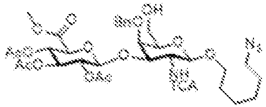

*From disaccharide **8:*** Disaccharide **8** (0.91 g, 1.06 mmol, 1.0 eq.) was dissolved in dry DCM (11 mL, 0.1M) and cooled to -78°C and stirred for 2h at that temperature. Triethylsilane (0.51 mL, 3.19 mmol, 3.0 eq.) and PhBCl₂ (0.41 mL, 3.19 mmol, 3.0 eq.) were added subsequently. After 2h TLC analysis (PE:EtOAc 2:3) showed full conversion to a lower running spot. Triethylamine ( 1 mL) and methanol (1 mL) were added and the reaction mixture was diluted in CHCl₃ (100 mL). The organic layer was washed with sat. aq. NaHCO₃ (2x) and brine (1x), dried over MgSO₄, filtered and concentrated. The crude yellow oil was purified using silicagel chromatography (PE:EtOAc, 1:1 → 2:3) giving the title compound as a white solid (0.61 g, 0.71 mmol, 67%).

*From disaccharide **20**:*
Disaccharide **20** (0.51 g, 0.53 mmol, 1.0 eq.) was dissolved in a mixture of pyridine/AcOH (5 mL, 01M, 4/1, v/v) and cooled to 0°C. Hydrazine acetate (0.24 g, 2.65 mmol, 5.0 eq.) was added and the reaction was stirred for 30 min. Acetone (0.5 mL) was added and the mixture was stirred for an additional 10 min., after which it was diluted in EtOAc and washed with aq. HCl (1M), sat. aq. NaHCO₃, and brine. The organic layer was dried over MgSO₄, filtered and concentrated. No further purification was required. The title compound was obtained as a white solid (0.34 g, 0.40 mmol, 75%). ¹H NMR (CDCl₃, 400 MHz): δ = 7.47 - 7.42 (m, 2H, arom.), 7.40 - 7.29 (m, 3H, arom.), 7.08 (d, 1H, *J*=6.9 Hz, NH), 5.28 - 5.17 (m, 2H, H-3', H-4'), 5.13 - 5.04 (m, 1H, H-2'), 4.91 (d, 1H, *J*=11.6 Hz, CH₂Bn), 4.83 (d, 1H, *J*=8.3 Hz, H-1), 4.74 (d, 1H, *J*=7.9 Hz, H-1'), 4.67 (d, 1H, *J*=11.7 Hz, CH₂Bn), 4.62 (dd, 1H, *J=11.0,* 2.9 Hz, H-3), 4.08 (d, 1H, *J*=9.7 Hz, H-5'), 4.01 (d, 1H, *J*=2.7 Hz, H-4), 3.91 - 3.82 (m, 1H, OCH₂), 3.76 (s, 3H, OMe), 3.74 - 3.64 (m, 2H, H-2, H-6), 3.53 - 3.38 (m, 3H, H-5, H-6, OCH₂), 3.24 (t, 2H, *J*=6.9 Hz, CH₂N₃), 2.07 (s, 3H, CH₃, Ac), 2.04 (s, 3H, CH₃, Ac), 2.01 (s, 3H, CH₃, Ac), 1.55 (m, 4H, CH₂, hexyl), 1.38 - 1.31 (m, 4H, CH₂, hexyl) ppm. ¹³C-APT NMR (CDCl₃, 101 MHz) δ 170.1, 169.6, 169.4 (C=O, Ac), 167.0 (C=O, CO₂Me), 162.3 (C=O, TCA), 138.0, 129.4, 128.6, 128.2 (arom.), 101.2 (C-1'), 98.8 (C-1), 92.4 (C_{q}, TCA), 77.3 (C-3), 74.6 C-5), 74.6 (CH₂Bn), 74.4 (C-4), 72.3 (C-5'), 72.1 (C-3'), 71.3 (C-2'), 69.9 (OCH₂), 69.5 (C-4'), 61.8 (C-6), 56.3 (C-2), 53.0 (OMe), 51.4 (CH₂N₃), 29.5, 28.8, 26.6, 25.7 (CH₂, hexyl), 20.9, 20.7, 20.6 (CH₃, Ac) ppm. [M+NH₄]⁺ for C₃₄H₄₅Cl₃N₄O₁₅ calculated: 872.22798, found 872.22853.

### 2-napthylmethyl 4,6-O-benzylidene-2-deoxy-3-O-(methyl 2,3,4-tri-O-acetyl-β-D-glucuronic acid)-2-(2,2,2-trichloroacetamido-β-D-galactopyranose (10)

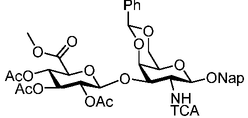

Donor **7** (1.76 g, 2.0 mmol, 1.0 eq.) and 20naphthalenemethanol (0.51 g, 3.2 mmol, 1.6 eq.) were co-evaporated thrice together with dry toluene and dissolved in dry DCM (15 mL, 0.15M). Flamedried molecular sieves (4Å) were added and the solution was cooled to -60°C and stirred at that temperature for 1h. BF₃·OEt₂ (0.2 mL, 1.0M in toluene, 0.1 eq.) was added and the reaction was allowed to warm to -40°C. After 90 min TLC analysis showed full consumption of the donor and the reaction was quenched by addition of Et₃N (0.1 mL). The cloudy mixture was filtered and concentrated. The yellow oil was purified by silicagel chromatography (PE:EtOAc 3:2) giving target compound **8** in 60% yield (1.03 g, 1.18 mmol). ¹H NMR (CDCl₃, 400 MHz): δ = 7.86 - 7.74 (m, 4H, arom.), 7.56 (m, 2H, arom.), 7.50 - 7.42 (m, 3H, arom.), 7.41 - 7.30 (m, 3H, arom.), 7.07 (d, 1H, *J*=6.9 Hz, NH), 5.61 (s, 1H, PhCH), 5.26 - 5.00 (m, 5H, H-1, H-2', H-3', H-4', CH₂Nap), 4.89 (d, 1H, *J*=7.6 Hz, H-1'), 4.76 (d, 1H, *J*=11.7 Hz, CH₂Nap), 4.66 (dd, 1H, *J*=11.2, 3.5 Hz, H-3), 4.44 (d, 1H, *J*=3.4 Hz, H-4), 4.39 (dd, 1H, *J*=12.3, 1.2 Hz, H-6), 4.12 (dd, 1H, *J*=12.3, 1.5 Hz, H-6), 4.00 (d, 1H, *J*=9.8 Hz, H-5'), 3.90 (dd, 1H, *J*=7.1, 3.2 Hz, H-2), 3.70 (s, 3H, OMe), 3.52 (s, 1H, H-5), 2.00 (s, 3H, CH₃, Ac), 1.99 (s, 3H, CH₃, Ac), 1.98 (s, 3H, CH₃, Ac) ppm. ¹³C-APT NMR (CDCl₃, 101 MHz) δ 170.2, 169.5, 169.3 (C=O, Ac), 167.2 (C=O, CO₂Me), 162.3 (C=O, TCA), 137.8, 134.4, 133.3, 129.0, 128.4, 128.2, 128.0, 127.8, 127.2, 126.3, 126.3, 126.2 (arom.), 100.8 (PhCH), 100.2 (C-1'), 97.8 (C-1), 75.8 (C-4), 74.0 (C-3), 72.5 (C-3'), 72.1 (C-5'), 71.3 (C-2'), 71.3 (CH₂Nap), 69.2 (C-6), 69.1 (C-4'), 66.8 (C-5), 55.3 (C-2), 53.0 (OMe), 20.9, 20.7, 20.6 (CH₃, Ac) ppm.

### 2-napthylmethyl 4-O-benzyl-2-deoxy-3-O-(methyl 2,3,4-tri-O-acetyl-β-D-glucuronic acid)-2-(2,2,2-trichloroacetamido-β-D-galactopyranose (11)

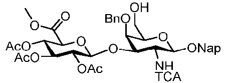

BH₃·THF (10 mL, 0.1M in THF, 3.0 eq.) was added to **10** (0.68 g, 0.78 mmol, 1.0 eq.) at 0°C, which was co-evaporated with dry toluene (2x) beforehand. CoCl₂ (0.30 g, 2.34 mmol, 3.0 eq.) was added and the reaction was left to stir for 2h. The reaction mixture was diluted in EtOAc (50 mL) and NaBH₄ (3 mL of a 1M in H₂O) was added. The black precipitate was filtered off and the layers were separated. The organic layer was washed with sat. aq. NaHCO₃ (1x) and brine (1x), dried over MgSO₄, filtered and concentrated *in vacuo.* The obtained crude was purified over silicagel chromatography (PE:EtOAc, 4:1→ 3:2) giving **11** (0.50 g, 0.57 mmol) in 74% yield. ¹H NMR (CDCl₃, 400 MHz): δ = 7.83 - 7.70 (m, 4H, arom.), 7.49 - 7.42 (m, 4H, arom), 7.42 - 7.30 (m, 4H, arom), 6.96 (d, 1H, *J*=7.0 Hz, NH), 5.27 - 5.15 (m, 2H, H-3', H-4' ), 5.12 - 5.05 (m, 1H, H-2'), 5.00 (d, 1H, *J*=11.8 Hz, CH₂arom.), 4.95 - 4.89 (m, 2H, H-1, CH₂arom.), 4.77 - 4.64 (m, 2H, H-1', CH₂arom.), 4.58 (dd, 1H, *J=11.0,* 2.9 Hz, H-3), 4.04 (d, 1H, *J*=9.6 Hz, H-5'), 4.00 (d, 1H, *J*=2.8 Hz, H-4), 3.85 (dt, 1H, *J*=11.0, 8.1 Hz, H-2), 3.75 (s, 3H, OMe), 3.70 (m, 1H, H-6), 3.52 - 3.39 (m, 2H, H-5, H-6), 2.05 (s, 3H, CH₃, Ac), 2.02 (s, 3H, CH₃, Ac), 2.00 (s, 3H, CH₃, Ac) ppm. ¹³C-APT NMR (CDCl₃, 101 MHz) δ 170.1, 169.6, 169.4 (C=O, Ac), 167.0 (C=O, CO₂Me), 162.3 (C=O, TCA), 138.0, 134.3, 133.3, 129.5, 128.6, 128.5, 128.3, 128.0, 127.8, 127.3, 126.4, 126.3, 126.1 (arom.), 101.2 (C-1'), 97.9 (C-1), 77.4 (C-3), 74.8 (C-5), 74.6 (CH₂Bn), 74.3 (C-4), 72.3 (C-5'), 72.1 (C-3'), 71.4 (CH₂Nap), 71.2 (C-2'), 69.5 (C-4'), 61.8 (C-6), 56.2 (C-2), 53.1 (OMe), 20.9, 20.7, 20.6 (CH₃, Ac) ppm. [M+NH₄]⁺ for C₃₉H₄₂Cl₃NO₁₅ calculated: 887.19554, found 887.19583.

### 2-napthylmethyl 4-O-benzyl-2-deoxy-6-O-levulinoyl-3-O-(methyl 2,3,4-tri-O-acetyl-β-D-glucuronic acid)-2-(2,2,2-trichloroacetamido-β-D-galactopyranose (12)

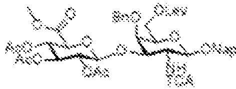

Compound **11** (0.50 g, 0.57 mmol, 1.0 eq.) was dissolved in dry DCM (3.0 mL, 0.2M) and cooled to 0°C. Levulinic acid (175 µL, 1.71 mmol, 3.0 eq), EDC·HCl (0.17 g, 0.86 mmol, 1.5 eq.) and DMAP (0.014 g, 0.11 mmol, 0.2 eq.) were added subsequently and the reaction was slowly warmed up to RT. After 1h TLC showed the formation of a new compound. The reaction mixture was diluted in EtOAc (50 mL) and washed with aq. HCl (1M, 2x), sat. aq. NaHCO₃ (2x) and brine (1x). The organic layer was then dried over MgSO₄, filtered and concentrated. Further purification was not required. Compound **18** was obtained as a white powder in a yield of 99% ( 0.55 g, 0.56 mmol). ¹H NMR (CDCl₃, 400 MHz): δ = 7.85 - 7.72 (m, 4H, arom.), 7.51 - 7.39 (m, 6H, arom.), 7.38 - 7.24 (m, 2H, arom.), 6.95 (d, 1H, *J*=7.0 Hz, NH), 5.24 - 5.14 (m, 2H, H-3', H-4'), 5.10 - 5.04 (m, 1H, H-2'), 5.01 (d, 1H, *J*=11.8 Hz, CH₂arom.), 4.95 (d, 1H, *J*=11.5 Hz, CH₂arom.), 4.92 (d, 1H, *J*=8.3 Hz, H-1), 4.73 (d, 1H, *J*=11.9 Hz, CH₂arom.), 4.70 (d, 1H, *J*=8.8 Hz, H-1'), 4.66 (d, 1H, *J*=11.5 Hz, CH₂arom.), 4.58 (dd, 1H, *J*=11.0, 3.0 Hz, H-3), 4.28 (dd, 1H, *J*=11.2, 6.8 Hz, H-6), 4.09 - 3.98 (m, 3H, H-4, H-6, H-5'), 3.85 (ddd, 1H, *J*=11.1, 8.3, 7.0 Hz, H-2), 3.74 (s, 3H, OMe), 3.72 - 3.64 (m, 1H, H-5), 2.73 (td, 2H, *J*=6.5, 1.6 Hz, CH₂Lev), 2.55 - 2.48 (m, 2H, CH₂Lev), 2.18 (s, 3H, CH₃lev), 2.04 (s, 3H, CH₃, Ac), 2.02 (s, 3H, CH₃, Ac), 1.99 (s, 3H, CH₃, Ac) ppm. ¹³C-APT NMR (CDCl₃, 101 MHz) δ 206.6 (C=O, MeC=O, Lev), 172.4 (C=O, CO₂, Lev), 170.1, 169.6, 169.4 (C=O, Ac), 167.0 (C=O, CO₂Me), 162.2 (C=O, TCA), 138.1, 134.3, 133.3, 133.2, 129.1, 128.4, 128.0, 127.9, 127.8, 127.4, 126.3, 126.2 (arom.), 101.1 (C-1'), 97.7 (C-1), 92.4 (C_{q}, TCA), 77.2(C-3), 75.0 (CH₂Bn), 74.9 (C-4), 72.3 (C-5'), 72.2 (C-5), 72.1 (C-3'), 71.2 (CH₂Nap), 71.2 (C-2'), 69.5 (C-4'), 63.0 (C-6), 56.0 (C-2), 53.0 (OMe), 38.0 (CH₂Lev), 30.0 (CH₃Lev), 27.9 (CH₂Lev), 20.9, 20.7, 20.6 (CH₃, Ac) ppm. [M+NH₄]⁺ for C₄₄H₄₈Cl₃NO₁₇ calculated: 985.23196, found 985.23261.

### 4-O-benzyl-2-deoxy-6-O-levulinoyl-3-O-(methyl 2,3,4-tri-O-acetyl-β-D-glucuronic acid)-2-(2,2,2-trichloroacetamido-α/β-D-galactopyranose (13)

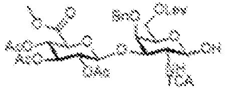

Silyl ether **18** (0.75 g, 0.70 mmol, 1.0 eq.) was dissolved in dry THF (7.0 mL, 0.1M) and cooled to 0°C. TBAF (1.1 mL, 1.0M in THF, 1.5 eq.) and acetic acid (60 µL, 1.1 mmol, 1.5 eq.) were added and the reaction was left overnight at RT. It was heated to 50°C for 3 hours after which TLC analysis showed full conversion to two lower running spots. The reaction mixture was diluted with EtOAc (100 mL) and washed with sat. aq. Ca₂CO₃ (1x), sat. aq. NaHCO₃ (1x) and brine (1x). the organic layer was dried over MgSO₄, filtered and concentrated. The residue was purified by silicagel chromatography (PE:EtOAc 3:3 → 2:3) giving **19** as a pale yellow foam (0.43 g, 0.52 mmol, 75%). ¹H NMR (CDCl₃, 400 MHz): δ = 7.45 - 7.25 (m, 6H), 6.84 (d, 1H, *J*=9.6 Hz), 5.29 - 5.15 (m, 3H), 5.16 - 5.06 (m, 1H), 4.99 (d, 1H, *J*=11.5 Hz), 4.72 (d, 1H, *J*=7.7 Hz), 4.64 - 4.49 (m, 2H), 4.32 (t, 1H, *J*=1.9 Hz), 4.16 (dd, 1H, *J*=7.9, 4.7 Hz), 4.12 - 3.98 (m, 4H), 3.94 (dd, 1H, *J*=2.9, 1.2 Hz), 3.78 (s, 3H), 2.86 - 2.65 (m, 2H), 2.49 (s, 0H), 2.18 (s, 3H), 2.11 (s, 3H), 2.03 (s, 3H), 2.00 (s, 3H) ppm. ¹³C-APT NMR (CDCl₃, 101 MHz) δ 208.6, 172.3, 170.0, 169.8, 169.5, 166.9, 161.4, 138.0, 129.0, 128.9, 128.4, 127.9, 101.3, 92.9, 91.7, 77.1, 75.1, 74.7, 72.5, 71.8, 70.5, 69.7, 67.9, 63.7, 53.0, 51.1, 38.3, 29.9, 28.2, 20.7, 20.6, 20.5 ppm. [M+NH₄]⁺ for C₃₃H₄₀Cl₃NO₁₇ calculated: 845.16978, found 845.17001.

### Trichloroacetamido 4-O-benzyl-2-deoxy-6-O-levulinoyl-3-O-(methyl 2,3,4-tri-O-acetyl-β-D-glucuronic acid)-2-(2,2,2-trichloroacetamido-α/β-D-galactopyranose (15)

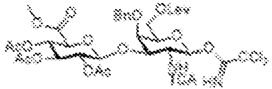

Hemiacetal **13** (0.33 g, 0.40 mmol, 1.0 eq.) was dissolved in dry DCM (4 mL, 0.1M). Trichloroacetonitrile (0.40 mL, 3.3 mmol, 10 eq.) was added followed by DBU (6 µL, 0.04 mmol, 0.1 eq.). The reaction was left to stir under nitrogen for 1h, at which point TLC analysis (PE:EtOAc 1:1) showed complete consumption of the starting material. The reaction mixture was concentrated and the resulting brown slurry was purified by silicagel chromatography (PE:EtOAc, 1:1). This gave the title compound as a yellow oil in 89% yield (0.35 g, 0.36 mmol). ¹H NMR (CDCl₃, 300 MHz): δ = 8.81 (s, 1H, NH, imidoyl), 7.54 - 7.20 (m, 5H, arom.), 6.65 (d, 1H, *J*=9.3 Hz, NH), 6.41 (d, 1H, *J*=3.9 Hz, H-1), 5.30 - 5.17 (m, 2H, H-3', H-4'), 5.16 - 5.07 (m, 1 H, H-2'), 5.02 (d, 1H, *J*=11.3 Hz, CH₂Bn), 4.88 - 4.77 (m, 2H, H-2, H-1'), 4.64 (d, 1H, *J*=11.3 Hz, CH₂Bn), 4.24 - 3.98 (m, 6H, H-3, H-5, H-6, H-4', H-5'), 3.77 (s, 3H, OMe), 2.79 - 2.64 (m, 2H, CH₂, Lev), 2.55 - 2.43 (m, 2H, CH₂, Lev), 2.16 (s, 3H, CH₃, Lev), 2.08 (s, 3H, CH₃, Ac), 2.04 (s, 3H, CH₃, Ac), 2.01 (s, 3H, CH₃, Ac) ppm. ¹³C-APT NMR (CDCl₃, 75 MHz) δ 206.6 (C=O, CH₃C=O), 172.3 (C=O, CO₂, Lev), 170.0 (C=O, CO₂Me), 169.6, 169.5, 166.9 (C=O, Ac), 161.6 (C=O, TCA), 160.1 (C=NH), 137.8, 128.8, 128.6, 128.5, 128.0 (arom.), 101.0 (C-1'), 95.2 (C-1), 90.8 (C_{q}, TCA), 76.9 (C-3), 75.2 (CH₂Bn), 74.8 (C-4), 72.6 (C-5), 71.8 (C-3'), 71.1 (C-5'), 70.8 (C-2'), 69.5 (C-4'), 62.9 (C-6), 53.1 (OMe), 50.4 (C-2), 37.9 (CH₂, Lev), 29.9 (CH₃, Lev), 27.8 (CH₂, Lev), 20.7, 20.6, 20.5 (CH₃, Ac) ppm.

### Tert-butyldimethylsilyl 4,6-O-benzylidene-2-deoxy-3-O-(methyl 2,3,4-tri-O-acetyl-β-D-glucuronic acid)-2-(2,2,2-trichloroacetamido-β-D-galactopyranose (16)

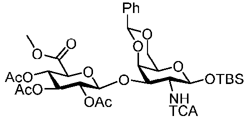

Compound **6** (4.27 g, 5.86 mmol, 1.0 eq.) was dissolved in dry DCM (12 mL, 0.5M) and cooled to 0°C. Imidazole (0.801 g, 11.72 mmol, 2.0 eq.) and TBS-Cl (0.97 g, 6.45 mmol, 1.1 eq.) were added subsequently and the reaction was allowed to cool to RT and stirred for 18h, after which it was diluted in EtOAc (150 mL) and washed with aq. HCl (1M) (2x), sat. aq. NaHCO₃ (2x) and brine (1x). The organic layer was dried over MgSO₄, filtered and all volatiles were removed. Disaccharide **16** was obtained after purification by silicagel chromatography (PE: EtOAc 4:1 → 3:2 as a white powder (2.801 g, 3.33 mmol) in a yield of 57%. ¹H NMR (CDCl₃, 400 MHz): δ = 7.58 - 7.51 (m, 2H, arom.), 7.42 - 7.32 (m, 3H, arom.), 7.07 (d, 1H, *J*=7.0 Hz, NH), 5.58 (s, 1 H, CHPh), 5.26 (d, 1H, *J*=7.8 Hz, H-1), 5.22 (t, 1H, *J*=9.5 Hz, H-4'), 5.15 (t, 1H, *J*=9.3, 8.8 Hz, H-3'), 5.04 (t, 1H, *J*=7.6 Hz, H-2'), 4.91 (d, 1H, *J*=7.6 Hz, H-1'), 4.66 (dd, 1H, *J*=11.2, 3.5 Hz, H-3), 4.41 (dd, 1H, *J*=3.5, 1.0 Hz, H-4), 4.26 (dd, 1H, *J*=12.2*,* 1.6 Hz, H-6), 4.08 (dd, 1H, *J*=12.3, 1.8 Hz, H-6), 4.02 (d, 1H, *J*=9.8 Hz, H-5'), 3.81 - 3.66 (m, 4H, H-2, OMe), 3.49 (q, 1H, *J*=1.5 Hz, H-5), 2.01 (s, 6H, CH₃, Ac), 1.99 (s, 3H, CH₃, Ac), 0.89 (s, 9H, t-Bu, TBS), 0.15 (s, 3H, CH₃, TBS), 0.11 (s, 3H, CH₃, TBS) ppm. ¹³C NMR (CDCl₃, 101 MHz) δ 170.2, 169.6, 169.3 (C=O, Ac), 167.3 (C=O, CO₂Me), 162.1 (C=O, TCA), 138.0, 129.0, 128.3, 126.3 (arom.), 100.8 (CHPh), 100.2 (C-1'), 94.0 (C-1), 75.9 (C-4), 73.7 (C-3), 72.5 (C-3'), 72.1 (C-5'), 71.3 (C-2'), 69.3 (C-6), 69.2 (C-4'), 66.7 (C-5), 57.4 (C-2), 53.0 (OMe), 25.9 (*t*-Bu, TBS), 21.0, 20.7, 20.6 (CH₃, Ac), 18.0 (C_{q}, TBS), -3.9, -4.7 (CH₃, TBS) ppm. [M+NH₄]⁺ for C₃₄H₄₆C_{I3}NO₁₅Si calculated: 859.20339, found 859.20405.

### Tert-butyldimethylsilyl 4-O-benzyl-2-deoxy-3-O-(methyl 2,3,4-tri-O-acetyl-β-D-glucuronic acid)-2-(2,2,2-trichloroacetamido-β-D-galactopyranose (17)

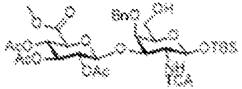

BH₃·THF (10 mL, 0.1M in THF, 3.0 eq.) was added to disaccharide **16** (2.80 g, 3.33 mmol, 1.0 eq.) at 0°C, which was co-evaporated with dry toluene (2x) beforehand. CoCl₂ (1.30 g, 10.0 mmol, 3.0 eq.) was added and the reaction was left to stir for 45 min. The reaction mixture was diluted in EtOAc (100 mL) and NaBH₄ (12 mL of a 1M in H₂O) was added. The black precipitate was filtered off and the layers were separated. The organic layer was washed with sat. aq. NaHCO₃ (1x) and brine (1x), dried over MgSO₄, filtered and concentrated *in vacuo.* The obtained crude was purified over silicagel chromatography (PE:EtOAc, 4:1→ 3:2) giving **17** (1.75 g, 2.1 mmol) in 63% yield. ¹H NMR (CDCl₃, 400 MHz): δ = 7.48 - 7.42 (m, 2H, arom.), 7.41 - 7.30 (m, 3H, arom.), 7.04 (d, 1H, *J*=7.0 Hz, NH), 5.27 - 5.16 (m, 2H, H-3', H-4'), 5.12 - 5.05 (m, 2H, H-1, H-2'), 4.91 (d, 1H, *J*=11.6 Hz, CH₂Bn), 4.73 (d, 1H, J=7.9 Hz, H-1'), 4.66 (d, 1H, *J*=11.7 Hz, CH₂Bn), 4.62 (dd, 1H, *J*=11.1*,* 3.1 Hz, H-3), 4.06 (d, 1H, J=9.6 Hz, H-5'), 3.98 (dd, 1H, *J*=3.1, 1.0 Hz, H-4), 3.76 (s, 3H, OMe), 3.73 - 3.61 (m, 2H, H-2, H-6), 3.53 - 3.45 (m, 1 H, H-5), 3.45 - 3.35 (m, 1 H, H-6), 2.08 (s, 3H, CH₃, Ac), 2.03 (s, 3H, CH₃, Ac), 2.00 (s, 3H, CH₃, Ac), 0.87 (s, 9H, t-Bu, TBS), 0.09 (s, 3H, CH₃, TBS), 0.07 (s, 3H, CH₃, TBS) ppm. ¹³C-APT NMR (CDCl₃, 101 MHz) δ 170.0, 169.5, 169.3 (C=O, Ac), 166.9 (C=O, CO₂Me), 162.0 (C=O, TCA), 138.0, 129.3, 128.5, 128.1 (arom.), 101.0 (C-1'), 93.9 (C-1), 92.3 (C_{q}, TCA), 76.8 (C-3), 74.7 (C-5), 74.5 (CH₂Bn), 74.3 (C-4), 72.2 (C-5'), 72.1 (C-3'), 71.1 (C-2'), 69.4 (C-4'), 61.7 (C-6), 58.3 (C-2), 52.9 (OMe), 25.7 (*t*-Bu, TBS), 20.9, 20.6, 20.5 (CH₃, Ac), 17.8 (C_{q}, t-Bu, TBS), -4.0, -5.1 (CH₃, TBS) ppm. [M+NH₄]⁺ for C₃₄H₄₈Cl₃NO₁₅Si calculated: 861.21954, found 861.21970.

### Tert-butyldimethylsilyl 4-O-benzyl-2-deoxy-6-O-levulinoyl-3-O-(methyl 2,3,4-tri-O-acetyl-β-D-glucuronic acid)-2-(2,2,2-trichloroacetamido-β-D-galactopyranose (18)

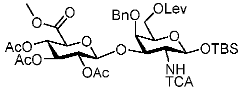

Disaccharide **17** (1.75 g, 2.0 mmol, 1.0 eq.) was dissolved in dry DCM (10 mL, 0.2M) and cooled to 0°C. Levulinic acid ( 0.62 mL, 6.0 mmol, 3.0 eq), EDC·HCl (0.58 g, 1.5 eq.) and DMAP (0.048 g, 0.40 mmol, 0.2 eq.) were added subsequently and the reaction was slowly warmed up to RT. TLC analysis using 2,4-Dinitrophenylhydrazine spray showed the formation of compound **18.** The reaction mixture was diluted in EtOAc (100 mL) and washed with aq. HCl (1M, 2x), sat. aq. NaHCO₃ (2x) and brine (1x). The organic layer was then dried over MgSO₄, filtered and concentrated. Further purification was not required. Compound **18** was obtained as a white powder in a yield of 99% ( 1.86 g, 1.97 mmol). ¹H NMR (CDCl₃, 400 MHz): δ = 7.45 - 7.25 (m, 5H, arom.), 7.09 (d, 1H, *J*=7.3 Hz, NH), 5.25 - 5.16 (m, 2H, H-3', H-4'), 5.11 - 5.02 (m, 2H, H-1, H-2'), 4.93 (d, 1H, *J*=11.4 Hz, CH₂Bn), 4.74 (d, 1H, *J*=7.9 Hz, H-1'), 4.64 (d, 1H, *J*=11.5 Hz, CH₂Bn), 4.60 (dd, 1H, *J*=11.1, 3.0 Hz, H-3), 4.17 (dd, 1H, *J*=11.2, 7.3 Hz, H-6), 4.09 - 4.03 (m, 1 H, H-5'), 4.01 - 3.92 (m, 2H, H-4, H-6), 3.75 (s, 3H, OMe), 3.74 - 3.61 (m, 2H, H-2, H-5), 2.76 - 2.65 (m, 2H, CH₂Lev), 2.48 (t, 2H, *J*=6.9 Hz, CH₂Lev), 2.19 (3, 3H, *J*=11.6 Hz, CH₃Lev), 2.07 (s, 3H, CH₃, Ac), 2.03 (s, 3H, CH₃, Ac), 2.00 (s, 3H, CH₃, Ac), 0.86 (s, 9H, t-Bu, TBS), 0.08 (s, 3H, CH₃, TBS), 0.07 (s, 3H, CH₃, TBS) ppm. ¹³C-APT NMR (CDCl₃, 101 MHz) δ 206.6 (CH₃C=O, Lev), 172.4 (CO₂, Lev), 170.0, 169.5, 169.3 (C=O, Ac), 167.0 (CO₂Me), 162.0 (C=O, TCA), 138.1, 129.1, 128.4, 127.8 (arom.), 101.0 (C-1'), 93.9 (C-1), 92.4 (C_{q}, TCA), 76.7 (C-3), 75.0 (C-4), 74.9 (CH₂Bn), 72.3 (C-5), 72.2 (C-5'), 72.0 (C-3'), 71.1 (C-2'), 69.5 (C-4'), 63.3 (C-6), 58.0 (C-2), 52.9 (OMe), 37.9 (CH₂Lev), 29.9 (CH₃Lev), 27.8 (CH₂Lev), 25.7 (t-Bu, TBS), 20.9, 20.6, 20.5 (CH₃, Ac), 17.9 (C_{q}, TBS), -4.1, -5.2 (CH₃, TBS) ppm. [M+NH₄]⁺ for C₃₉H₅₄Cl₃NO₁₇Si calculated: 959.25653 found 959.25648.

### Trifluoro (N-Phenyl)-acetamido 4-O-benzyl-2-deoxy-6-O-levulinoyl-3-O-(methyl 2,3,4-tri-O-acetyl-β-D-glucuronic acid)-2-(2,2,2-trichloroacetamido-α/β-D-galactopyranose (19)

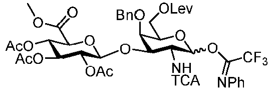

Hemiacetal **13** (0.52 g, 0.62 mmol, 1.0 eq.) was dissolved in acetone (3.1 mL, 0.2M). Cs₂CO₃ (0.40 g, 1.24 mmol, 2.0 eq.) and CF₃(C=NPh)-Cl (0.19 mL, 1.24 mmol, 2.0 eq.) were added subsequently and the reaction was left to stir overnight at RT. The suspension was filtered over celite and concentrated *in vacuo.* The brown foam was purified by silicagel chromatography (PE:EtOAc 4:1 → 1:1) giving donor **19** as a yellow foam (0.47 g, 0.47 mmol, 76%). ¹H NMR (CD₃CN, 400 MHz): δ = 7.66 (d, 1H, *J*=7.9 Hz), 7.49 - 7.27 (m, 10H), 7.20 - 7.08 (m, 1H), 6.82 (d, 2H, *J*=7.9 Hz), 6.26 (d, 0H, *J*=6.6 Hz), 5.45 - 5.37 (m, 0H), 5.34 (t, 1H, *J*=9.5 Hz), 5.20 - 4.98 (m, 5H), 4.92 (d, 0H, *J*=11.4 Hz), 4.61 (t, 1H, *J*=11.4 Hz), 4.31 - 4.02 (m, 7H), 3.90 (dd, 0H, *J*=8.0, 2.5 Hz), 3.77 - 3.61 (m, 4H), 2.85 - 2.60 (m, 3H), 2.48 (dt, 3H, *J*=12.8, 6.3 Hz), 2.29 (s, 3H), 2.10 (s, 4H), 2.06 - 1.93 (m, 12H) ppm. ¹³C-APT NMR (CD₃CN, 101 MHz) δ 207.7, 173.3, 173.2, 170.7, 170.7, 170.6, 170.6, 170.4, 170.3, 168.4, 163.2, 163.0, 144.4, 139.5, 139.4, 129.9, 129.7, 129.7, 129.3, 129.1, 128.8, 128.7, 125.4, 120.1, 107.1, 101.3, 101.1, 93.2, 81.0, 76.3, 75.9, 75.5, 75.0, 73.6, 73.5, 72.8, 72.8, 72.4, 72.3, 72.0, 71.9, 70.4, 70.4, 67.4, 63.4, 63.4, 53.4, 53.3, 51.6, 38.4, 38.4, 29.9, 28.6, 28.5, 21.1, 21.0, 20.9, 20.8, 20.8 ppm.

### 6-azidohexyl 4-O-benzyl-2-deoxy-6-O-levulinoyl-3-O-(methyl 2,3,4-tri-O-acetyl-β-D-glucoronic acid)-2-(2,2,2-trichloroacetamido-β-D-galactopyranose (20)

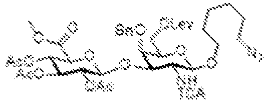

Disaccharide **19** (0.10 g, 0.10 mmol, 1.0 eq.) and 6-azidohexanol (0.028 g, 0.2 mmol, 2.0 eq.) were co-evaporated together with dry toluene (3x) and dissolved in a mixture of dry DCM/ACN (1 mL, 0.1M). MS (4Å) were added and the reaction was stirred for 30 min. at RT. TfOH (0.2 mL, 0.2 eq., of a 0.1M solution of TfOH in dry DCM) was added and the reaction was stirred at RT for 45 min. Et₃N (0.05 mL) was added and the reaction mixture was filtered over celite and diluted with EtOAc. The organic layer was washed with sat. aq. NaHCO₃ and brine, dried over MgSO₄, filtered and concentrated. The yellow crude was purified by silicagel chromatography (PE:EtOAc 3:2 → 2:3) giving the title compound in 93% yield (0.089 g, 0.093 mmol). ¹H NMR (CDCl₃, 400 MHz): δ = 7.43 - 7.39 (m, 2H), 7.34 (ddd, 2H, *J*=7.9, 6.9, 1.0 Hz), 7.27 (s, 3H), 7.01 (d, 1H, *J*=6.9 Hz), 5.26 - 5.16 (m, 2H), 5.13 - 5.02 (m, 1H), 4.94 (d, 1H, *J*=11.5 Hz), 4.83 (d, 1H, *J*=8.3 Hz), 4.74 (d, 1H, *J*=7.9 Hz), 4.68 - 4.59 (m, 2H), 4.21 (dd, 1H, *J*=11.1, 6.7 Hz), 4.10 - 3.97 (m, 3H), 3.86 (dt, 1H, *J*=9.8, 6.2 Hz), 3.75 (s, 3H), 3.75 - 3.61 (m, 2H), 3.44 (dt, 1H, *J*=9.6, 6.7 Hz), 3.24 (t, 2H, *J*=6.9 Hz), 2.72 (td, 2H, *J*=6.5, 1.9 Hz), 2.53 - 2.43 (m, 2H), 2.18 (s, 3H), 2.06 (s, 3H), 2.03 (s, 3H), 2.00 (s, 3H), 1.65 - 1.49 (m, 5H), 1.40 - 1.30 (m, 5H) ppm. ¹³C-APT NMR (CDCl₃, 101 MHz) δ 206.6, 172.4, 170.1, 169.6, 169.3, 167.0, 162.3, 138.2, 129.1, 128.4, 127.9, 101.1, 98.7, 92.4, 77.2, 75.0, 74.9, 72.2, 72.1, 72.0, 71.3, 70.0, 69.5, 62.9, 56.2, 53.0, 51.5, 38.0, 29.9, 29.5, 28.8, 27.8, 26.6, 25.7, 20.9, 20.7, 20.6 ppm.

### 6-azidohexyl 4-O-benzyl-2-deoxy-3-O-(methyl 2,3,4-tri-O-acetyl-β-D-glucoronic acid)-6-O-(4-O-benzyl-2-deoxy-6-O-levulinoyl-3-O-(methyl 2,3,4-tri-O-acetyl-β-D-glucuronic acid)-2-(2,2,2-trichloroacetamido)-β-D-galactopyranose)-2-(2,2,2-trichloroacetamido-β-D-galactopyranose (21)

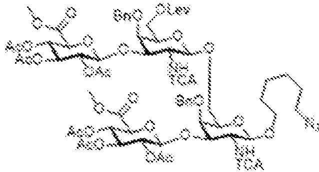

Disaccharide acceptor **9** (0.12 g, 0.14 mmol, 1.0 eq.) and disaccharide donor **19** (0.21 g, 0.21 mmol, 1.5 eq.) were co-evaporated together thrice with dry toluene and dissolved in a mixture of dry DCM (0.8 mL) and dry ACN (0.3 mL). MS (4Å) were added and the solution was stirred for 30 min. before addition of TfOH (0.28 mL, of a 0.1M solution, 0.2 eq.) The reaction was stirred for 3 hours after which Et₃N (0.05 mL) was added. The solution was diluted in EtOAc and filtered over celite and transferred to a seperatory funnel. The organic layer was washed with sat. aq. NaHCO₃ and brine, dried over MgSO₄, filtered and concentrated. The yellow oil was purified by SE (DCM/MeOH, 1/1, v/v) followed by silicagel chromatography (PE:EtOAc 2:3) giving tetrasaccharide **21** (0.134 g, 0.080 mmol, 63%). ¹H NMR (CDCl₃, 400 MHz): δ = 7.41 - 7.23 (m, 11H), 7.12 (d, 1H, *J*=7.0 Hz), 7.08 (d, 1H, *J*=7.3 Hz), 5.26 - 5.11 (m, 4H), 5.11 - 4.97 (m, 2H), 4.93 (d, 1H, *J*=11.3 Hz), 4.88 - 4.79 (m, 2H), 4.77 - 4.67 (m, 3H), 4.62 (dt, 3H, *J*=11.1, 4.1 Hz), 4.48 (dd, 1H, *J*=11.0, 3.0 Hz), 4.19 (dd, 1H, *J*=11.1, 6.7 Hz), 4.12 - 4.03 (m, 2H), 4.03 - 3.93 (m, 3H), 3.88 - 3.76 (m, 1H), 3.79 - 3.54 (m, 12H), 3.40 (dt, 1H, J=9.7, 6.6 Hz), 3.23 (t, 2H, J=6.9 Hz), 2.82 - 2.61 (m, 2H), 2.57 - 2.37 (m, 2H), 2.19 (s, 3H), 2.06 - 1.97 (m, 18H), 1.61 - 1.45 (m, 3H), 1.39 - 1.28 (m, 4H) ppm. ¹³C-APT NMR (CDCl₃, 101 MHz) δ 207.1, 172.4, 170.0, 170.0, 169.6, 169.4, 169.4, 169.3, 167.1, 167.0, 162.2, 162.1, 138.2, 138.1, 129.0, 128.9, 128.8, 128.4, 128.3, 128.2, 127.8, 127.8, 101.1, 101.0, 98.9, 98.6, 92.5, 92.4, 77.1, 77.0, 75.2, 75.0, 75.0, 74.7, 73.8, 72.3, 72.2, 72.1, 72.0, 71.9, 71.2, 71.0, 69.7, 69.4, 69.4, 68.5, 62.9, 56.0, 55.8, 53.1, 52.9, 51.4, 37.9, 30.0, 29.4, 28.8, 27.8, 26.5, 25.6, 20.9, 20.8, 20.6, 20.5 ppm. [M+NH₄]⁺ for C₆₇H₈₃Cl₆N₅O₃₁ calculated: 1683.35140, found 1683.35260.

### 6-azidohexyl 4-O-benzyl-2-deoxy-3-O-(methyl 2,3,4-tri-O-acetyl-β-D-glucoronic acid)-6-O-(4-O-benzyl-2-deoxy-3-O-(methyl 2,3,4-tri-O-acetyl-β-D-glucuronic acid)-2-(2,2,2-trichloroacetamido)-β-D-galactopyranose)-2-(2,2,2-trichloroacetamido-β-D-galactopyranose (22)

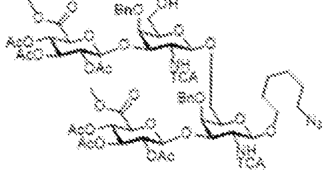

Fully protected tetrasaccharide **21** (0.12 g, 0.073 mmol, 1.0 eq.) was dissolved in a mixture of pyridine and acetic acid (1.0 mL, 4:1, v/v, 0.07M) and cooled to 0°C. After 30 min hydrazine acetate (0.020 g, 0.22 mmol, 3.0 eq.) was added and the ice bath was removed. TLC-MS showed full conversion of the starting material and acetone (0.1mL) was added to quench the remaining hydrazine. After 30 min. the solution was diluted in EtOAc and washed with NaHCO₃ and brine. The organic layer was dried over MgSO₄, filtered and concentrated. The title compound was obtained after purification by silicagel chromatography (PE:EtOAc, 2:3) as a white powder (0.10 g, 0.064 mmol, 88%). ¹H NMR (CDCl₃, 400 MHz): δ = 7.45 - 7.23 (m, 12H), 7.04 (d, 1H, J=7.0 Hz), 6.98 (d, 1H, J=6.9 Hz), 5.28 - 5.12 (m, 5H), 5.16 - 4.99 (m, 2H), 4.94 - 4.83 (m, 2H), 4.81 - 4.68 (m, 4H), 4.67 - 4.62 (m, 2H), 4.61 - 4.49 (m, 2H), 4.10 - 3.98 (m, 4H), 3.92 - 3.79 (m, 1H), 3.78 - 3.60 (m, 12H), 3.60 - 3.52 (m, 1H), 3.49 - 3.34 (m, 3H), 3.24 (t, 2H, J=6.9 Hz), 2.07 - 1.97 (m, 20H), 1.60 - 1.47 (m, 2H), 1.40 - 1.29 (m, 4H) ppm. ¹³C-APT NMR (CDCl₃, 101 MHz) δ 170.1, 169.6, 169.5, 169.4, 169.4, 167.2, 167.0, 162.3, 138.3, 138.1, 129.3, 129.1, 128.6, 128.3, 128.2, 127.8, 101.2, 101.1, 98.7, 98.6, 92.4, 77.3, 77.0, 75.1, 74.7, 74.7, 74.5, 74.1, 72.3, 72.2, 72.1, 71.3, 71.2, 69.8, 69.5, 69.4, 68.6, 61.8, 56.2, 56.2, 53.2, 53.1, 51.5, 29.5, 28.8, 26.6, 25.7, 20.9, 20.7, 20.6 ppm. [M+NH₄]⁺ for C₆₂H₇₇Cl₆N₅O₂₉ calculated: 1585.31432, found 1585.31566.

### 6-azidohexyl 4-O-benzyl-2-deoxy-3-O-(methyl 2,3,4-tri-O-acetyl-β-D-glucoronic acid)-6-O-(4-O-benzyl-2-deoxy-3-O-(methyl 2,3,4-tri-O-acetyl-β-D-glucuronic acid)-6-O-(4-O-benzyl-2-deoxy-6-O-levulinoyl-3-O-(methyl 2,3,4-tri-O-acetyl-β-D-glucuronic acid)-2-(2,2,2-trichloroacetamido-β-D-galactopyranose)-2-(2,2,2-trichloroacetamido)-β-D-galactopyranose)-2-(2,2,2-trichloroacetamido-β-D-galactopyranose (23)

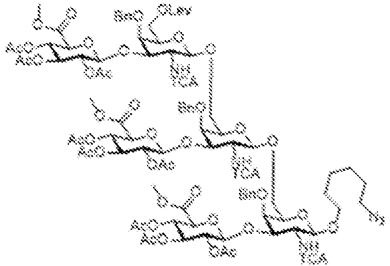

Tetrasaccharide acceptor **22** (0.094 g, 0.060 mmol, 1.0 eq.) and disaccharide donor (0.12 g, 0.12 mmol, 2.0 eq.) were co-evaporated together thrice with dry toluene, after which they were dissolved in amixture of dry DCM/ACN (0.6 ml, 0.1M, 4/1, v/v). MS (3Å) were added and the mixture was stirred 45 min. under nitrogen atmosphere. TfOH (120 µL, 0.2 eq. from a 0.1M solution in dry DCM) was added and the reaction was stirred at RT. After 45 min. TLC analysis showed full consumption of the acceptor and Et₃N (0.1 mL) was added. The solution was diluted in EtOAc and washed with sat. aq. NaHCO₃ and brine, followed by drying and concentrating *in vacuo.* Purification by silicagel (PE:EtOAc, 2:3) followed by SE (MeOH/DCM, 1/1) gave the title compound in 46% yield (0.066 g, 0.027 mmol). ¹H NMR (CDCl₃, 500 MHz): δ = 7.40 - 7.20 (m, 15H), 7.10 (d, 1H, *J*=7.2 Hz), 7.05 (2x d, 2H, *J*=6.7 Hz), 5.23 - 5.11 (m, 6H), 5.09 - 4.96 (m, 3H), 4.92 (d, 1H, *J*=11.4 Hz), 4.89 - 4.67 (m, 9H), 4.68 - 4.59 (m, 3H), 4.54 - 4.41 (m, 3H), 4.16 (dd, 1H, *J*=11.1*,* 6.7 Hz), 4.10 - 3.95 (m, 7H), 3.87 - 3.53 (m, 22H), 3.41 (d, 1H, *J*=9.8 Hz), 3.23 (t, 2H, *J*=6.9 Hz), 2.69 (ddd, 2H, *J*=12.4, 7.1, 5.9 Hz), 2.51 - 2.36 (m, 2H), 2.18 (s, 3H), 2.07 - 1.94 (m, 27H), 1.61 - 1.45 (m, 4H), 1.41 - 1.28 (m, 4H) ppm. ¹³C-APT NMR (CDCl₃, 126 MHz) δ 172.4, 170.1, 170.0, 169.6, 169.5, 169.5, 169.4, 169.3, 169.3, 167.1, 162.3, 162.2, 162.1, 138.5, 138.2, 129.5, 128.9, 128.8, 128.7, 128.4, 128.4, 128.3, 127.9, 127.8, 127.7, 120.6, 101.1, 101.0, 101.0, 99.1, 98.8, 98.6, 92.5, 77.5, 77.0, 75.3, 75.2, 75.0, 74.8, 73.7, 73.5, 72.3, 72.2, 72.2, 72.1, 72.1, 71.2, 71.2, 71.1, 69.8, 69.5, 69.3, 67.8, 67.7, 63.1, 56.1, 55.8, 55.6, 53.1, 53.1, 53.0, 51.5, 37.9, 30.0, 29.5, 28.8, 27.8, 26.6, 25.7, 20.9, 20.7, 20.6 ppm. [M+ 2 NH₄]²⁺ for C₉₅H₁₁₅Cl₉N₆O₄₅ calculated: 1207.23613, found 1207.23718.

### 6-azidohexyl 4-O-benzyl-2-deoxy-3-O-(methyl 2,3,4-tri-O-acetyl-β-D-glucoronic acid)-6-O-(4-O-benzyl-2-deoxy-3-O-(methyl 2,3,4-tri-O-acetyl-β-D-glucuronic acid)-6-O-(4-O-benzyl-2-deoxy-3-O-(methyl 2,3,4-tri-O-acetyl-β-D-glucuronic acid)-2-(2,2,2-trichloroacetamido-β-D-galactopyranose)-2-(2,2,2-trichloroacetamido)-β-D-galactopyranose)-2-(2,2,2-trichloroacetamido-β-D-galactopyranose (24)

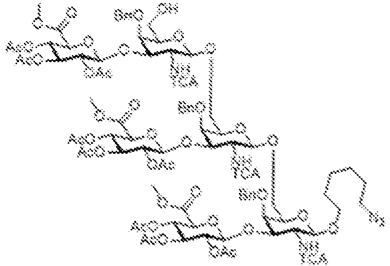

Hexasaccharide **23** (0.062 g, 0.026 mmol, 1.0 eq.) was dissolved in a mixture of pyridine/AcOH (0.50 mL, 0.05M, 4/1, v/v). Hydrazine acetate (7.0 mg, 0.078 mmol, 3.0 eq.) was added and the reaction was stirred for 2,5 hours. TLC staining with 2,4-dinitrophenylhydrazine in acidic methanol, showed full conversion into a new compound after 1.5h. The reaction mixture was diluted in EtOAc and washed with 1M HCl (4x) and sat. aq. NaHCO₃ (3x) followed by brine (1x). The organic layer was dried over MgSO₄, filtered and concentrated. No further purification was needed. The title compound was obtained as a white solid (0.056 g, 0.024 mmol, 91%). ¹H NMR (CDCl₃, 500 MHz): δ = 7.44 - 7.20 (m, 18H), 7.13 (d, 1H, *J*=7.0 Hz), 7.05 (d, 2H, *J*=8.5 Hz), 5.26 - 5.11 (m, 6H), 5.10 - 4.97 (m, 3H), 4.89 (dd, 2H, *J*=11.5, 2.5 Hz), 4.87 - 4.76 (m, 3H), 4.71 (m, 4H), 4.67 - 4.60 (m, 3H), 4.51 (ddd, 2H, *J*=20.3, 11.4, 2.7 Hz), 4.34 (d, 1H, *J*=11.1 Hz), 4.12 - 3.93 (m, 6H), 3.88 - 3.54 (m, 21H), 3.47 - 3.37 (m, 2H), 3.28 (m, 1H), 3.24 (t, 2H, *J*=6.9 Hz), 2.09 - 1.96 (m, 27H), 1.63 - 1.49 (m, 4H), 1.38 - 1.31 (m, 4H) ppm. ¹³C-APT NMR (CDCl₃, 126 MHz) δ 170.0, 169.5, 169.5, 169.4, 169.3, 169.3, 167.3, 167.3, 167.2, 162.2, 162.2, 138.5, 138.1, 129.5, 129.2, 128.8, 128.6, 128.6, 128.5, 128.3, 128.1, 127.8, 127.8, 101.2, 101.0, 101.0, 99.4, 98.6, 92.6, 92.5, 92.4, 77.8, 75.5, 75.4, 75.0, 74.9, 74.8, 74.7, 74.7, 74.1, 73.8, 72.2, 72.2, 72.1, 72.1, 71.2, 71.2, 71.1, 69.9, 69.5, 69.4, 69.3, 68.0, 61.7, 56.1, 55.6, 53.1, 53.1, 53.1, 53.1, 51.4, 29.8, 29.5, 28.8, 26.6, 25.6, 20.9, 20.6, 20.6, 20.6 ppm.

### 6-azidohexyl 4-O-benzyl-2-deoxy-3-O-(methyl 2,3,4-tri-O-acetyl-β-D-glucoronic acid)-6-O-(4-O-benzyl-2-deoxy-3-O-(methyl 2,3,4-tri-O-acetyl-β-D-glucuronic acid)-6-O-(4-O-benzyl-2-deoxy-3-O-(methyl 2,3,4-tri-O-acetyl-β-D-glucuronic acid)-6-O-(4-O-benzyl-2-deoxy-6-O-levulinoyl-3-O-(methyl 2,3,4-tri-O-acetyl-β-D-glucuronic acid)-2-(2,2,2-trichloroacetamido-β-D-galactopyranose)-2-(2,2,2-trichloroacetamido)-β-D-galactopyranose)-2-(2,2,2-trichloroacetamido-β-D-galactopyranose-2-(2,2,2-trichloroacetamido-β-D-galactopyranose. (25)

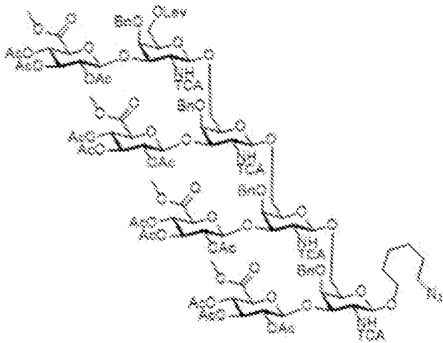

Acceptor **22** (0.112 g, 0.071 mmol, 1.0 eq.) and donor **29** (0.207 g, 0.12 mmol, 1.7 eq.) were co-evaporated together with dry toluene (3x). Molecular sieves (4Å) were added after which a solution of TfOH in DCM/ACN (0.7 mL, 0.014 mmol, 0.2 eq., 0.02M 4/1, v/v) was added. The reaction was left to stir for 24h at RT. The reaction was quenched by addition of Et₃N (0.1 ml), diluted in EtOAc and transferred to a seperatory funnel. The organic phase was washed with sat. aq. NaHCO₃, and brine, dried over MgSO₄, filtered and concentrated. The resulting yellow oil was purified using size exclusion chromatography (DCM/MeOH, 1/1, v/v), followed by silicagel chromatography (PE:EtOAc 1:1 → 3:7). This gave octasaccharide **25** as a white solid in 24% yield (0.055 g, 0.018 mmol.) ¹H NMR (CDCl₃, 500 MHz): δ = 7.49 - 7.18 (m, 20H), 7.17 - 7.10 (m, 1H), 7.09 - 6.98 (m, 3H), 5.24 - 4.90 (m, 15H), 4.88 - 4.58 (m, 16H), 4.49 (m, 3H), 4.41 (d, 1H, *J*=11.3 Hz), 4.17 (dt, 1H, *J*=12.6, 6.4 Hz), 4.10 - 3.95 (m, 11H), 3.89 - 3.49 (m, 30H), 3.41 (q, 1H, *J*=7.2 Hz), 3.23 (t, 2H, *J*=6.9 Hz), 2.72 - 2.61 (m, 2H), 2.48 - 2.41 (m, 2H), 2.17 (s, 3H), 2.06 - 1.96 (m, 36H), 1.62 - 1.49 (m, 4H), 1.41 - 1.29 (m, 4H) ppm. ¹³C-APT NMR (CDCl₃, 126 MHz) δ 207.0, 172.4, 170.0, 170.0, 169.6, 169.5, 169.5, 169.4, 169.4, 169.3, 169.3, 167.1, 167.1, 167.0, 162.3, 162.2, 162.1, 138.7, 138.5, 138.4, 138.2, 128.9, 128.9, 128.7, 128.5, 128.4, 128.3, 128.3, 128.2, 127.9, 127.7, 127.6, 101.1, 101.0, 101.0, 100.9, 99.3, 99.1, 98.6, 92.6, 92.5, 92.5, 77.2, 77.0, 75.2, 75.1, 75.0, 75.0, 74.9, 74.7, 73.9, 73.2, 72.2, 72.2, 72.1, 71.1, 71.0, 69.8, 69.5, 69.3, 69.2, 67.9, 67.7, 67.3, 63.0, 56.1, 56.0, 55.5, 53.1, 53.0, 51.4, 37.9, 30.3, 30.0, 29.8, 29.4, 28.8, 27.8, 26.6, 25.7, 20.9, 20.8, 20.6, 20.6 ppm.

### 6-azidohexyl 4-O-benzyl-2-deoxy-3-O-(methyl 2,3,4-tri-O-acetyl-β-D-glucoronic acid)-6-O-(4-O-benzyl-2-deoxy-3-O-(methyl 2,3,4-tri-O-acetyl-β-D-glucuronic acid)-6-O-(4-O-benzyl-2-deoxy-3-O-(methyl 2,3,4-tri-O-acetyl-β-D-glucuronic acid)-6-O-(4-O-benzyl-2-deoxy-3-O-(methyl 2,3,4-tri-O-acetyl-β-D-glucuronic acid)-2-(2,2,2-trichloroacetamido-β-D-galactopyranose)-2-(2,2,2-trichloroacetamido)-β-D-galactopyranose)-2-(2,2,2-trichloroacetamido-β-D-galactopyranose-2-(2,2,2-trichloroacetamido-β-D-galactopyranose. (26)

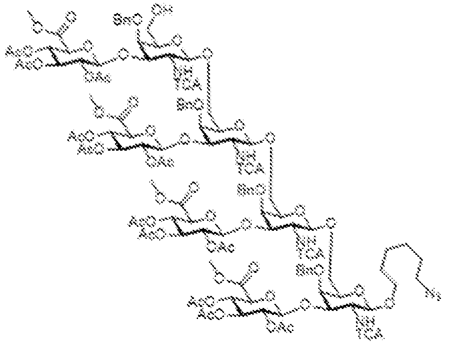

Octasaccharide **25** (0.055 g, 0.018 mmol, 1.0 eq.) was dissolved in a mixture of pyridine/AcOH (0.36 mL, 0.05M, 4/1, v/v). Hydrazine acetate (8.3 mg, 0.090 mmol, 5.0 eq.) was added and the reaction was stirred for 2,5 hours. TLC staining with 2,4-dinitrophenylhydrazine in acidic methanol, showed full conversion into a new compound. The reaction mixture was diluted in EtOAc and washed with 1M HCl (4x) and sat. aq. NaHCO₃ (3x) followed by brine (1x). The organic layer was dried over MgSO₄, filtered and concentrated. No further purification was needed. The title compound was obtained as a white solid (0.052 g, 0.017 mmol, 96%). ¹H NMR (CDCl₃, 400 MHz): δ = 7.44 - 7.20 (m, 20H), 7.17 - 7.03 (m, 4H), 5.26 - 5.10 (m, 9H), 5.10 - 4.96 (m, 4H), 4.93 - 4.77 (m, 5H), 4.80 - 4.57 (m, 9H), 4.55 - 4.36 (m, 4H), 4.10 - 3.94 (m, 8H), 3.86 - 3.60 (m, 25H), 3.55 (q, 2H, J=6.0 Hz), 3.47 - 3.36 (m, 3H), 3.23 (t, 2H, *J*=6.9 Hz), 2.08 - 1.95 (m, 36H), 1.61 - 1.49 (m, 4H), 1.39 - 1.30 (m, 4H) ppm. ¹³C-APT NMR (CDCl₃, 101 MHz) δ 170.0, 170.0, 169.5, 169.4, 169.4, 169.3, 169.3, 169.2, 167.2, 167.1, 167.0, 162.2, 162.2, 162.1, 138.5, 138.5, 138.2, 138.1, 129.1, 128.9, 128.6, 128.5, 128.5, 128.2, 128.0, 127.7, 101.1, 100.9, 99.2, 99.0, 98.5, 92.5, 92.4, 77.6, 76.8, 75.2, 75.1, 75.0, 74.8, 74.7, 73.8, 73.7, 73.2, 72.1, 72.1, 72.0, 71.1, 69.7, 69.4, 69.2, 67.7, 61.7, 56.0, 55.9, 55.4, 53.1, 53.0, 53.0, 51.4, 31.9, 31.4, 30.3, 30.2, 29.7, 29.4, 28.7, 26.5, 25.6, 20.8, 20.8, 20.6, 20.5 ppm.

### tert-butyldimethylsilyl 4-O-benzyl-2-deoxy-3-O-(methyl 2,3,4-tri-O-acetyl-β-D-glucoronic acid)-6-O-(4-O-benzyl-2-deoxy-6-O-levulinoyl-3-O-(methyl 2,3,4-tri-O-acetyl-β-D-glucuronic acid)-2-(2,2,2-trichloroacetamido)-β-D-galactopyranose)-2-(2,2,2-trichloroacetamido)-β-D-galactopyranose (27)

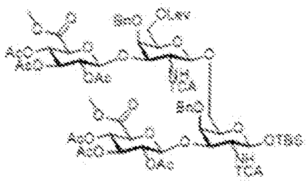

Acceptor (0.11 g, 0.13 mmol, 1.0 eq.) and donor (0.19 g, 0.19 mmol, 1.7 eq.) were co-evaporated with dry toluene (3x) and dissolved in a mixture of dry DCM/ACN (1.0 mL, 0.1M, 4/1, v/v). MS (4Å) were added and the mixture was stirred for 30 min. at RT. TfOH (0.2 mL, 0.2 eq. of a 0.1M solution in DCM) was added. Additional TfOH (0.2 mL, 0.2 eq. of a 0.1M solution in DCM) was added if only oxazoline product was observed. After 1h TLC showed full consumption of the donor. Et₃N (0.1 mL) was added and the solution was filtered over celite and diluted with EtOAc. The organic layer was washed with sat. aq. NaHCO₃ and brine, dried over MgSO₄, filtered and concentrated. The crude mixture was purified by size exclusion (DCM/MeOH, 1/1, v/v) giving the title compound in a 83% yield (0.18 g, 0.11 mmol). ¹H NMR (CDCl₃, 500 MHz): δ = 7.37 (dt, 4H, *J*=8.1, 1.4 Hz), 7.34 - 7.23 (m, 6H), 7.16 (d, 1H, *J*=7.8 Hz), 7.07 (d, 1H, *J*=6.8 Hz), 5.23 - 5.18 (m, 2H), 5.16 - 5.11 (m, 2H), 5.09 - 5.00 (m, 2H), 4.94 (d, 1H, *J*=11.3 Hz), 4.90 - 4.84 (m, 2H), 4.81 (d, 1H, *J*=8.2 Hz), 4.75 (d, 1H, *J*=7.8 Hz), 4.71 (d, 1H, J=7.9 Hz), 4.67 - 4.58 (m, 3H), 4.42 (dd, 1H, *J*=11.0, 3.1 Hz), 4.22 (dd, 1H, *J*=11.2, 7.3 Hz), 4.09 - 4.03 (m, 2H), 4.00 - 3.90 (m, 3H), 3.82 (dt, 1H, *J*=11.1, 7.8 Hz), 3.75 (s, 3H), 3.74 - 3.61 (m, 7H), 2.80 (ddd, 1H, *J*=18.2, 8.1, 5.5 Hz), 2.64 (ddd, 1H, *J*=18.2, 6.7, 5.4 Hz), 2.53 (ddd, 1H, *J*=17.3, 8.1, 5.3 Hz), 2.42 (ddd, 1H, *J*=17.3, 6.7, 5.6 Hz), 2.21 (s, 3H), 2.09 - 1.94 (m, 16H), 0.82 (s, 10H), 0.04 (s, 3H), 0.01 (s, 3H) ppm. ¹³C NMR (CDCl₃, 126 MHz) δ 207.6, 172.3, 170.0, 170.0, 169.6, 169.5, 169.4, 169.3, 167.2, 167.0, 162.4, 161.9, 138.5, 138.2, 129.0, 128.9, 128.8, 128.4, 128.2, 127.9, 127.8, 127.7, 101.1, 100.9, 99.0, 94.6, 92.7, 92.4, 77.1, 76.9, 76.9, 75.3, 75.1, 75.1, 74.8, 73.4, 72.4, 72.2, 72.1, 72.0, 71.3, 70.9, 69.5, 69.4, 68.8, 63.5, 57.7, 56.1, 53.0, 53.0, 37.9, 30.1, 27.8, 25.8, 25.8, 20.9, 20.8, 20.6, 20.6, 17.9, -3.9, -5.2 ppm. [M+Na]⁺ for C₆₇H₈₆Cl₆N₂O₃₁Si calculated: 1677.29752, found 1677.29921.

### 4-O-benzyl-2-deoxy-3-O-(methyl 2,3,4-tri-O-acetyl-β-D-glucoronic acid)-6-O-(4-O-benzyl-2-deoxy-6-O-levulinoyl-3-O-(methyl 2,3,4-tri-O-acetyl-β-D-glucuronic acid)-2-(2,2,2-trichloroacetamido)-β-D-galactopyranose)-2-(2,2,2-trichloroacetamido-α/β-D-galactopyranose (28)

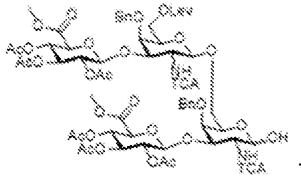

Tetrasaccahride 27 (0.19 g, 0.12 mmol, 1.0 eq.) was dissolved in THF (0.92 mL, 0.1M final volume) and cooled to 0°C. AcOH (10 µL, 0.18 mmol, 1.5 eq.) and TBAF (0.18 mL, 1.5 eq. from a 1M solution in THF) were added. The reaction was allowed to warm to RT overnight. TLC (PE:EtOAc 3:7) showed a more polar spot and the reaction mixture was diluted in EtOAc. The organic layer was washed with sat. aq. NaHCO₃ was added and brine. The organic layer was dried over MgSO₄, filtered and concentrated. The crude mixture was purified by silicagel chromatography (PE:EtOAc 3:2 → 3:7) giving 28 in 76% yield (0.14 g, 0.090 mmol). ¹H NMR (CDCl₃, 400 MHz): δ = 7.43 - 7.30 (m, 9H), 7.30 - 7.18 (m, 4H), 6.88 (d, 1H, J=9.6 Hz), 5.18 (ddt, 5H, J=9.3, 7.8, 3.5 Hz), 5.12 - 4.97 (m, 3H), 4.93 (d, 1H, J=5.0 Hz), 4.90 (d, 1H, J=4.8 Hz), 4.76 (d, 1H, *J*=7.8 Hz), 4.72 (d, 1H, *J*=7.8 Hz), 4.62 (d, 1H, *J*=11.3 Hz), 4.58 (d, 1H, *J*=11.4 Hz), 4.49 (tdd, 2H, *J*=11.0, 7.0, 3.2 Hz), 4.21 - 4.09 (m, 2H), 4.09 - 3.93 (m, 5H), 3.93 - 3.81 (m, 2H), 3.78 - 3.61 (m, 8H), 3.45 (dd, 1H, *J*=11.9, 3.2 Hz), 2.77 - 2.57 (m, 1H), 2.55 - 2.32 (m, 1H), 2.18 (d, 3H, *J*=2.0 Hz), 2.09 (s, 3H), 2.05 (s, 3H), 2.03 (d, 3H, *J*=1.3 Hz), 2.02 (d, 3H, *J*=1.4 Hz), 2.00 (d, 3H, *J*=1.2 Hz), 1.99 (s, 3H) ppm. ¹³C-APT NMR (CDCl₃, 101 MHz) δ 207.2, 172.4, 170.1, 170.0, 169.8, 169.6, 169.6, 169.4, 167.1, 167.0, 162.5, 161.5, 138.1, 137.9, 129.1, 129.1, 128.5, 128.4, 128.0, 127.9, 101.1, 99.1, 93.0, 92.4, 91.7, 77.2, 76.6, 75.7, 75.0, 75.0, 74.8, 72.5, 72.2, 72.0, 71.9, 71.2, 70.7, 70.6, 69.8, 69.5, 69.4, 62.8, 55.9, 53.1, 53.0, 51.2, 37.9, 30.0, 27.8, 20.9, 20.7, 20.6, 20.6 ppm. [M+NH₄]⁺ for C₆₁H₇₂Cl₆N₂O₃₁ calculated: 1558.25425, found 1558.25712.

### Trifluoro (N-Phenyl)-acetamido 4-O-benzyl-2-deoxy-3-O-(methyl 2,3,4-tri-O-acetyl-β-D-glucoronic acid)-6-O-(4-O-benzyl-2-deoxy-6-O-levulinoyl-3-O-(methyl 2,3,4-triO-acetyl-β-D-glucuronic acid)-2-(2,2,2-trichloroacetamido)-β-D-galactopyranose)-2-(2,2,2-trichloroacetamido-α/β-D-galactopyranose (29)

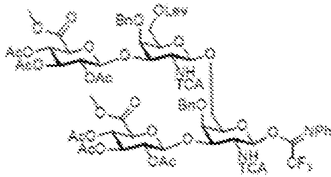

Hemi acetal **28** (0.20 g, 0.13 mmol, 1.0 eq.) was dissolved in dry acetone (1.3 mL, 0.1M). Cs₂CO₃ (0.084 g, 0.26 mmol, 2.0 eq.) and PTFAI-CI (42 µL, 0.26 mmol, 2.0 eq.) were added at RT. The reaction was stirred under inert atmosphere for 14h, after which it was filtered over celite and concentrated. The resulting yellow oil was purified over neutralized silicagel (PE:EtOAc 3:2 → 3:7). The title compound was obtained as a yellow foam (0.21 g, 0.12 mmol, 93%). ¹H NMR (CD₃CN, 400 MHz): δ = 7.63 (d, 1H, *J*=8.3 Hz), 7.53 (d, 1H, *J*=9.2 Hz), 7.47 - 7.23 (m, 10H), 7.15 - 7.06 (m, 1H), 6.83 - 6.74 (m, 1H), 6.20 (d, 0H, *J*=6.6 Hz), 5.39 - 5.24 (m, 2H), 5.17 - 5.01 (m, 4H), 4.96 (ddd, 2H, *J*=12.6, 7.8, 2.9 Hz), 4.88 - 4.79 (m, 1H), 4.64 - 4.49 (m, 3H), 4.42 (d, 1H, *J*=14.2 Hz), 4.29 - 3.86 (m, 8H), 3.82 - 3.74 (m, 0H), 3.73 - 3.55 (m, 7H), 2.70 (td, 2H, *J*=7.2, 6.8, 1.6 Hz), 2.46 - 2.40 (m, 2H), 2.21 (s, 5H), 2.10 (d, 3H, J=1.2 Hz), 2.04 - 1.90 (m, 20H) ppm. ¹³C-APT NMR (CD₃CN, 101 MHz) δ 207.9, 173.3, 170.8, 170.7, 170.6, 170.5, 170.4, 170.3, 168.4, 168.3, 163.2, 163.1, 163.1, 144.4, 139.7, 139.7, 139.5, 130.2, 129.9, 129.7, 129.6, 129.6, 129.5, 129.3, 129.3, 129.2, 129.2, 129.2, 129.1, 128.7, 128.6, 125.3, 120.1, 107.8, 102.0, 101.9, 101.9, 101.2, 100.9, 93.8, 93.1, 81.0, 79.1, 76.2, 76.2, 76.2, 76.1, 75.7, 75.6, 75.3, 75.1, 74.7, 73.7, 72.9, 72.9, 72.8, 72.8, 72.5, 72.4, 72.3, 72.0, 71.9, 71.8, 70.5, 70.4, 70.4, 69.3, 68.8, 66.8, 63.4, 55.0, 54.9, 53.5, 53.4, 53.3, 51.9, 50.6, 38.4, 29.9, 29.9, 28.6, 28.6, 21.1, 21.1, 21.0, 20.9, 20.8, 20.8, 20.8 ppm.

### 6-aminohexyl 2-acetamido-2-deoxy-3-O-(β-D-glucoronic acid)-β-D-galactopyranose (30)

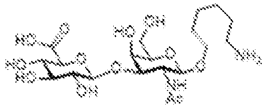

Disaccharide (0.048 g, 0.042 mmol, 1.0 eq.) was dissolved in THF (1.0 mL). Freshly prepared Li₂O₂ (0.30 mL, 1M in H₂O) was added and the mixture was stirred for 1h. The mixture was diluted with H₂O (5 mL) and the pH was adjusted to pH 3.5 by addition of amberlite H⁺ resin. The resin was filtered off and the filtrate was dried *in vacuo.* The resulting crude was dissolved in a mixture of degassed H₂O/*t*-BuOH (1 mL, 1/1, v/v). Pearlman's catalyst (30 mg) and NaOAc (0.016 g, 0.20 mmol, 9 eq.) were added and the suspension was purged with nitrogen for 5 min. before introduction of hydrogen atmosphere. The suspension was stirred under H₂ atmosphere for 4 days. It was then filtered over a whatmann filter covered by celite. The filtrate was concentrated *in vacuo* and the resulting crude disaccharide was purified by SE (aqueous 0.15M NH₄HCO₃ buffer) and subsequently lyophilized yielding the title compound as a white fluffy powder (0.011 g, 0.022 mmol, 53%). The spectral data was in accordance with those reported in literature.^{[8]}

### 6-aminohexyl 2-acetamido-2-deoxy-3-O-(β-D-glucoronic acid)-6-O-(2-acetamido-2-deoxy-3-O-(β-D-glucuronic acid)-β-D-galactopyranose)-β-D-galactopyranose (31)

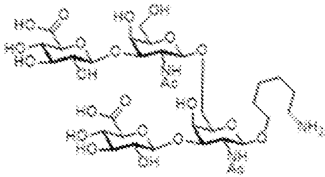

Tetrasaccahride **22** (0.059 g, 0.038 mmol, 1.0 eq.) was dissolved in THF (0.36 mL). Freshly prepared Li₂O₂ (0.45 mL, 1M in H₂O) was added and the mixture was stirred for 1h. The mixture was diluted with H₂O (5 mL) and the pH was adjusted to pH 3.5 by addition of amberlite H⁺ resin. The resin was filtered off and the filtrate was dried *in vacuo.* The resulting crude was dissolved in a mixture of degassed H₂O/*t*-BuOH (1 mL, 1/1, v/v). Pearlman's catalyst (30 mg) and NaOAc (0.016 g, 0.20 mmol, 9 eq.) were added and the suspension was purged with nitrogen for 5 min. before introduction of hydrogen atmosphere. The suspension was stirred under H₂ atmosphere for 4 days. It was then filtered over a whatmann filter covered by celite. The filtrate was concentrated *in vacuo* and the resulting crude tetrasaccharide was purified by SE (aqueous 0.15M NH₄HCO₃ buffer) and subsequently lyophilized and when NMR showed aromatic signals the crude was redissolved in degassed H₂O (1 mL). Palladium black (30 mg) was added, and after 5 min of purging with nitrogen, hydrogen was introduced via a balloon. The suspension was stirred for an additional 4 days, after which the palladium was filtered off as before. The resulting crude tetrasaccharide was purified by SE (aqueous 0.15M NH₄HCO₃ buffer) and subsequently lyophilized yielding the title compound as a white fluffy powder (0.009 g, 0.010 mmol, 27%). ¹H NMR (D₂O, 500 MHz): δ = 4.51 - 4.28 (m, 4H), 4.21 - 3.53 (m, 15H), 3.53 - 3.44 (m, 1H), 3.46 - 3.30 (m, 4H), 3.30 - 3.15 (m, 3H), 2.89 (t, 2H, *J*=7.6 Hz), 1.98 - 1.83 (m, 9H), 1.52 (dt, 5H, *J*=41.3, 6.8 Hz), 1.36 - 1.19 (m, 5H) ppm. ¹³C-APT NMR (D₂O, 126 MHz) δ 176.0, 175.9, 174.7, 104.1, 104.1, 101.7, 101.2, 80.1, 79.9, 76.2, 75.4, 74.9, 73.7, 72.8, 71.8, 68.1, 67.8, 61.1, 51.3, 51.2, 39.5, 28.3, 26.7, 25.3, 24.7, 22.4, 22.3 ppm. [M+H]⁺ For C₃₄H₅₇N₃O₂₃ calculated: 876.34993, found 876.34556.

### 6-aminohexyl 2-acetamido-2-deoxy-3-O-(β-D-glucoronic acid)-6-O-(2-acetamido-2-deoxy-3-O-(β-D-glucuronic acid)-6-O-(2-acetamido-2-deoxy-3-O-(β-D-glucuronic acid)-β-D-galactopyranose)-β-D-galactopyranose)-β-D-galactopyranose (32)

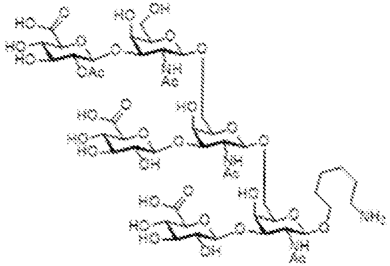

Hexasaccharide **24** (0.056 g, 0.024 mmol, 1.0 eq.) was dissolved in THF (0.36 mL). Freshly prepared Li₂O₂ (0.32 mL, 1M in H₂O) was added and the mixture was stirred for 4h. The mixture was diluted with H₂O (5 mL) and the pH was adjusted to pH 3.5 by addition of amberlite H⁺ resin. The resin was filtered off and the filtrate was dried *in vacuo.* The resulting crude was dissolved in a mixture of degassed H₂O/*t*-BuOH (1 mL, 1/1, v/v). Palladium black (30 mg) and NaOAc (0.016 g, 0.20 mmol, 9 eq.) were added and the suspension was purged with nitrogen for 5 min. before introduction of hydrogen atmosphere. The suspension was stirred under H₂ atmosphere for 4 days. It was then filtered over a whatmann filter covered by celite. The filtrate was concentrated *in vacuo.* The resulting crude hexasaccharide was purified by SE (aqueous 0.15M NH₄HCO₃ buffer) and subsequently lyophilized yielding the title compound as a white fluffy powder (0.016 g, 0.013 mmol, 53%). ¹H NMR (D₂O, 500 MHz): δ = 4.53 - 4.32 (m, 6H), 4.12 - 3.98 (m, 5H), 3.90 (m, 9H), 3.84 - 3.55 (m, 24H), 3.55 - 3.31 (m, 12H), 3.23 (m, 5H), 2.89 (t, 2H, *J*=7.6 Hz), 1.99 - 1.79 (m, 9H), 1.52 (dt, 4H), 1.26 (m, 4H) ppm. ¹³C-APT NMR (D₂₀, 126 MHz) δ 175.0, 174.7, 104.1, 101.9, 101.5, 101.2, 80.4, 80.2, 80.0, 75.8, 75.3, 75.2, 74.9, 73.5, 73.1, 72.8, 72.7, 71.7, 70.1, 70.0, 69.8, 68.1, 67.8, 61.8, 61.1, 51.3, 46.7, 42.4, 39.5, 28.3, 26.7, 25.3, 24.7, 22.5, 22.4, 22.3, 8.3 ppm. [M+H]⁺ for C₄₃H₇₃N₄O₃₄ calculated: 1255.45681, found 1255.45702.

### 6-aminohexyl 2-acetamido-2-deoxy-3-O-(β-D-glucoronic acid)-6-O-(2-acetamido-2-deoxy-3-O-(β-D-glucuronic acid)-6-O-(2-acetamido-2-deoxy-3-O-(β-D-glucuronic acid)-6-O-(2-acetamido-2-deoxy-3-O-(β-D-glucuronic acid)-β-D-galactopyranose)-β-D-galactopyranose)-β-D-galactopyranose-β-D-galactopyranose (33).

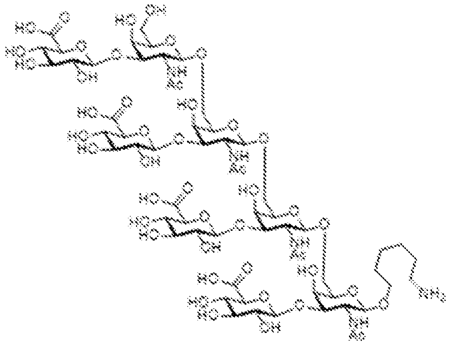

Octasaccharide **26** (0.052 g, 0.017 mmol, 1.0 eq.) was dissolved in THF (0.36 mL). Freshly prepared Li₂O₂ (0.288 mL, 1M in H₂O) was added and the mixture was stirred for 4h. The mixture was diluted with H₂O (5 mL) and the pH was adjusted to pH 3.5 by addition of amberlite H⁺ resin. The resin was filtered off and the filtrate was dried *in vacuo.* The resulting crude was dissolved in a mixture of degassed H₂O/*t*-BuOH (1 mL, 1/1, v/v). Palladium black (30 mg) and NaOAc (0.017, 0.216 mmol, 12 eq.) were added and the suspension was purged with nitrogen for 5 min. before introduction of hydrogen atmosphere. The suspension was stirred under H₂ atmosphere for 4 days. It was then filtered over a whatmann filter covered by celite. The filtrate was concentrated *in vacuo.* The resulting crude octasaccharide was purified by SE (aqueous 0.15M NH₄HCO₃ buffer) and subsequently lyophilized yielding the title compound as a white fluffy powder (0.016 g, 0.010 mmol, 58%). ¹H NMR (D₂O, 500 MHz): δ = 4.51 - 4.31 (m, 8H), 4.13 - 3.57 (m, 21H), 3.54 - 3.45 (m, 1H), 3.44 - 3.33 (m, 9H), 3.29 - 3.17 (m, 5H), 2.89 (t, 2H, *J*=7.6 Hz), 2.01 - 1.83 (m, 14H), 1.66 - 1.40 (m, 4H), 1.33 - 1.20 (m, 4H) ppm. ¹³C-APT NMR (D₂O, 126 MHz) δ 175.5, 175.1, 174.9, 174.8, 174.7, 174.7, 104.2, 104.1, 104.1, 103.9, 103.2, 101.9, 101.6, 101.2, 80.8, 80.4, 80.2, 79.9, 76.3, 76.1, 76.0, 75.9, 75.4, 75.3, 75.3, 75.0, 74.9, 73.6, 72.9, 72.8, 72.7, 72.7, 72.5, 72.4, 71.9, 71.9, 71.8, 71.2, 70.2, 68.2, 68.1, 67.9, 61.8, 61.3, 51.4, 51.3, 39.5, 31.4, 28.5, 28.3, 26.7, 25.3, 24.9, 24.8, 24.6, 22.5, 22.4, 22.3 ppm. [M+2H]²⁺ for C₆₂H₉₉N₅O₄₅ calculated: 817.78762, found 817.78788.

### 6-azidohexyl 4-O-benzyl-2-deoxy-3-O-(methyl 2,3,4-tri-O-acetyl-β-D-glucoronic acid)-6-O-(4-O-benzyl-2-deoxy-3-O-(methyl 2,3,4-tri-O-acetyl-β-D-glucuronic acid)-6-O-(4-O-benzyl-2-deoxy-3-O-(methyl 2,3,4-tri-O-acetyl-β-D-glucuronic acid)-6-O-(4-O-benzyl-2-deoxy-3-O-(methyl 2,3,4-tri-O-acetyl-β-D-glucuronic acid)-6-O-(4-O-benzyl-2-deoxy-6-O-levulinoyl-3-O-(methyl 2,3,4-tri-O-acetyl-β-D-glucuronic acid)-2-(2,2,2-trichloroacetamido-β-D-galactopyranose)-2-(2,2,2-trichloroacetamido)-β-D-galactopyranose)-2-(2,2,2-trichloroacetamido-β-D-galactopyranose-2-(2,2,2-trichloroacetamido-β-D-galactopyranose. (34)

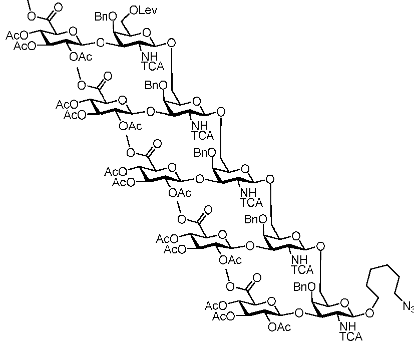

Acceptor **29** (0.078 g, 0.034 mmol, 1.0 eq.) and donor **24** (0.117 g, 0.068 mmol, 2.0 eq.) were co-evaporated together with dry toluene (3x) and dissolved in dry DCM (1.0 mL, 0.1M). MS (5Å) were added and the mixture was stirred for 30 min. at RT. Yb(OTf)₃ ( 4.2 mg, 0.2eq. of a 0.05M solution in acetonitrile) was added and the reaction was stirred for 3 hours at RT. The reaction was quenched by addition of Et₃N (0.1 ml), diluted in DCM and transferred to a seperatory funnel. The organic phase was washed with brine, dried over MgSO₄, filtered and concentrated. The resulting oil was purified using size exclusion chromatography (DCM/MeOH, 1/1, v/v). This gave decasaccharide **34** as a white solid in 61% yield (0.079 g, 0.020 mmol.) ¹H NMR (CDCl₃, 500 MHz): δ 7.40 - 7.18 (m, 26H), 7.11 - 6.99 (m, 5H), 5.27 - 4.96 (m, 15H), 4.93 (d, J = 11.3 Hz, 1H), 4.86 - 4.56 (m, 19H), 4.49 (m, 3H), 4.39 (m, 2H), 4.17 (dd, J = 11.2, 6.7 Hz, 1H), 4.09 - 3.95 (m, 11H), 3.89 - 3.47 (m, 34H), 3.41 (dt, J = 10.0, 6.6 Hz, 1H), 3.23 (t, J = 6.9 Hz, 2H), 2.76 - 2.59 (m, 2H), 2.48 - 2.36 (m, 2H), 2.16 (s, 3H), 2.07 - 1.94 (m, 46H), 1.60 - 1.47 (m, 4H), 1.38 - 1.29 (m, 4H), ppm. ¹³C-APT NMR (CDCl₃, 126 MHz) δ 207.01, 172.44, 170.07, 170.04, 169.58, 169.53, 169.50, 169.44, 169.41, 169.38, 169.30, 167.24, 167.18, 167.16, 167.03, 162.29, 162.20, 162.15, 138.75, 138.63, 138.58, 138.37, 138.20, 128.90, 128.71, 128.64, 128.59, 128.44, 128.33, 128.30, 128.28, 127.91, 127.79, 127.73, 101.16, 101.01, 100.97, 99.35, 99.18, 98.59, 92.62, 92.47, 77.41, 77.28, 77.16, 76.91, 75.19, 75.13, 75.10, 75.03, 74.94, 74.76, 73.88, 73.33, 73.24, 72.23, 71.15, 71.04, 69.80, 69.54, 69.34, 69.27, 69.23, 67.85, 67.42, 63.02, 56.15, 56.03, 55.62, 55.52, 55.43, 53.15, 53.09, 53.00, 51.46, 37.95, 29.98, 29.46, 28.82, 27.82, 26.58, 25.68, 20.91, 20.88, 20.67, 20.62.6 ppm.

### 6-aminohexyl 2-acetamido-2-deoxy-3-O-(β-D-glucoronic acid)-6-O-(2-acetamido-2-deoxy-3-O-(β-D-glucuronic acid)-6-O-(2-acetamido-2-deoxy-3-O-(β-D-glucuronic acid)-6-O-(2-acetamido-2-deoxy-3-O-(β-D-glucuronic acid)-6-O-(2-acetamido-2-deoxy-3-O-(β-D-glucuronic acid)-β-D-galactopyranose)-β-D-galactopyranose)-β-D-galactopyranose-β-D-galactopyranose (35).

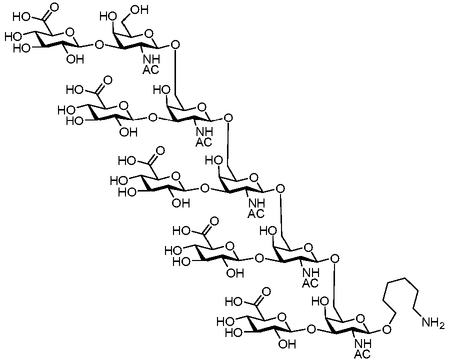

Decasaccharide **34** (0.042 g, 0.011 mmol, 1.0 eq.) was dissolved in THF (1 mL). Freshly prepared Li₂O₂ (0.221 mL, 1M in H₂O) was added and the mixture was stirred for 4h. The mixture was diluted with H₂O (5 mL) and the pH was adjusted to pH 3.5 by addition of amberlite H⁺ resin. The resin was filtered off and the filtrate was dried *in vacuo.* The resulting crude was dissolved in a mixture of degassed H₂O/*t*-BuOH (3 mL, 1/1, v/v). Palladium black (30 mg) and NaOAc (0.013g, 0.158 mmol, 15 eq.) were added and the suspension was purged with nitrogen for 5 min. before introduction of hydrogen atmosphere. The suspension was stirred under H₂ atmosphere for 4 days. It was then filtered over a whatmann filter covered by celite. The filtrate was concentrated *in vacuo.* The resulting crude octasaccharide was purified by SE (aqueous 0.15M NH₄HCO₃ buffer) and subsequently lyophilized yielding the title compound as a white fluffy powder (0.013 g, 0.006 mmol, 60%). ¹H NMR (D₂O, 850 MHz): δ 4.54 - 4.29 (m, 10H), 4.17 - 3.56 (m, 45H), 3.53 - 3.36 (m, 14H), 3.25 (m, 6H), 2.91 (t, J = 7.6 Hz, 2H), 2.02 - 1.90 (m, 16H), 1.58 (m, 2H), 1.53 - 1.45 (m, 3H), 1.33 - 1.20 (m, 5H). ppm. ¹³C-APT NMR (D₂O, 126 MHz) δ 174.87, 174.72, 174.65, 174.62, 104.05, 104.02, 103.94, 103.85, 103.80, 103.76, 102.75, 101.96, 101.38, 101.20, 81.09, 80.68, 80.36, 80.03, 79.94, 76.62, 76.27, 76.00, 75.35, 75.30, 75.23, 75.20, 74.97, 73.76, 72.88, 72.84, 72.82, 72.81, 72.73, 72.69, 72.67, 72.33, 72.07, 71.96, 71.94, 71.91, 71.80, 71.78, 71.61, 70.29, 68.28, 68.21, 68.03, 67.97, 67.88, 67.51, 61.26, 51.60, 51.51, 51.47, 51.37, 51.27, 39.41, 39.39, 28.27, 26.65, 25.19, 24.53, 22.48, 22.43, 22.41, 22.29. ppm. [M+2H]²⁺ for C₇₆H₁₂₀N₆O₅₆ calculated: 1007.34361, found 1007.34342.

### Microarrays

### Construction of microarrays

Immunopurified native schistosome CAA, synthetic di-, tetra-, hexa-, and octasaccharides with appropriate positive and negative controls were printed in triplicates onto epoxilane coated glass slides (Nexterion Slide E, Schott 1066643) with a MicroGrid robot (BioRobotics). See general methodology in (de Boer, Hokke et al. 2007).

### Microarray incubations

Microarrays with fitted silicone gaskets (64 samples/slide) were reconstituted in 1xPBS and blocked for minimum one hour (PBS, 2% bovine serum albumin (Sigma-Aldrich A3059) with 50mM ethanolamine (>99.5%, Sigma-Aldrich 411000)) on shaker. Each array washed in PBS Tween 0,05%, PBS and 30ul serum sample 1:100 in buffer/array added for 1hour incubation at room temperature on shaker. Arrays were then washed (as above) and 1:1000 detection antibodies added (goat anti-human IgG-Cy-3, Sigma-Aldrich C2571; goat anti-human IgM-AF647, Invitrogen, A21249) for 30min room temperature incubation on shaker. Finally, slides were washed as above with an additional wash in milli-Q before they were spun dry and scanned (Agilent Scan Control^{™} 2006). For nasosorption samples, they were incubated as above with the following changes. Samples were eluted in PBS 1% BSA 0.05% Triton-X and incubated as 1:2 dilution. For detection goat anti-human IgA-Dylight 650 (Novus NBP 1-75754) was used in 500x dilution.

### Data processing and analyses

Microarray scan images were obtained through Agilent Feature Extraction 10.7.3.1 and imported into GenePixPro vs7. Spot morphology quality was inspected and median fluorescence intensity (MFI) values obtained per nanodot with an average of a triplicate per target used for analyses. Further data processing was done in Microsoft Excel and graphs were generated using GraphPad Prism 9.3.1.

### Direct-CAA & Octamer-nanoparticle ELISAs and dotblots

***Synthetic octamer conjugated to nanoparticles:*** Conjugation of 30uM Octamer to 5nm gold nanoparticles was done according to manufacturer's instructions using NHS-Activated Gold Nanoparticle Conjugation Kit (Cytodiagnostics, NHS-Activated Gold Nanoparticles (N-Hydroxysuccinimide (PEG linker)-activated colloidal gold, 0,1mg CAS# 7440-57-5). Conjugated nanoparticles were stored in Dulbecco's (Sigma-Aldrich D8662) 1% w/v BSA (Bovine Serum Albumin, Sigma-Aldrich, A3059) 0,025% Tween20 (Merck, #822184) at 4°C.

### Solutions:

Sample/secondary detection antibody buffer: 1x PBS 0.05% Tween20, 4% BSA Coating buffer: 0,1 M Na-carbonate coating buffer: 0,1M NaHCO₃ (Sigma S5761) adjusted to pH 9.6 (pH strips, Machery-Nagel) with 0.1 M N Na₂CO₃ (Sigma S2127)
Blocking solution: 5 % BSA in PBS
*ELISA* wash buffer: 0.05% Tween in 1x PBS
*Immunoblot* wash buffer: 0.01% Tween in 1x PBS (PBST_{0,01})
*ELISA* substrate solution: 10 mg p-NPP ((4-Nitrophenyl phosphate magnesium salt, Sigma N 2507) in 10 ml DEA buffer (4,75ml DEA (diethylanolamine) and 250ul MgCL (2.03 mg MgCL2.6H2O / 10ml distilled water) adjusted to pH 9,8 with HCl in a total volume of 500ml, stored dark at 4°C.
*Immunoblot:* NBT/BCIP substrate solution: 1ml 1M Tris pH9.5, 200ul 5M NaCl, 50ul 1M MgCl2, 50ul BCIP (100% in DMF, X-phosphate/5-Bromo-4-chloro-3-indolyl-phosphate, Roche 760994), 50ul NBT (70% in DMF, 4-nitro blue tetrazolium chloride, Sigma-Aldrich N6876)
Samples: 7 paired controlled human infection (20 cercariae) baseline and 12 weeks post infection samples (1:20 dilution) and a sample pool from schistosomiasis endemic area (run in 1:20 and 1:100 dilutions)

### ELISA procedure:

100ul/well (Nunc maxisorp 96-well conical plates, 430341) 1ug/ml immunopurified CAA or octamer-nanoparticles (unknown exact quantity) in coating buffer. Incubation for 2 hours at room temperature. After three washes, 100ul/well blocking solution was added for 1 hour incubation at 37 °C on shaker. After four washes 50ul/well 1:20 diluted sample was added to relevant wells for 1 hour incubation at 37 °C on shaker. Before adding either anti-human IgG/IgM - AP antibodies (Goat anti-human IgM-AP (Sigma, A9794), Goat anti-human IgG-AP (Sigma, A9544)) in 1:2500 plates were washed four times. Incubation was done at 37 °C on shaker. Fresh substrate was prepared and 50ul/well added after four washes and incubation done dark without shaking. Absorbance was measured on ThermoFischer MultiScan ELISA reader at 405 nm (and 620nm for reference). Relevant controls were included in plates (blanks, no sample, no detection, no substrate, positive monoclonal antibody control for IgG anti-CAA (Humanised hIgG1-147-3G4-A monoclonal IgG1)).

### Immunoblot procedure:

Nitrocellulose membrane hole punched and placed in 96 well flat bottom polypropylene plate. Membrane "dots" were coated with 50ul/well and left to airdry o/n. 200ul/well block was done for 1 hour at 37 °C on shaker. Blocking solution was removed and 50ul/well sample was added for for 1 hour at 37 °C on shaker. After sample incubation, washing with PBST_{0,01} 100ul/well for 5min was done. Then 100ul/well 1:1000 respectively anti-human IgG/IgM - AP antibodies (Goat anti-human IgM-AP (Sigma, A9794), Goat anti-human IgG-AP (Sigma, A9544)) was added for 1 hour at 37 °C on shaker. Four times five minutes PBST_{0,01} washes followed by one wash in PBS only was done before freshly made NBT/BCIP substrate 50ul/well was added. Development was done dark and without shaking. After development washes in milli-Q were done. Pictures of immunoblots were taken both wet and dry. Relevant controls were included in wells (no sample, no detection, positive monoclonal antibody control for IgG anti-CAA (Humanised hIgG1-147-3G4-A monoclonal IgG1)).

### Results:

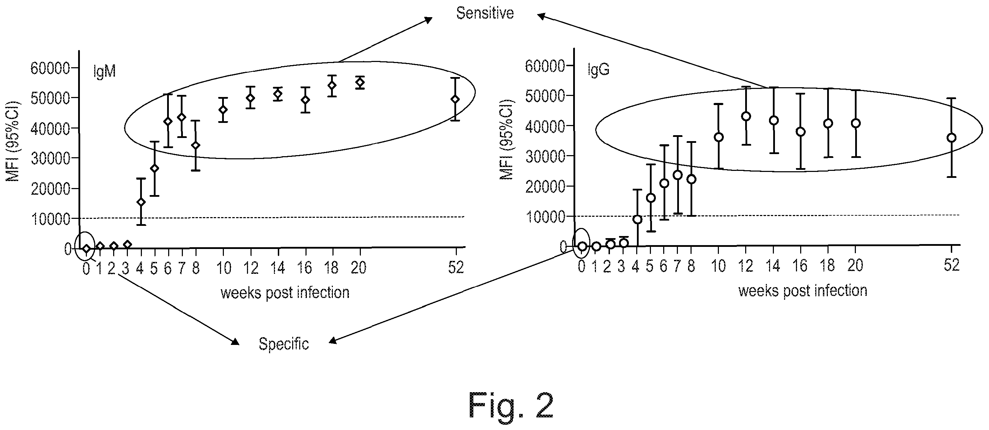

IgM and IgG antibodies binding to native CAA in controlled human infection samples (n=17, average mean fluorescence intensity (MFI)) is shown in **Figure 2****.**

Performance of synthetic CAA repeat structures should mimic responses to native CAA, both in terms of specificity (low background at baseline and early time points) and sensitivity in terms of having detectable response to the structures. The inventors have evaluated this on an individual level. Data is shown herein for three different infection doses; 10, 20, 30 cercariae (parasite larvae). Data for IgG and/or IgM for individuals (n=9, coded with letters) are shown from three different challenge groups.

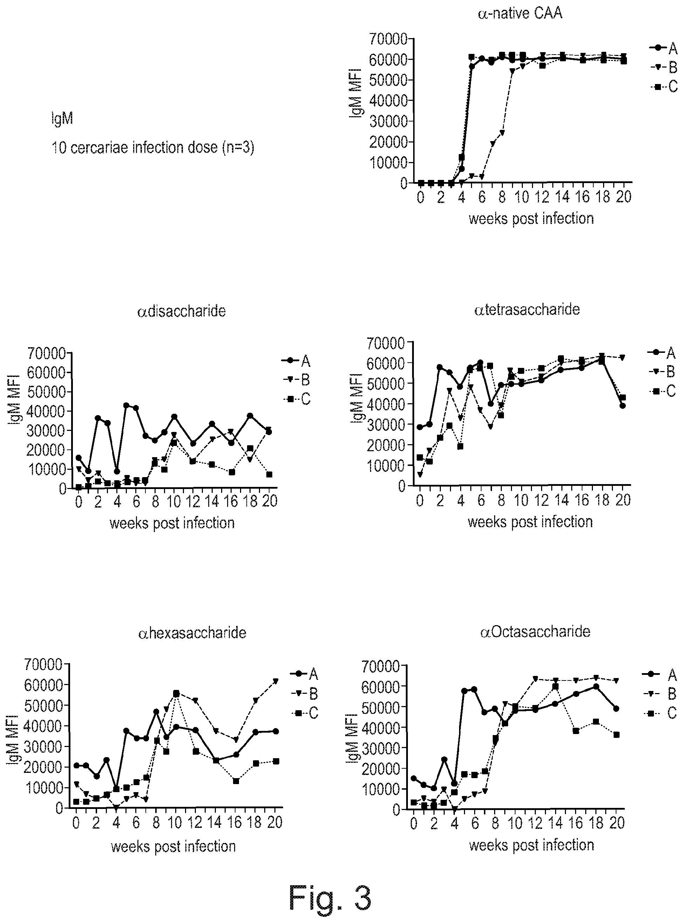

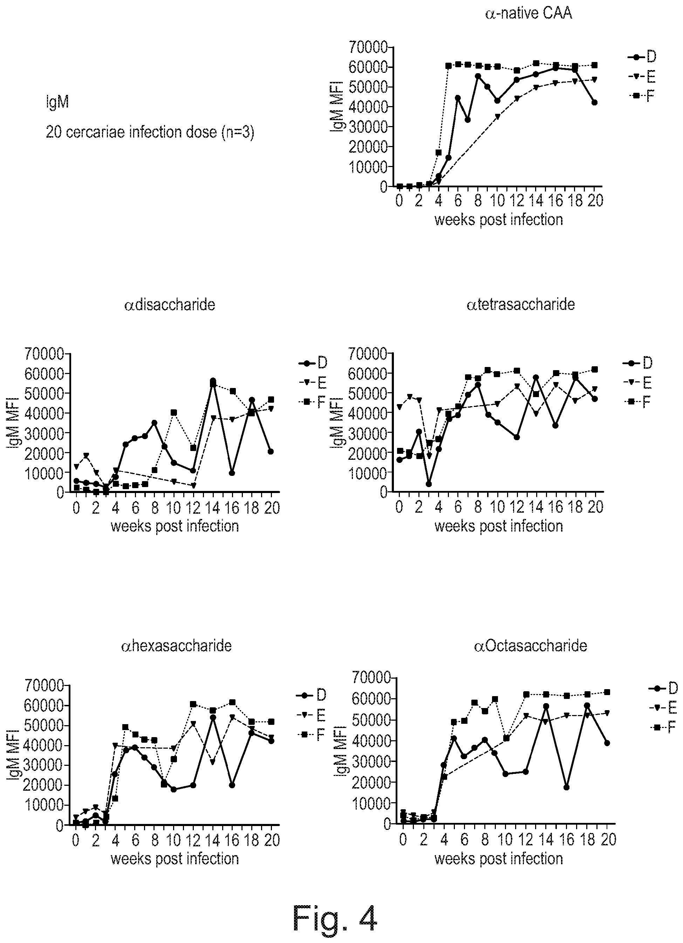

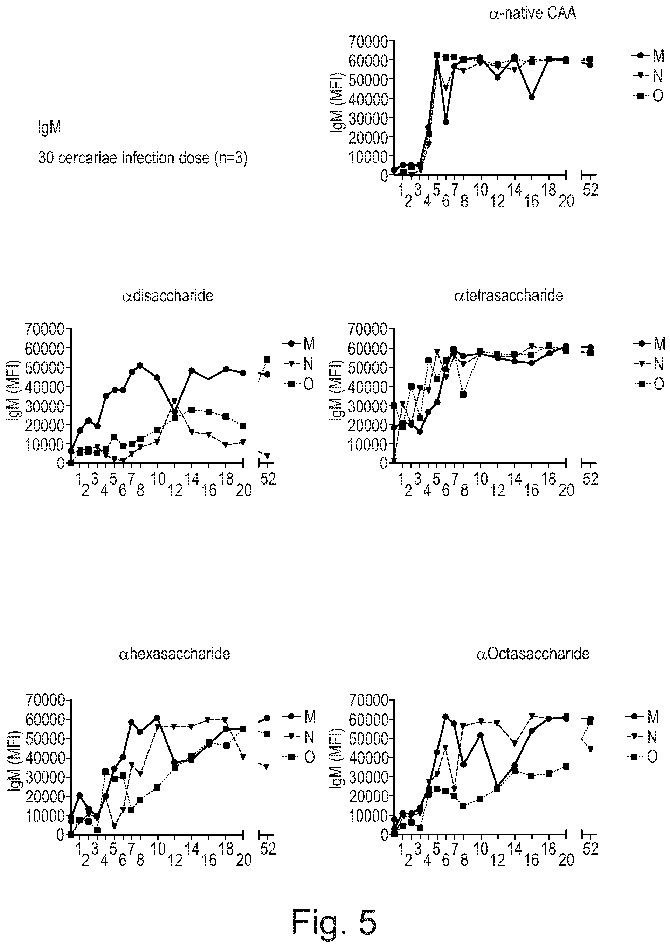

Three examples of how increasing length of structures show increasing specificity is shown in Figure 3, 4, 5 for IgM. Native CAA is shown as a control.
Individual level IgM responses to synthetic CAA repeats for infection with 10 cercariae (Figure 3 n=3t), 20 cercariae (Figure 4 n=3, note, no data day 5-9 incl) and 30 cercariae (Figure 5 n=3). Increasing structure length increases specificity as can be observed by the decrease in signal at baseline on individual level as well as emerging overall response pattern resemblance. The disaccharide is not representative of CAA (see Vermeer et al.). For the tetrasaccharide, a dynamic range resembling native material is starting to appear, but the background at baseline is too high indicating lack of specificity. The background at baseline is improved when the hexasaccharide is used, and improves further when an octasaccharide is used. Even at these very low infection doses, IgM binding to the synthetic structures can be detected. These data show that as the length of the polyssaccharide increases, the specificity and sensitivity of the assay improves.

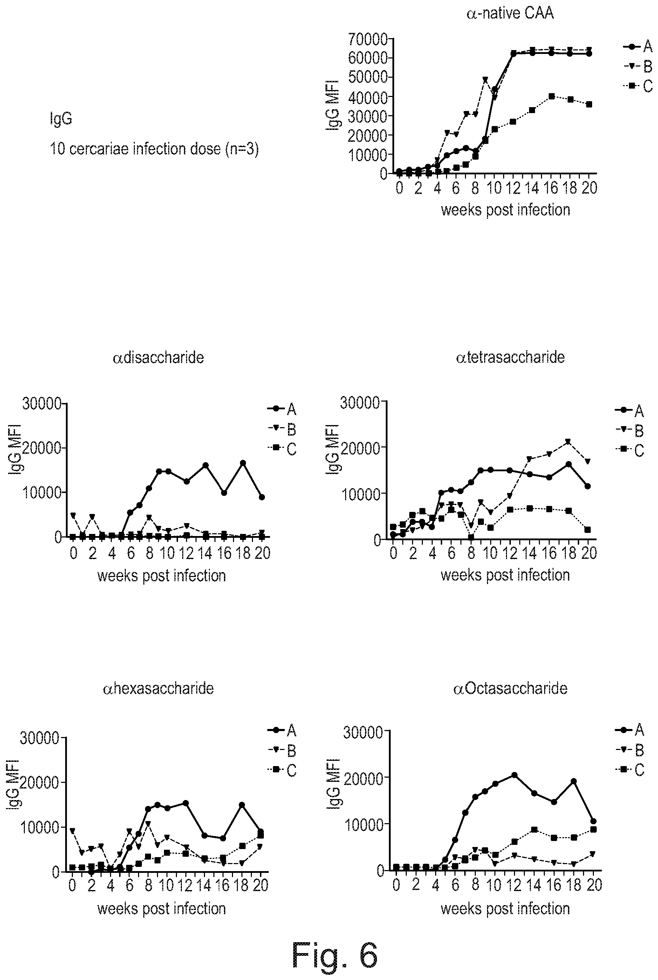

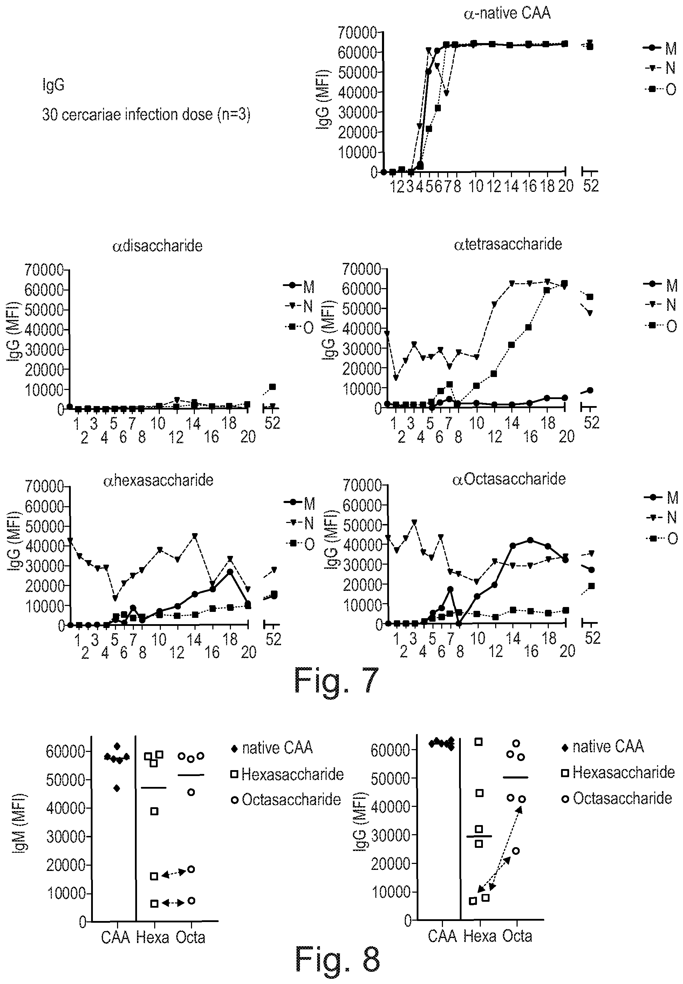

The antigen dose to induce isotype switch from IgM to IgG needs to be sufficient, so it is anticipated that IgG responses to synthetic CAA repeats will be lower than those to native CAA for the very low dose controlled human infections. Figure 6 and 7 show IgG responses to disaccharide, tetrasaccharide, hexasaccharide and octasaccharide for two groups of individuals infected with 10 (Figure 6 n=3) and 30 (Figure 7 n=3 ) cercariae. Antibody response to native CAA is included as control. Note that the axis for the lowest dose infection (10 cercariae, Figure 6) is different to better visualise the antibody dynamics. As observed for IgM, IgG responses to synthetic structures better recapitulate responses to native CAA as structure length increases.

The infection doses used in controlled human infections (10, 20, 30) are very low compared to what an individual is likely to be exposed to when for example swimming, rafting or washing in fresh water bodies with each infected snail capable of releasing hundreds to thousands of infective cercariae. Figure 8 show data from individuals naturally exposed to schistosomes. This data has been generated from six primary infection travel cases with a single exposure, where all individuals showed symptoms of acute schistosomiasis. This is an example of travellers swimming and being exposed naturally to cercariae in the water. It is likely that the dose from such an exposure is much higher than what is used for controlled human infection described in Figures 3 to 7 (10, 20, 30 cercariae). This is also reflected in the high antibody levels seen for all individuals for the synthetic structures as well as the native CAA.

IgM (Figure 8, left) and IgG (Figure 8, right) to native CAA and synthetic CAA-repeat hexasaccharide (hexa) and octasaccaride (octa). All individuals had high levels of antinative CAA antibodies, note that the response to the native CAA is complex and contains binding to several epitopes and therefore can be higher in magnitude than antibody responses to defined fragments comprising less available epitopes. Medians are indicated with horizontal bars. Arrows indicate samples belonging to the same individuals for IgM and IgG. The lowest responders for IgM and IgG are not the same individuals, meaning all individuals show high either IgM or IgG to the synthetic structures. With higher antigenic dose, a hexasaccharide can give high enough sensitivity for diagnostic purposes. **If** the response magnitude is increased, it is also possible to set technical cutoffs high enough to counter the background reactivity observed for some individuals in the controlled human infection sample set.

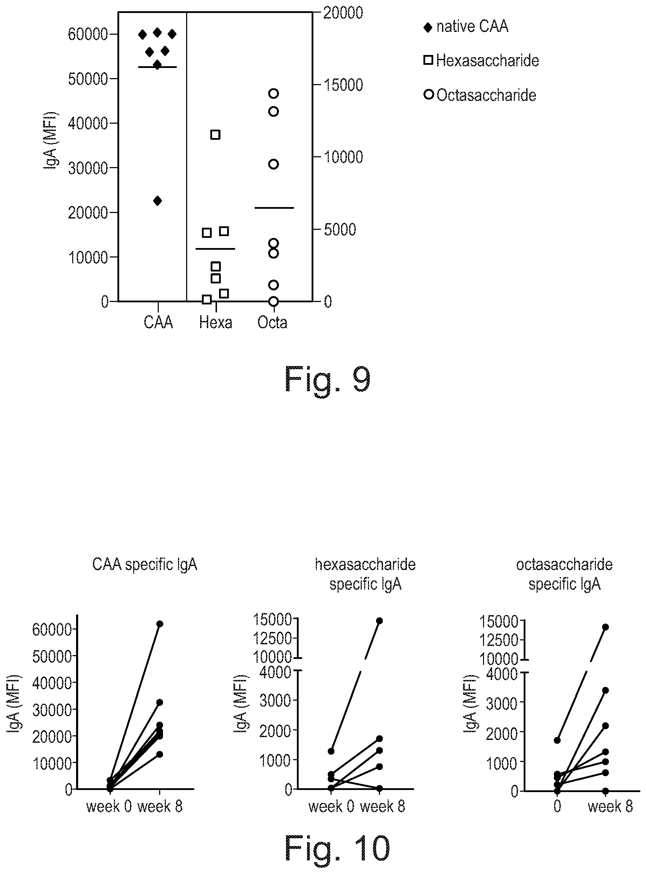

Figure 9 shows detection of IgA specific for native CAA, hexasaccharide and octasaccharide in serum samples from individuals infected with 20 cercariae (n=7) at eight weeks post infection. IgA antibodies to native CAA (left y-axis), hexa- and octasaccharide (right y-axis) are shown. This IgA is likely monomeric as it is measured in sera. Medians are indicated with horizontal bars. IgA specific for native CAA, hexasaccharide and octasaccharide is also detectable in nasosorption (non-invasive method) samples as shown in Figure 10. IgA antibodies specific for native CAA (left), for hexasaccharide (middle) and for octasaccharide (right) shown for the same individuals as Figure 9. This IgA is likely dimeric as it is of mucosal surface origin. Data are shown paired per individual at baseline and 8 weeks post infection.

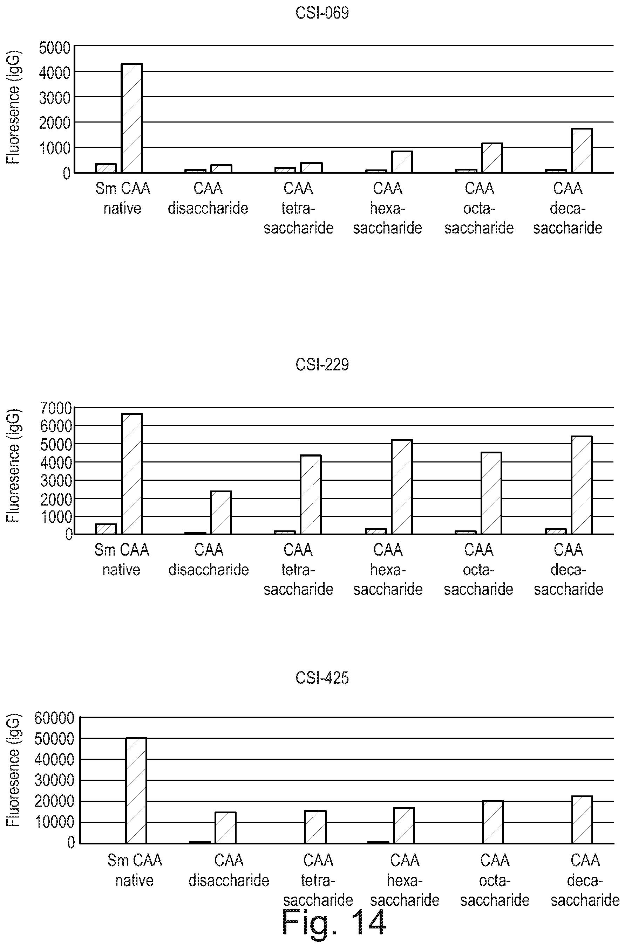

Figure 14 shows detection of IgG specific for CAA decasaccharide is equal or stronger than to shorter CAA fragments (CAA di- to octasaccharide), at 8 weeks after controlled human schistosome infection, with negligible pre-infection response. Results of three individual human serum IgG levels to CAA targets in arbitrary fluorescence units are shown (CSI-069, CSI-229, CSI-425). Dark grey bars, IgG response prior to infection; light grey bars, IgG response at week 8 after infection (exposure to 20 male S. *mansoni cercariae*); Sm CAA native, immunopurified native CAA.

The data shown in Figures 2 to 10 and 14 was generated using microarray experiments (details of the methodology used is found elsewhere herein). Through these microarray experiments the inventors have shown that antibodies recognising native CAA (carbohydrate antigen consisting of approximately 20 or more repetitive disaccharide units) are highly sensitive and specific for primary/acute schistosomiasis infection. Furthermore, they have determined that a minimum length of a hexasaccharide to an eicosaccharide will be needed for a synthetic structure to sufficiently mimic native CAA structure and be used as basis for measuring specific antibody responses in an accurate manner.

### Detection of antibodies specific for native CAA and synthetic octamer on alternative platforms

The inventors have also performed ELISAs and immunoblots to demonstrate that antibodies specific for native CAA and the synthetic CAA repeat octasaccharide structure can be detected on platforms frequently used in serology. The experiments were conducted as a proof of principle of that CAA and CAA repeat octasaccharide antibody reactivity can be measured in these alternative platforms as well.

### Assays:

- Direct CAA antigen ELISA
- Gold-nanoparticle conjugated synthetic octasaccharide ELISA
- Immunoblot (dotblot) for CAA and synthetic octasaccharide

It is to be expected, that direct CAA antigen coating and coating of gold-nanoparticle coupled octasaccharide can favour different antibody isotypes due to density and multivalence differences.

### Samples:

Seven paired baseline to 12 weeks post infection samples from controlled human schistosome infection were chosen as increase in titre post infection can be demonstrated per individual. Additionally, a known pool of schistosomiasis endemic area samples was used.

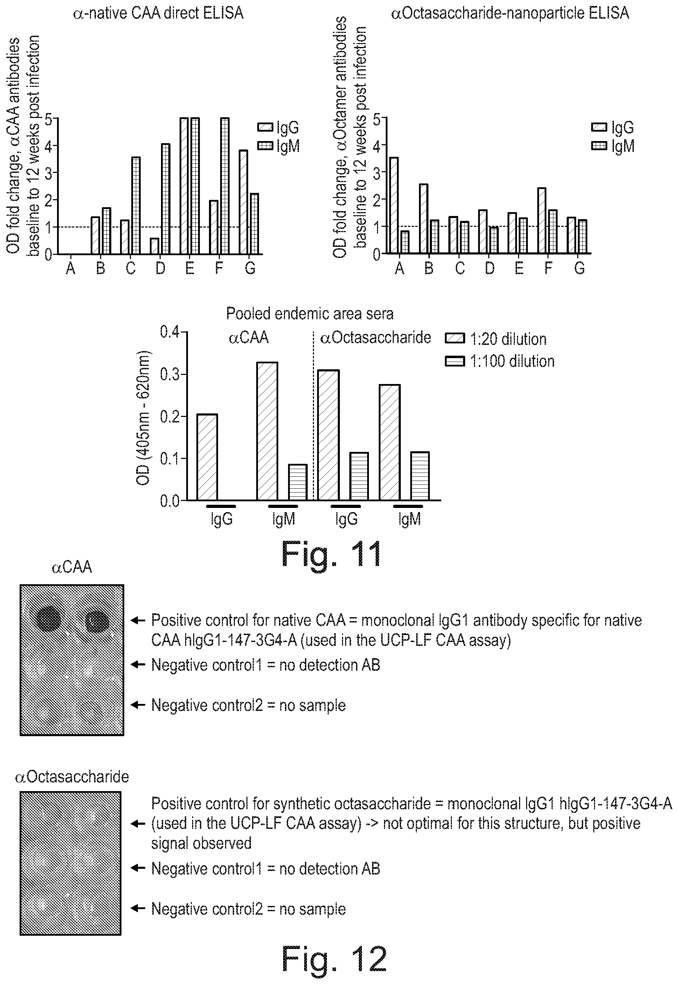

Figure 11 shows that IgM and IgG can be measured by ELISAs: direct antigen coating (CAA, top left) and gold nanoparticle-antigen conjugation coated (octasaccharide, top right). The data show an increase in IgM and IgG detection specific for CAA and octasaccharide from baseline to 12 weeks post infection detected by ELISAs for seven controlled human infection samples (A-G). Data shown as fold change in OD (optical density) from baseline to 12 weeks post infection per individual. Antibodies were also measured in a pool of schistosomiasis endemic area samples by ELISAs (bottom graph). Here the ODs for two dilutions (1:20 and 1:100) of sample are shown for IgG and IgM on both the direct anti-CAA and octasaccharide-gold nanoparticle ELISAs. This pooled sample has higher titers of specific antibodies than the individual controlled human infection samples.

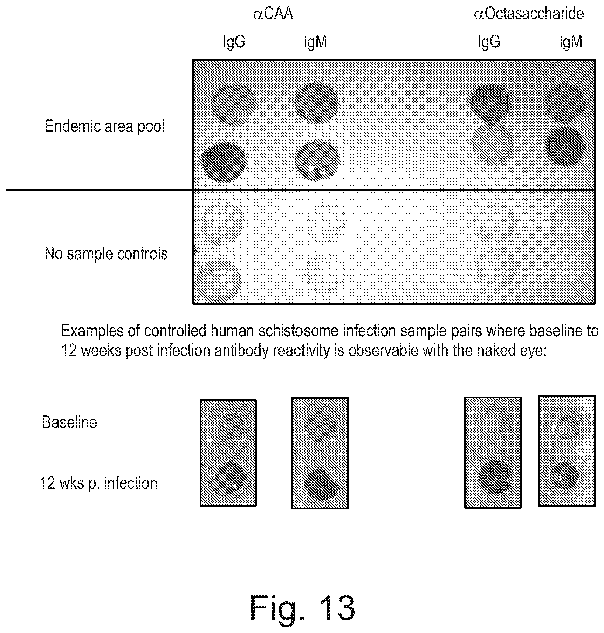

Figure 12 and Figure 13 show IgM and IgG measured by immunoblots as alternative platform: direct antigen coating (CAA) and gold nanoparticle-antigen conjugation coated (octasaccharide). The inventors demonstrate anti-CAA and anti-octasaccharide antibody binding on dot blots where nitrocellulose membrane was used to immobilize the antigen (native CAA, gold nanoparticle-conjugated octasaccharide). This material is frequently used in lateral flow test formats too, so points to future rapid diagnostic test applicability. α -IgG left, α- IgM right.

### Summary of findings:

- Increasing synthetic CAA repeat number increases specificity
- IgM performs better than IgG at lower repeat numbers, likely due to its pentavalent nature
- Both IgM and IgG require a degree of tertiary CAA-like structure (conformational epitope(s) essential for high accuracy)
- With either a longer structure or higher numbers of infective parasites, IgG also performs with higher sensitivity (refer to travel samples)
- IgA responses to native CAA as well as hexa- and octasaccharide is demonstrated in serum (monomeric IgA) and in nasosorption samples (dimeric IgA, non-invasive sampling site).

The methods described herein may be used as a diagnostic test for primary/acute schistosomiasis in non-endemic area travel medicine (including for people from non-endemic area entering an endemic region and suspected of primary infection/exposure). Such a test can be advantageous to current in house immunofluorescence assays (IFA) as observer variation can be eliminated. Furthermore, many travellers present with no eggs rendering stool/urine DNA detection methods as well as microscopy insufficiently sensitive. Antigen detection tests such as the UCP-LF CAA test are both sensitive and specific, however, some travellers still do not have high enough CAA levels to be detected and here an highly specific and sensitive antibody test is very valuable for differential and earlier diagnosis. Most existing serological tests are based on crude parasite antigen mixes, which contain cross-reactive epitopes, this defined synthetic antigen or antigen mix will have higher specificity. In addition, antibody detection in contrast to Ag detection is cheap, can easily be adapted to existing platforms, and is sensitive without sample treatment or instrumentation for readout.

The methods described herein may also be used for surveillance in emerging transmission zones. This relates to areas where climate change renders habitats suitable for intermediate hosts snails. With introduction of the parasite to such water bodies, novel transmission sites can emerge with introduction of parasites from urine, genital fluids and/or faecal matter containing viable eggs. Here surveillance can be done in local residents never before exposed to schistosomiasis and using suspected water bodies for occupational or recreational activities. Surveillance in people in contact with water bodies where compatible intermediate snails already exist, such as many places in the Mediterranean, is also relevant. Autochtonous schistosomiasis transmission has been recorded several times the last 100 years in Southern Europe, the latest in Corsica.

The methods described herein may also be used for surveillance for transmission mapping and prevalence estimations in endemic areas in targeted risk groups to inform intervention programmes. As antibody diagnostics cannot distinguish prior from current infection, serology must be used in defined risk groups such as young children, which are likely to have a recent primary infection. Alternatively, as 1st line screening tool in combination with second line current infection specific tools (which is often more expensive); this is the scenario for endemic areas in the WHO TPP.

The methods described herein may also be used for near and post-elimination as well as eradication surveillance. Screening in areas where schistosomiasis is believed to be eliminated and/or eradicated. The timing of when elimination and/or eradication status defines at which age individuals can be indicators for transmission recurrence.

The reader's attention is directed to all papers and documents which are filed concurrently with or previous to this specification in connection with this application and which are open to public inspection with this specification.

All of the features disclosed in this specification (including any accompanying claims, abstract and drawings), and/or all of the steps of any method or process so disclosed, may be combined in any combination, except combinations where at least some of such features and/or steps are mutually exclusive.

The invention is not restricted to the details of any foregoing embodiments.

### References

de Boer, A. R., et al. (2007). "General microarray technique for immobilization and screening of natural glycans." Anal Chem 79(21): 8107-8113.
Bergwerff, A. A., et al. (1994). "The immunologically reactive part of immunopurified circulating anodic antigen from Schistosoma mansoni is a threonine-linked polysaccharide consisting of -> 6)-(beta-D-GlcpA-(1 -> 3))-beta-D-GalpNAc-(1 -> repeating units." Journal of Biological Chemistry 269(50): 31510-31517.
Vermeer, H. J., et al. (2003). "Immunodiagnostically applicable monoclonal antibodies to the circulating anodic antigen of Schistosoma mansoni bind to small, defined oligosaccharide epitopes." Parasitol Res 90(4): 330-336.

## Claims

1. A method of detecting an *anti-Schistosoma* antibody in a biological sample obtained from a subject, the method comprising:
a) contacting the sample with a compound R-X-Y, wherein R is according to Formula I: 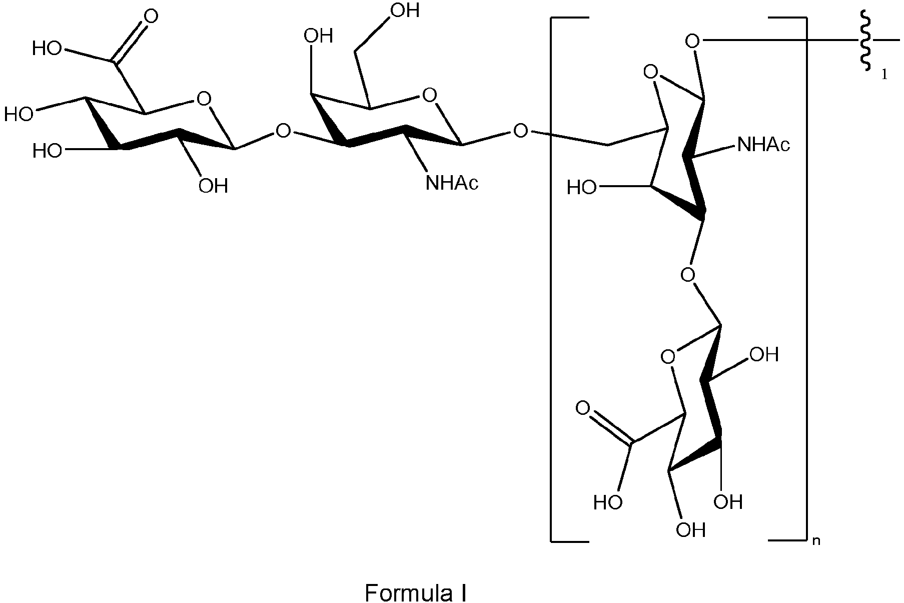
wherein n is a whole integer selected from 2 to 19; and
wherein 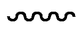 1 denotes the point of attachment of R to X, wherein X is selected from the group consisting of: a bond and a linker; and wherein Y is selected from the group consisting of: H, a reporter molecule, a carrier molecule, and a solid support; and b) determining if binding between the sample and the compound occurs;
wherein binding between the sample and the compound is indicative of the presence of the antibody in the sample.

2. The method of claim 1, wherein:
A) n = 2, 3, 4, or 5; and/or
B) X is C₂-C₁₀-alkylene-NH- (e.g. C₆H₁₂NH-); optionally wherein:
(i) Y is H; or
(ii) Y is a reporter molecule, a carrier molecule, or a solid support; and/or
C) the presence of the antibody is indicative of a current or prior *Schistosoma* infection in the subject; and/or
D) the sample is obtained from a subject that is at risk of contracting a *Schistosoma* infection and/or has one or more symptoms indicative of a *Schistosoma* infection, optionally wherein:
(i) the subject is at risk of contracting a *Schistosoma* infection if they have been to a location where *Schistosoma* infection is prevalent and/or endemic; or
(ii) symptoms indicative of a *Schistosoma* infection include: a skin rash, fever, headache, myalgia, general malaise, diarrhoea, non-productive cough, muscle pain, joint pain, abdominal tenderness or pain, hepatosplenomegaly, eosinophilia, urticaria, angiooedema, (micro)haematuria, transient pulmonary infiltrates and/or parasite eggs in one or more of stool, urine and genital fluids; and/or
E) the sample is obtained from the subject at least 4 weeks after having one or more symptoms indicative of a *Schistosoma* infection, optionally wherein symptoms indicative of a *Schistosoma* infection include: a skin rash, fever, headache, myalgia, general malaise, diarrhoea, non-productive cough, muscle pain, joint pain, abdominal tenderness or pain, hepatosplenomegaly, eosinophilia, urticaria, angiooedema, (micro)haematuria, transient pulmonary infiltrates and/or parasite eggs in one or more of stool, urine and genital fluids.

3. A method of monitoring a subject to determine if they have contracted a *Schistosoma* infection, the method comprising:
a) contacting a first biological sample obtained from the subject at a first time point with a compound R-X-Y, wherein R is according to Formula I: 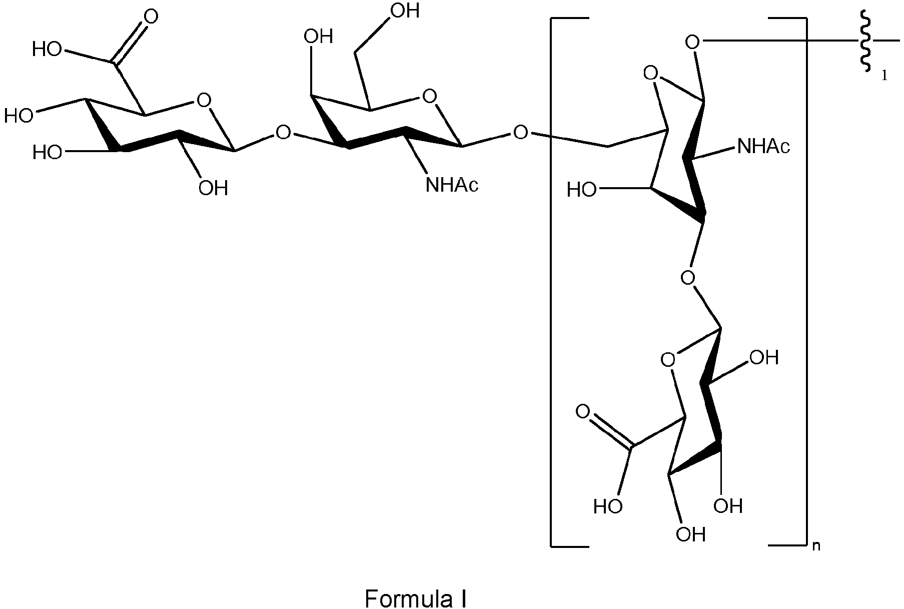 wherein 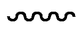 1 denotes the point of attachment of R to X, wherein X is selected from the group consisting of: a bond and a linker; and wherein Y is selected from the group consisting of: H, a reporter molecule, a carrier molecule, and a solid support, and n is a whole integer selected from 2 to 19; and determining if binding between the compound and the first sample occurs;
b) contacting a second biological sample with the compound, wherein the second sample is obtained from the subject at a later time point than the first sample, and determining if binding between the compound and the second sample occurs;
c) comparing the binding determined in step b) with the binding determined in step a), wherein an increase in b) as compared to a) is indicative of the subject having contracted the infection.

4. The method of claim 3, wherein:
A) n = 2, 3, 4, or 5; and/or
B) X is --C₂-C₁₀-alkylene-NH- (e.g. C₆H₁₂NH-), optionally wherein:
(i) Y is H or
(ii) Y is a reporter molecule, a carrier molecule, or a solid support; and/or
C) the time period between the first and second sample is at least 4 weeks; and/or
D) an increase in b) as compared to a) is indicative of the subject having contracted the infection between the first time point and later time point; and/or
E) the second sample is obtained from a subject that is at risk of contracting the *Schistosoma* infection and/or has one or more symptoms indicative of the *Schistosoma* infection, optionally wherein:
(i) the subject is at risk if they have been to a location where *Schistosoma* infection is prevalent and/or endemic; or
(ii) the symptoms include: a skin rash, fever, headache, myalgia, general malaise, diarrhoea, non-productive cough, muscle pain, joint pain, abdominal tenderness or pain, hepatosplenomegaly, eosinophilia, urticaria, angiooedema, (micro)haematuria, transient pulmonary infiltrates and/or parasite eggs in one or more of stool, urine and genital fluids; and/or
F) the second sample is obtained from the subject at least 4 weeks after having one or more symptoms indicative of the *Schistosoma* infection, optionally wherein the symptoms include: a skin rash, fever, headache, myalgia, general malaise, diarrhoea, non-productive cough, muscle pain, joint pain, abdominal tenderness or pain, hepatosplenomegaly, eosinophilia, urticaria, angiooedema, (micro)haematuria, transient pulmonary infiltrates and/or parasite eggs in one or more of stool, urine and genital fluids.

5. The method of any one of the preceding claims, wherein the subject has:
A) acute schistosomiasis; and/or
B) a primary *Schistosoma* infection.

6. The method of any one of claims 2 (C) to 5, wherein the *Schistosoma* infection is caused by a *Schistosoma* species selected from the group consisting of: *Schistosoma mansoni, Schistosoma japonicum, Schistosoma mekongi, Schistosoma guineensis, Schistosoma intercalatum,* and *Schistosoma haematobium.*

7. The method of any one of the preceding claims, wherein:
A) the antibody is selected from the group consisting of: IgM, IgA, and IgG; and/or
B) the subject is human; and/or
C) the sample is selected from the group consisting of: a blood sample, nasal sample, urine sample, genital fluid sample and a stool sample, optionally wherein:
(i) the blood sample is a serum sample, plasma sample, or a whole blood sample, further optionally when the sample is a blood sample, the antibody is IgM and/or IgG; or
(ii) the nasal sample is obtained by nasosorption sampling, further optionally when the sample is a nasal sample or a blood sample, the antibody is IgA; or
(iii) when the sample is a nasal sample or a blood sample, the antibody is IgA,
(iv) when the sample is a blood sample, the antibody is IgM and/or IgG; and/or
D) binding between the sample and the compound is detected by a technique selected from the group consisting of: microarray assay, ELISA, immunoblotting assay, bead-based multiplex assay, and lateral-flow assay.

8. A compound R-X-Y, wherein R is according to Formula I: 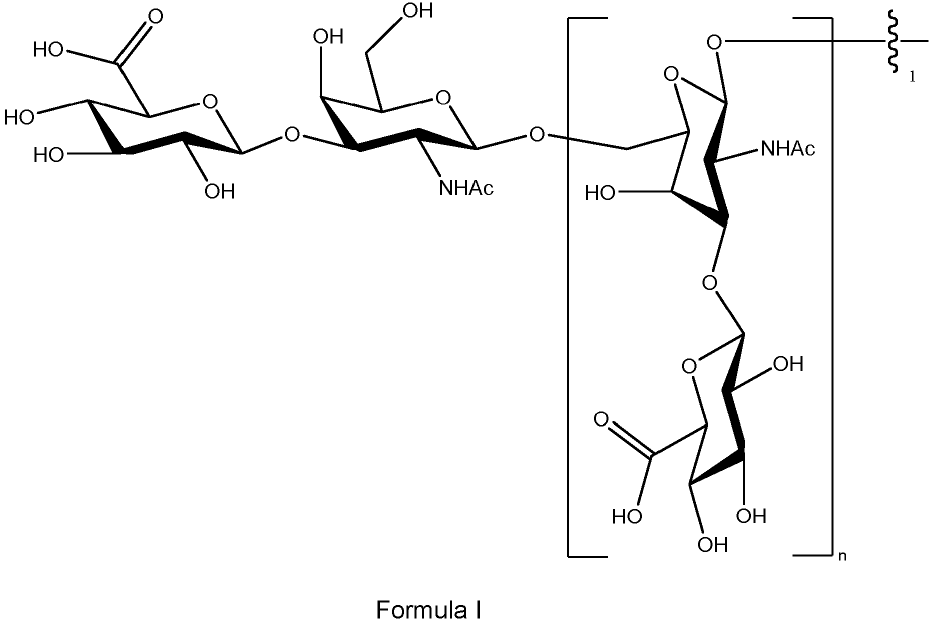 wherein 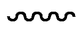 1 denotes the point of attachment of R to X, wherein X is selected from the group consisting of: a bond and a linker; and Y is selected from the group consisting of: H, a reporter molecule, a carrier molecule, and a solid support; and n is whole integer selected from 2 to 19.

9. The compound of claim 8, wherein:
A) n = 2, 3, 4, or 5; and/or
B) the X is --C₂-C₁₀-alkylene-NH- (e.g. C₆H₁₂NH-), optionally wherein:
(i) Y is H or
(ii) Y is a reporter molecule, a carrier molecule, or a solid support; and/or
C) Y comprises a solid support, optionally wherein the solid support is selected from the group consisting of a membrane, plastic, polymer, and glass.

10. A composition comprising a plurality of distinct compounds according to claim 8.

11. The composition of claim 10, wherein the composition comprises at least two compounds selected from the group consisting of:
(i) a compound of Formula I, wherein n = 2;
(ii) a compound of Formula I, wherein n = 3;
(iii) a compound of Formula I, wherein n = 4; and
(iv) a compound of Formula I, wherein n = 5.

## Patentansprüche

1. Verfahren zum Nachweisen eines *Anti-Schistosoma-Antikörpers* in einer biologischen Probe, die von einem Subjekt erlangt wurde, wobei das Verfahren Folgendes umfasst:
a) Inkontaktbringen der Probe mit einer Verbindung R-X-Y, wobei R gemäß der Formel I ist: 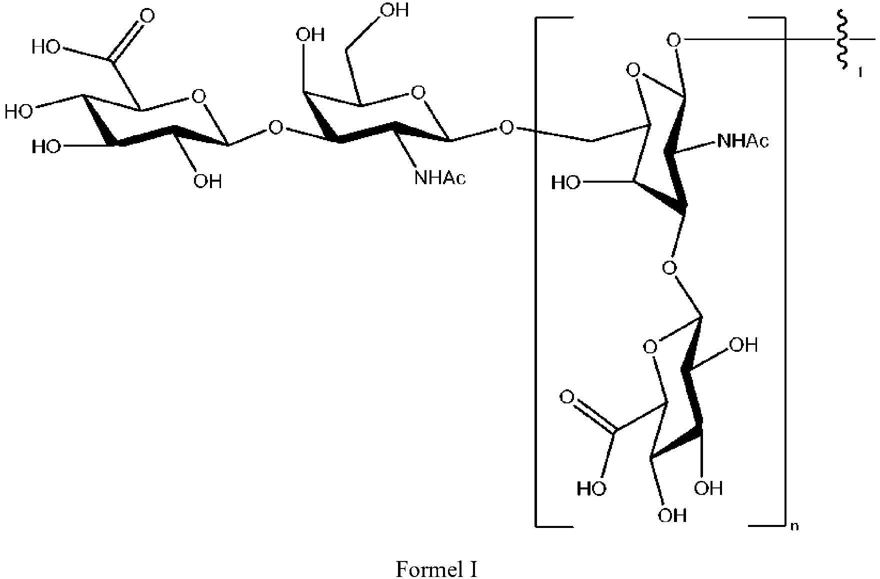
wobei n eine ganze Ganzzahl ausgewählt aus 2 bis 19 ist; und
wobei 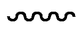 1 den Bindungspunkt von R an X bezeichnet, wobei X ausgewählt ist aus der Gruppe bestehend aus: einer Bindung und einem Linker; und wobei Y ausgewählt ist aus der Gruppe bestehend aus: H, einem Reportermolekül, einem Transportmolekül und einem festen Träger; und
b) Bestimmen, ob ein Binden zwischen der Probe und der Verbindung auftritt;
wobei das Binden zwischen der Probe und der Verbindung das Vorhandensein des Antikörpers in der Probe angibt.

2. Verfahren nach Anspruch 1, wobei:
A) n = 2, 3, 4 oder 5; und/oder
B) X C₂-C₁₀-Alkylen-NH- (z. B. C₆H₁₂NH-) ist; optional wobei:
(i) Y H ist; oder
(ii) Y ein Reportermolekül, ein Transportmolekül oder ein fester Träger ist; und/oder
C) das Vorhandensein des Antikörpers eine aktuelle oder frühere *Schistosoma-Infektion* bei dem Subjekt angibt; und/oder
D) die Probe von einem Subjekt erlangt ist, das in Gefahr ist, sich eine *Schistosoma-*Infektion zuzuziehen, und/oder ein oder mehrere Symptome aufweist, die eine *Schistosoma-*Infektion angeben, wobei optional:
(i) das Subjekt in Gefahr ist, sich eine *Schistosoma-Infektion* zuzuziehen, falls es an einem Ort gewesen ist, an dem eine *Schistosoma-Infektion* verbreitet und/oder endemisch ist; oder
(ii) Symptome, die eine *Schistosoma-Infektion* angeben, Folgendes enthalten: einen Hautausschlag, Fieber, Kopfschmerzen, Myalgie, allgemeines Unwohlsein, Durchfall, unproduktiven Husten, Muskelschmerzen, Gelenkschmerzen, abdominale Druckempfindlichkeit oder Schmerzen, Hepatosplenomegalie, Eosinophilie, Urtikaria, Angioödem, (Mikro-)Hämaturie, vorübergehende Lungeninfiltrate und/oder Parasiteneier in einem oder mehreren von Stuhl, Urin und Genitalflüssigkeiten; und/oder
E) die Probe von dem Subjekt mindestens 4 Wochen nach Aufweisen von einem oder mehreren Symptomen, die eine *Schistosoma-Infektion* angeben, erlangt wird, wobei die Symptome, die eine *Schistosoma-Infektion* angeben, optional Folgendes enthalten: einen Hautausschlag, Fieber, Kopfschmerzen, Myalgie, allgemeines Unwohlsein, Durchfall, unproduktiven Husten, Muskelschmerzen, Gelenkschmerzen, abdominale Druckempfindlichkeit oder Schmerzen, Hepatosplenomegalie, Eosinophilie, Urtikaria, Angioödem, (Mikro-)Hämaturie, vorübergehende Lungeninfiltrate und/oder Parasiteneier in einem oder mehreren von Stuhl, Urin und Genitalflüssigkeiten.

3. Verfahren zum Überwachen eines Subjekts, um zu bestimmen, ob es sich eine *Schistosoma-Infektion* zugezogen hat, wobei das Verfahren Folgendes umfasst:
a) Inkontaktbringen einer ersten biologischen Probe, die von dem Subjekt zu einem ersten Zeitpunkt erlangt wurde, mit einer Verbindung R-X-Y, wobei R gemäß der Formel I ist: 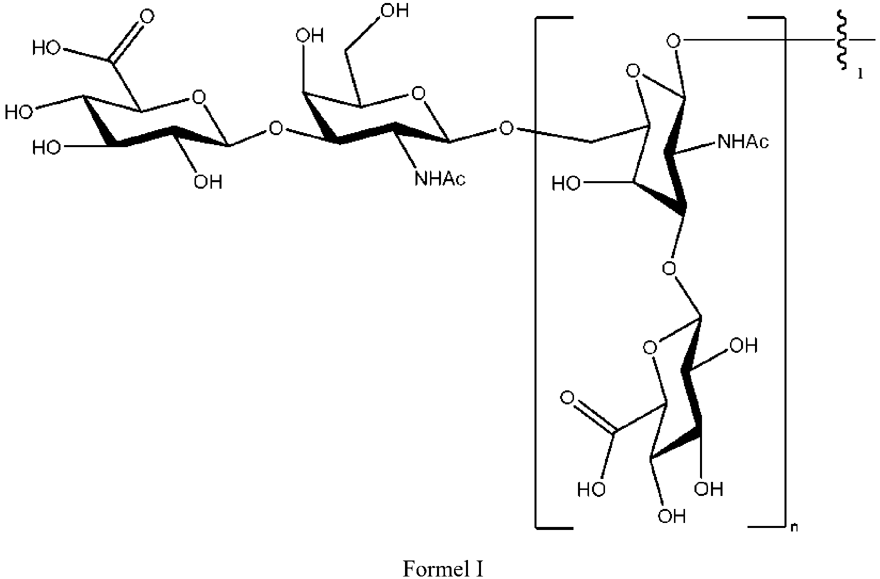 wobei 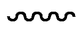 1 den Bindungspunkt von R an X bezeichnet, wobei X ausgewählt ist aus der Gruppe bestehend aus: einer Bindung und einem Linker; und wobei Y ausgewählt ist aus der Gruppe bestehend aus: H, einem Reportermolekül, einem Transportmolekül und einem festen Träger, und wobei n eine ganze Ganzzahl ausgewählt aus 2 bis 19 ist; und Bestimmen, ob ein Binden zwischen der Verbindung und der ersten Probe auftritt;
b) Inkontaktbringen einer zweiten biologischen Probe mit der Verbindung, wobei die zweite Probe von dem Subjekt zu einem späteren Zeitpunkt als die erste Probe erlangt wird, und Bestimmen, ob ein Binden zwischen der Verbindung und der zweiten Probe auftritt;
c) Vergleichen des in Schritt b) bestimmten Bindens mit dem in Schritt a) bestimmten Binden, wobei eine Erhöhung in b) im Vergleich zu a) angibt, dass sich das Subjekt die Infektion zugezogen hat.

4. Verfahren nach Anspruch 3, wobei:
A) n = 2, 3, 4 oder 5; und/oder
B) X --C₂-C₁₀-Alkylen-NH- (z. B. C₆H₁₂NH-) ist, optional wobei:
(i) Y H ist oder
(ii) Y ein Reportermolekül, ein Transportmolekül oder ein fester Träger ist; und/oder
C) der Zeitraum zwischen der ersten und der zweiten Probe mindestens 4 Wochen beträgt; und/oder
D) eine Erhöhung in b) im Vergleich zu a) angibt, dass sich das Subjekt die Infektion zwischen dem ersten Zeitpunkt und dem späteren Zeitpunkt zugezogen hat; und/oder
E) die zweite Probe von einem Subjekt erlangt ist, das in Gefahr ist, sich die *Schistosoma-*Infektion zuzuziehen, und/oder ein oder mehrere Symptome aufweist, die die *Schistosoma-*Infektion angeben, wobei optional:
(i) das Subjekt in Gefahr ist, falls es an einem Ort gewesen ist, an dem eine *Schistosoma-*Infektion verbreitet und/oder endemisch ist; oder
(ii) die Symptome Folgendes enthalten: einen Hautausschlag, Fieber, Kopfschmerzen, Myalgie, allgemeines Unwohlsein, Durchfall, unproduktiven Husten, Muskelschmerzen, Gelenkschmerzen, abdominale Druckempfindlichkeit oder Schmerzen, Hepatosplenomegalie, Eosinophilie, Urtikaria, Angioödem, (Mikro-)Hämaturie, vorübergehende Lungeninfiltrate und/oder Parasiteneier in einem oder mehreren von Stuhl, Urin und Genitalflüssigkeiten; und/oder
F) die zweite Probe von dem Subjekt mindestens 4 Wochen nach Aufweisen von einem oder mehreren Symptomen, die die *Schistosoma-*Infektion angeben, erlangt wird, wobei die Symptome optional Folgendes enthalten: einen Hautausschlag, Fieber, Kopfschmerzen, Myalgie, allgemeines Unwohlsein, Durchfall, unproduktiven Husten, Muskelschmerzen, Gelenkschmerzen, abdominale Druckempfindlichkeit oder Schmerzen, Hepatosplenomegalie, Eosinophilie, Urtikaria, Angioödem, (Mikro-)Hämaturie, vorübergehende Lungeninfiltrate und/oder Parasiteneier in einem oder mehreren von Stuhl, Urin und Genitalflüssigkeiten.

5. Verfahren nach einem der vorhergehenden Ansprüche, wobei das Subjekt Folgendes aufweist:
A) akute Schistosomiasis; und/oder
B) eine primäre *Schistosoma-*Infektion.

6. Verfahren nach einem der Ansprüche 2 (C) bis 5, wobei die *Schistosoma*-Infektion durch eine *Schistosoma*-Spezies verursacht wird, die aus der Gruppe ausgewählt ist, bestehend aus: *Schistosoma mansoni, Schistosoma japonicum, Schistosoma mekongi, Schistosoma guineensis, Schistosoma intercalatum* und *Schistosoma haematobium.*

7. Verfahren nach einem der vorhergehenden Ansprüche, wobei:
A) der Antikörper ausgewählt ist aus der Gruppe bestehend aus: IgM, IgA und IgG; und/oder
B) das Subjekt menschlich ist; und/oder
C) die Probe ausgewählt ist aus der Gruppe bestehend aus: einer Blutprobe, einer nasalen Probe, einer Urinprobe, einer Genitalflüssigkeitsprobe und einer Stuhlprobe, wobei optional:
(i) die Blutprobe eine Serumprobe, Plasmaprobe oder eine Vollblutprobe ist, ferner optional wobei der Antikörper IgM und/oder IgG ist, wenn die Probe eine Blutprobe ist; oder
(ii) die nasale Probe durch Nasosorptionsprobenahme erlangt wird, wobei der Antikörper ferner optional IgA ist, wenn die Probe eine nasale Probe oder eine Blutprobe ist; oder
(iii) wenn die Probe eine nasale Probe oder eine Blutprobe ist, der Antikörper IgA ist,
(iv) wenn die Probe eine Blutprobe ist, der Antikörper IgM und/oder IgG ist; und/oder
D) ein Binden zwischen der Probe und der Verbindung durch eine Technik nachgewiesen wird, die ausgewählt ist aus der Gruppe bestehend aus: Mikroarray-Assay, ELISA, Immunoblotting-Assay, perlenbasiertem Multiplex-Assay und Lateral-Flow-Assay.

8. Verbindung R-X-Y, wobei R gemäß der Formel I ist: 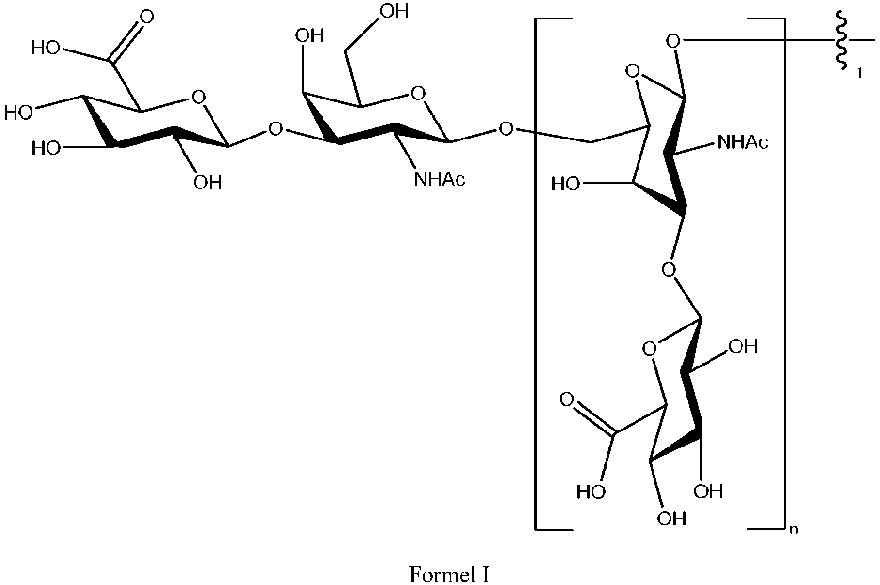 wobei 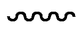 1 den Bindungspunkt von R an X bezeichnet, wobei X ausgewählt ist aus der Gruppe bestehend aus: einer Bindung und einem Linker; und Y ausgewählt ist aus der Gruppe bestehend aus: H, einem Reportermolekül, einem Transportmolekül und einem festen Träger; und n eine ganze Ganzzahl ausgewählt aus 2 bis 19 ist.

9. Verbindung nach Anspruch 8, wobei:
A) n = 2, 3, 4 oder 5; und/oder
B) das X --C₂-C₁₀-Alkylen-NH- (z. B. C₆H₁₂NH-) ist, optional wobei:
(i) Y H ist oder
(ii) Y ein Reportermolekül, ein Transportmolekül oder ein fester Träger ist; und/oder
C) Y einen festen Träger umfasst, wobei optional der feste Träger ausgewählt ist aus der Gruppe bestehend aus einer Membran, Kunststoff, Polymer und Glas.

10. Zusammensetzung, umfassend eine Vielzahl von unterschiedlichen Verbindungen gemäß Anspruch 8.

11. Zusammensetzung nach Anspruch 10, wobei die Zusammensetzung mindestens zwei Verbindungen umfasst, die ausgewählt sind aus der Gruppe bestehend aus:
(i) einer Verbindung der Formel I, wobei n = 2;
(ii) einer Verbindung der Formel I, wobei n = 3;
(iii) einer Verbindung der Formel I, wobei n = 4; und
(iv) einer Verbindung der Formel I, wobei n = 5.

## Revendications

1. Procédé de détection d'un anticorps *anti-Schistosoma* dans un échantillon biologique obtenu auprès d'un sujet, le procédé comprenant :
a) la mise en contact de l'échantillon avec un composé R-X-Y, dans lequel R est conforme à la Formule I : 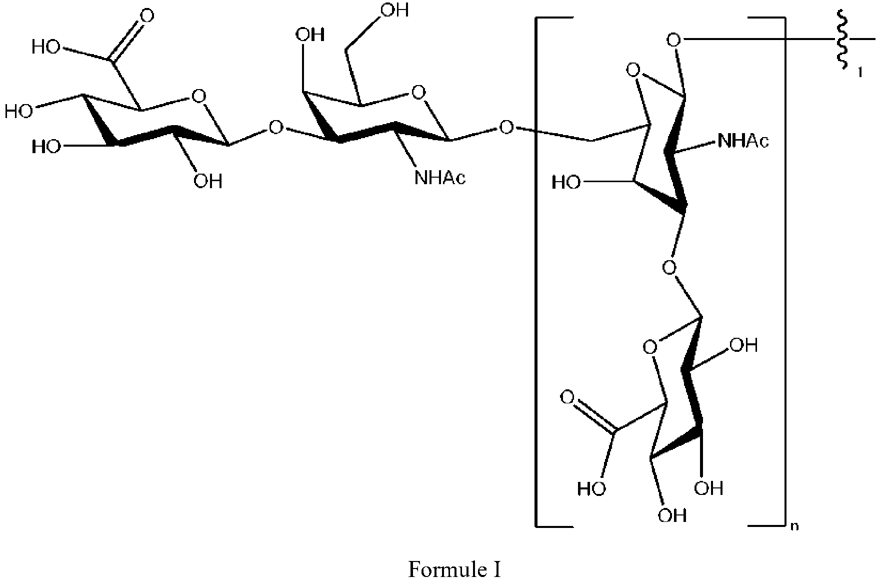
dans lequel n est un nombre entier choisi entre 2 et 19 ; et
dans lequel 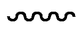 1 désigne le point de fixation de R à X, dans lequel X est choisi dans le groupe constitué par : une liaison et un coupleur ; et dans lequel Y est choisi dans le groupe constitué par : H, une molécule rapporteuse, une molécule porteuse et un support solide ; et
b) la détermination de l'existence d'une liaison entre l'échantillon et le composé ;
dans lequel la liaison entre l'échantillon et le composé est indicative de la présence de l'anticorps dans l'échantillon.

2. Procédé de la revendication 1, dans lequel :
A) n = 2, 3, 4 ou 5 ; et/ou
B) X est alkylène en C₂-C₁₀-NH- (par exemple C₆H₁₂NH-) ; éventuellement dans lequel :
(i) Y est H ; ou
(ii) Y est une molécule rapporteuse, une molécule porteuse ou un support solide ; et/ou
C) la présence de l'anticorps est indicative d'une infection *à Schistosoma* actuelle ou antérieure chez le sujet ; et/ou
D) l'échantillon est obtenu auprès d'un sujet présentant un risque de contracter une infection *à Schistosoma* et/ou présentant un ou plusieurs symptômes indicatifs d'une infection à *Schistosoma,* éventuellement dans lequel :
(i) le sujet présente un risque de contracter une infection *à Schistosoma* s'il s'est rendu dans un endroit où l'infection *à Schistosoma* est répandue et/ou endémique ; ou
(ii) les symptômes indicatifs d'une infection *à Schistosoma* comprennent : une éruption cutanée, de la fièvre, des maux de tête, des myalgies, un malaise général, une diarrhée, une toux non productive, des douleurs musculaires, des douleurs articulaires, une sensibilité ou une douleur abdominale, une hépatosplénomégalie, une éosinophilie, une urticaire, un angio-œdème, une (micro)hématurie, des infiltrats pulmonaires transitoires et/ou des œufs de parasites dans un ou plusieurs des selles, de l'urine et des fluides génitaux ; et/ou
E) l'échantillon est obtenu auprès du sujet au moins 4 semaines après l'apparition d'un ou de plusieurs symptômes indicatifs d'une infection *à Schistosoma,* éventuellement dans lequel les symptômes indicatifs d'une infection *à Schistosoma* comprennent : une éruption cutanée, de la fièvre, des maux de tête, des myalgies, un malaise général, une diarrhée, une toux non productive, des douleurs musculaires, des douleurs articulaires, une sensibilité ou une douleur abdominale, une hépatosplénomégalie, une éosinophilie, une urticaire, un angio-œdème, une (micro)hématurie, des infiltrats pulmonaires transitoires et/ou des œufs de parasites dans un ou plusieurs des selles, de l'urine et des fluides génitaux.

3. Procédé de surveillance d'un sujet pour déterminer s'il a contracté une infection à *Schistosoma,* le procédé comprenant :
a) la mise en contact d'un premier échantillon biologique obtenu auprès du sujet à un premier instant avec un composé R-X-Y, dans lequel R est conforme à la Formule I : 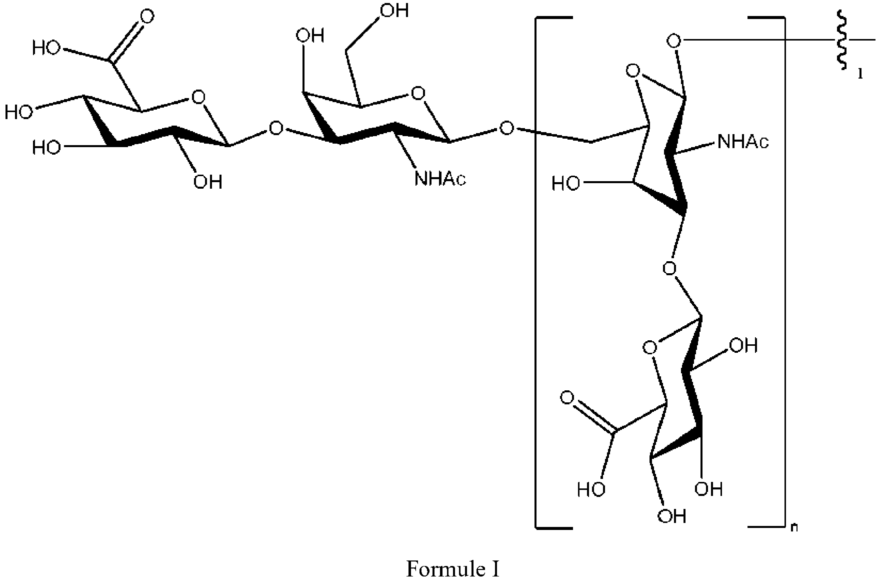 dans lequel 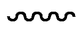 1 désigne le point de fixation de R à X, dans lequel X est choisi dans le groupe constitué par : une liaison et un coupleur ; et dans lequel Y est choisi dans le groupe constitué par : H, une molécule rapporteuse, une molécule porteuse et un support solide, et n est un nombre entier choisi entre 2 et 19 ; et la détermination de l'existence d'une liaison entre le composé et le premier échantillon ;
b) la mise en contact d'un second échantillon biologique avec le composé, dans lequel le second échantillon est obtenu auprès du sujet à un instant ultérieur au premier échantillon, et la détermination de l'existence d'une liaison entre le composé et le second échantillon ;
c) la comparaison de la liaison déterminée à l'étape b) avec la liaison déterminée à l'étape a), dans lequel une augmentation à l'étape b) par rapport à l'étape a) indique que le sujet a contracté l'infection.

4. Procédé de la revendication 3, dans lequel :
A) n = 2, 3, 4 ou 5 ; et/ou
B) X est --alkylène en C₂-C₁₀-NH- (par exemple C₆H₁₂NH-), éventuellement dans lequel :
(i) Y est H ou
(ii) Y est une molécule rapporteuse, une molécule porteuse ou un support solide ; et/ou
C) la période de temps entre le premier et le second échantillon est d'au moins 4 semaines ; et/ou
D) une augmentation à l'étape b) par rapport à l'étape a) indique que le sujet a contracté l'infection entre le premier instant et un instant ultérieur ; et/ou
E) le second échantillon est obtenu auprès d'un sujet présentant un risque de contracter l'infection *à Schistosoma* et/ou présentant un ou plusieurs symptômes indicatifs de l'infection à *Schistosoma,* éventuellement dans lequel :
(i) le sujet présente un risque s'il s'est rendu dans un endroit où l'infection *à Schistosoma* est répandue et/ou endémique ; ou
(ii) les symptômes comprennent : une éruption cutanée, de la fièvre, des maux de tête, des myalgies, un malaise général, une diarrhée, une toux non productive, des douleurs musculaires, des douleurs articulaires, une sensibilité ou une douleur abdominale, une hépatosplénomégalie, une éosinophilie, une urticaire, un angio-œdème, une (micro)hématurie, des infiltrats pulmonaires transitoires et/ou des œufs de parasites dans un ou plusieurs des selles, de l'urine et des fluides génitaux ; et/ou
F) le second échantillon est obtenu auprès du sujet au moins 4 semaines après l'apparition d'un ou de plusieurs symptômes indicatifs de l'infection *à Schistosoma,* dans lequel éventuellement les symptômes comprennent : une éruption cutanée, de la fièvre, des maux de tête, des myalgies, un malaise général, une diarrhée, une toux non productive, des douleurs musculaires, des douleurs articulaires, une sensibilité ou une douleur abdominale, une hépatosplénomégalie, une éosinophilie, une urticaire, un angio-œdème, une (micro)hématurie, des infiltrats pulmonaires transitoires et/ou des œufs de parasites dans un ou plusieurs des selles, de l'urine et des fluides génitaux.

5. Procédé de l'une quelconque des revendications précédentes, dans lequel le sujet présente :
A) une schistosomiase aiguë ; et/ou
B) une infection primaire *à Schistosoma.*

6. Procédé de l'une quelconque des revendications 2(C) à 5, dans lequel l'infection à *Schistosoma* est causée par une espèce de *Schistosoma* choisie dans le groupe constitué par : *Schistosoma mansoni, Schistosoma japonicum, Schistosoma mekongi, Schistosoma guineensis, Schistosoma intercalatum* et *Schistosoma haematobium.*

7. Procédé de l'une quelconque des revendications précédentes, dans lequel :
A) l'anticorps est choisi dans le groupe constitué par : IgM, IgA et IgG ; et/ou
B) le sujet est un humain ; et/ou
C) l'échantillon est choisi dans le groupe constitué par : un échantillon de sang, un échantillon nasal, un échantillon d'urine, un échantillon de fluide génital et un échantillon de selles, éventuellement dans lequel :
(i) l'échantillon de sang est un échantillon de sérum, un échantillon de plasma ou un échantillon de sang total, en outre éventuellement lorsque l'échantillon est un échantillon de sang, l'anticorps est IgM et/ou IgG ; ou
(ii) l'échantillon nasal est obtenu par échantillonnage par nasosorption, en outre éventuellement lorsque l'échantillon est un échantillon nasal ou un échantillon de sang, l'anticorps est IgA ; ou
(iii) lorsque l'échantillon est un échantillon nasal ou un échantillon de sang, l'anticorps est IgA,
(iv) lorsque l'échantillon est un échantillon de sang, l'anticorps est IgM et/ou IgG ; et/ou
D) la liaison entre l'échantillon et le composé est détectée par une technique choisie dans le groupe constitué par : le dosage par microréseau, ELISA, le dosage par immunobuvardage, le dosage multiplex à base de billes et le test à flux latéral.

8. Composé R-X-Y, dans lequel R est conforme à la Formule I : 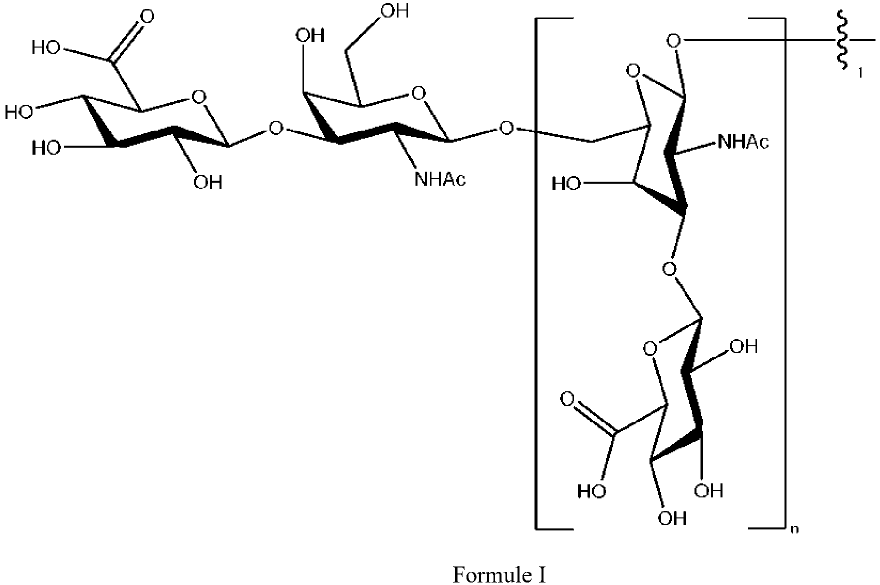 dans lequel 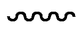 1 désigne le point de fixation de R à X, dans lequel X est choisi dans le groupe constitué par : une liaison et un coupleur ; et Y est choisi dans le groupe constitué par : H, une molécule rapporteuse, une molécule porteuse et un support solide ; et n est un nombre entier choisi entre 2 et 19.

9. Composé de la revendication 8, dans lequel :
A) n = 2, 3, 4 ou 5 ; et/ou
B) X est --alkylène en C₂-C₁₀-NH- (par exemple C₆H₁₂NH-), éventuellement dans lequel :
(i) Y est H ou
(ii) Y est une molécule rapporteuse, une molécule porteuse ou un support solide ; et/ou
C) Y comprend un support solide, dans lequel éventuellement le support solide est choisi dans le groupe constitué d'une membrane, d'une matière plastique, d'un polymère et de verre.

10. Composition comprenant une pluralité de composés distincts selon la revendication 8.

11. Composition de la revendication 10, dans laquelle la composition comprend au moins deux composés choisis dans le groupe constitué par :
(i) un composé de Formule I, dans lequel n = 2 ;
(ii) un composé de Formule I, dans lequel n = 3 ;
(iii) un composé de Formule I, dans lequel n = 4 ; et
(iv) un composé de Formule I, dans lequel n = 5.
